# EUROPEAN PATENT APPLICATION

(11) **EP 4 159 718 A1**
(43) Date of publication of application: **05.04.2023**
(21) Application number: 21814082.0
(22) Date of filing: 05.04.2021
(51) Int. Cl.: C07D 211/34, A61K 31/445, A61K 31/454, A61P 25/00, A23L 33/10, A61P 25/18, C07D 401/04

(54) **SULFONAMIDE DERIVATIVE AND PHARMACEUTICAL COMPOSITION COMPRISING SAME AS ACTIVE INGREDIENT FOR PREVENTING OR TREATING MENTAL ILLNESS**

(30) Priority: 29.05.2020 KR 20200065505; 31.03.2021 KR 20210041812
(71) Applicant: Daegu-Gyeongbuk Medical Innovation Foundation, Daegu 41061 (KR); Trineuro, Seoul 05029 (KR)
(72) Inventor: SONG, Minsoo, Daegu 41061 (KR); IM, Chun Young, Daegu 41061 (KR); PARK, Ga Young, Daegu 41061 (KR); KO, Eun Bi, Daegu 41061 (KR); KANG, Jihee, Daegu 41061 (KR); BAE, Seri, Daegu 41061 (KR); KIM, Soong-Hyun, Daegu 41061 (KR); PARK, Yoojin, Daegu 41061 (KR); LEE, Eunhye, Daegu 41061 (KR); CHOI, Yujeong, Daegu 41061 (KR); JOO, Jeongmin, Daegu 41061 (KR); LEE, Sion, Daegu 41061 (KR); SHIN, Chan Young, Seoul 05029 (KR); KWON, Kyoung Ja, Seoul 05029 (KR); HWANG, Hee Jong, Daegu 41106 (KR); PAUDEL, Suresh, Seoul 05029 (KR); JEON, Se Jin, Seoul 05029 (KR); CHO, Kyu Suk, Seoul 05029 (KR)
(74) Representative: EP&C
(86) International application number: PCT/KR2021/004197
(87) International publication number: WO 2021/241875

(57) **Abstract**

Disclosed are a sulfonamide derivative and a pharmaceutical composition comprising same as an active ingredient for use in preventing or treating mental illness. The derivative is superbly effective in a triple reuptake inhibitor of serotonin, norepinephrine, and dopamine, and thus can be effectively used in treating mental illness.

## Description

### TECHNICAL FIELD

The present invention relates to a sulfonamide and a pharmaceutical composition for use in preventing or treating mental illness, containing same as an active ingredient.

### BACKGROUND ART

Mental illness refers to pathological abnormalities of the brain characterized by identifiable symptoms resulting in abnormalities of cognition, emotion, mood, or emotion. Representative mental illness includes attention deficit hyperactivity disorder (ADHD), panic disorder, bipolar disorder, depression, schizophrenia, eating disorder, dissociative disorder, post-traumatic stress disorder, etc. and these disorders are characterized by various dysfunctions, severity and duration of symptoms, etc. Factors that cause mental illness include: 1) brain secretion disorder caused by an excess or deficiency of neurotransmitters affecting cerebral nerve functions, 2) brain damage and degeneration of brain functions, 3) abnormal secretion of hormones, 4) stress, etc.

Numerous neurons constituting the brain regulate nerve functions by communicating with different neurons, and these neurons have two main transmission methods. One method is an electrical transmission, which is a method of transmission within neurons, and the other method is a chemical transmission, which is a method of extraneuronal transmission between neurons. The chemical transmission occurs at the synapse (the junction between the axon terminal of one neuron and the dendrite of the next neuron), and it is a method of secreting neurotransmitters (i.e., chemical substances) in the pre-synaptic process and transferring them to another synapse. As a postsynaptic process, the neuron which has received neurotransmitters through the synapse is activated and excited when the threshold is exceeded according to the amount of the neurotransmitters. The thus activated neuron generates an electrical signal in the dendrite and transmits the signal to the axon, and the axon secretes neurotransmitters through the synapse, and this whole process is repeated. Neurotransmitters secreted from neurons to regulate synaptic responses with neurons and eventually to control neural circuits are known to be the most important factors regulating human behavior.

When neurotransmitters are secreted from a neuron, specific receptors in a postsynaptic neuron forming a synapse with the neuron bind to the neurotransmitters to be activated, postsynaptic neurons and synaptic activation can be regulated depending on the signaling of receptors. Human behavior is presumed to be regulated by the regulation of activation of neurons and synapses by neurotransmitters, and mutations in genes of neurotransmitters (when the neurotransmitters are peptides), enzymes that produce neurotransmitters, or genes of neurotransmitter receptors have been reported to be associated with behavioral control disorders. Additionally, it is known that drugs that regulate the amount of neurotransmitters or signal transmission through receptors can regulate human behavior.

Neurotransmitters that regulate behavior as described above include monovalent amines (e.g., catecholamine and serotonin), acetylcholine, excitatory or inhibitory amino acids, and various neuropeptides and receptors thereof are largely classified into ionotropic receptors, G-protein-coupled receptors, and tyrosine kinase receptors.

Among various neurotransmitters in the brain, dopamine, which is a catecholamine-based neurotransmitter, may be an excitatory signal or inhibitory signal depending on the type of postsynaptic dopamine receptors. Dopamine is associated with behavior, attention, learning, motivation, reward system, reinforcing effect, etc. in our body. When dopamine activity is reduced, depressive symptoms (e.g., lethargy, decreased motivation, decreased energy, etc.) appear. In addition, dopamine excites neurons in the frontal lobe and affects mental activities such as short-term memory, planning, and strategy. In particular, the reward given by dopamine plays a very important role in the construction of neural pathways in the early brain because the reward makes humans repeat the behavior and learning to feel the pleasure of this reward.

Norepinephrine, which is a catecholamine-based neurotransmitter found in the autonomic nervous system, is known as a secondary neurotransmitter of dopamine, which is secreted by the release action of dopamine. Norepinephrine is associated with arousal, which makes the body tense, pay attention and alert to its surroundings, and involved in fear or stress response. In particular, norepinephrine is closely associated with emotions and is considered a cause of mood disorders (e.g., depression and bipolar disorder). That is, there is a hypothesis that depression is a state in which the function of norepinephrine or dopamine is reduced, and mania is a state in which the function of norepinephrine is excessively accelerated.

Serotonin, which is a neurotransmitter that usually acts in an inhibitory way, is associated with many basic human physiological phenomena (e.g., sleep, appetite, pain, regulation of body temperature, cardiovascular response, sexual desire, anxiety, depression, etc.). Clinically, serotonin is considered to be involved in the causes of various neuropsychiatric disorders (e.g., depression, schizophrenia, obsessive-compulsive disorder, anxiety disorder, eating disorder, sleep disorder, sexual disorder, impulse control disorder, developmental disorder, degenerative brain diseases, stress disorder, motor disorder, etc.), and in particular, the association with depression is very important.

Neurotransmitters are contained in synaptic vesicles of a neuron, and when stimulation is transferred to the neuron, calcium ion channels open before the synapse between neuron, leading to an influx of calcium into the neuron to move synaptic vesicles to the cell membrane of the neuron. The vesicles migrated to the cell membrane and is released into the synapse by exocytosis at the axon terminal. The released neurotransmitters interact with the receptors to excite or inhibit the neuron that has received the neurotransmitters, and the remaining neurotransmitters after acting on the receptors are reuptaken at the axon terminal of the presynaptic neuron, degraded by degradation, enzymes present in the postsynaptic membrane, or diffused outward and removed by glial cells.

The reuptake process occurs by a neurotransmitter transporter. Depending on the type of neurotransmitters, specific neurotransmitter transporters exist, which are present in the synaptic membrane and are responsible for reuptake of neurotransmitters present in the synaptic cleft into the presynaptic neuron. The regulation of the concentration of neurotransmitters through this process would ultimately affect the intensity and duration of activation of various receptors present in the postsynaptic neuron.

As described above, the reuptake of neurotransmitters refers to the process in which neurotransmitters are inactivated by the reuptake into the presynaptic neuron by specific neurotransmitter transporters in the membrane of the presynaptic neuron from which the neurotransmitters have been released. The increase in reuptake of neurotransmitters causes deficiency and imbalance of neurotransmitters, resulting in diseases associated with reuptake of neurotransmitters. Treatments for these reuptake-related diseases improve symptoms by increasing the concentration of neurotransmitters in the synapse by blocking the reuptake of these substances.

Neurotransmitter reuptake inhibitors include selective serotonin reuptake inhibitors, serotonin-norepinephrine reuptake inhibitors, selective norepinephrine reuptake inhibitors, dopamine-norepinephrine reuptake inhibitors, serotonin receptor antagonists-serotonin reuptake inhibitors, etc., and these inhibitors are used to treat depression, mood disorder, ADHD, chronic neuropathic pain, obsessive-compulsive disorder, panic disorder, anxiety disorder, menopausal symptoms, etc.

Drugs for treating mental illness are continuously under development, and among them, as related art associated with neurotransmitter reuptake inhibitors, Korean Patent Laid-open No. 10-2009-0089439 discloses chromen-2-one derivatives, a use thereof as monoamine neurotransmitter reuptake inhibitors, and a use for the treatment of mental illness, and Korean Patent Laid-open No. 10-2009-0117736, which is directed to a composition for selective serotonin reuptake inhibition and a method for producing the same, discloses a composition containing asiaticoside and madecassoside.

However, currently-used treatments for mental illness in the field of cranial nerves act merely on one nervous system among the various neurotransmitters suggested as related etiological mechanisms or have insufficient selectivity for neurotransmitters, and thus have many problems in therapeutic effects and compliance of patients, and cause serious adverse effects (e.g., growth retardation, sleep disorder, and dependence); therefore, there is a need to develop drugs that can flexibly regulate multiple targets with various differential selectivity for neurotransmitters.

In this regard, development on drugs that regulate multiple targets is in progress, in which Korean Patent Laid-open No. 10-2013-0026292, which is directed to novel azetidine derivatives and antidepressant composition containing the same, discloses an antidepressant composition containing an azetidine derivative capable of simultaneously inhibiting reuptake of dopamine, serotonin, and norepinephrine; Korean Patent Laid-open No. 2002-0079730 discloses a use of aryl- and heteroaryl-substituted tetrahydroisoquinolines to block reuptake of norepinephrine, dopamine, and serotonin; and Korean Patent Laid-open No. 10-2010019982, which is directed to phenyl substituted cycloalkylamines as monoamine reuptake inhibitors, discloses phenyl substituted cycloalkylamines which inhibit reuptake of endogenous monoamines, such as dopamine, serotonin and norepinephrine from the synaptic cleft and modulate one or more monoamine transporter.

The present inventors have made efforts to develop drugs that could flexibly regulate multiple targets by securing the differential selectivity of neuronal function control thereof, based on substances that simultaneously regulate dopamine, norepinephrine, and serotonin nervous system, and confirmed that a sulfonamide has the triple reuptake inhibitory effect of serotonin, norepinephrine, and dopamine, and thus can be useful in the treatment of mental illness, thereby completing the present invention.

### DISCLOSURE OF THE INVENTION

### TECHNICAL PROBLEM

One object of the present invention is to provide a sulfonamide derivative.

Another object of the present invention is to provide a pharmaceutical composition for use in preventing or treating mental illness, containing a sulfonamide derivative as an active ingredient.

Still another object of the present invention is to provide a triple reuptake inhibitor of serotonin, norepinephrine, and dopamine for use in preventing or treating mental illness, containing a sulfonamide derivative as an active ingredient.

Still another object of the present invention is to provide a health functional food composition for use in preventing or improving mental illness, containing a sulfonamide derivative as an active ingredient.

### TECHNICAL SOLUTION

In order to accomplish the objects,
an aspect of the present invention provides a compound represented by Formula 1, an isomer thereof, a solvate thereof, a hydrate thereof, or a pharmaceutically acceptable salt thereof.

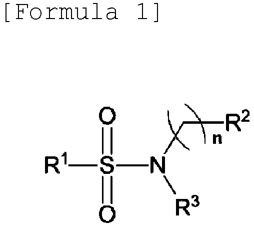

In Formula 1, R¹, R², R³ and n are the same as defined in the description.

Another aspect of the present invention provides a pharmaceutical composition for use in preventing or treating mental illness, containing the compound represented by Formula 1 above, an isomer thereof, a solvate thereof, a hydrate thereof, or a pharmaceutically acceptable salt thereof as an active ingredient.

Still another aspect of the present invention provides a triple reuptake inhibitor of serotonin, norepinephrine, and dopamine for use in preventing or treating mental illness, containing the compound represented by Formula 1 above, an isomer thereof, a solvate thereof, a hydrate thereof, or a pharmaceutically acceptable salt thereof as an active ingredient.

Still another aspect of the present invention provides a health functional food composition for use in preventing or improving mental illness, containing the compound represented by Formula 1 above, an isomer thereof, a solvate thereof, a hydrate thereof, or a pharmaceutically acceptable salt thereof as an active ingredient.

Still another aspect of the present invention provides a method for use in preventing or treating mental illness, which includes administering a pharmaceutical composition or health functional food composition containing the compound represented by Formula 1 above, an isomer thereof, a solvate thereof, a hydrate thereof, or a pharmaceutically acceptable salt thereof as an active ingredient to a subject in need thereof.

Still another aspect of the present invention provides a use of a pharmaceutical composition or health functional food composition containing the compound represented by Formula 1 above, an isomer thereof, a solvate thereof, a hydrate thereof, or a pharmaceutically acceptable salt thereof as an active ingredient in the prevention or treatment of mental illness.

### ADVANTAGEOUS EFFECTS

The compound represented by Formula 1 according to the present invention is excellent in the triple reuptake inhibitory effect of serotonin, norepinephrine, and dopamine, and thus can be useful in the treatment of mental illness.

### MODE FOR CARRYING OUT THE INVENTION

Hereinafter, the present invention will be described in detail.

Meanwhile, the embodiments of the present invention may be modified in various forms, and the scope of the present invention is not limited to the embodiments described below. In addition, the embodiments of the present invention are provided to more completely explain the present invention to those skilled in the art. Furthermore, "including" a certain component throughout the specification means that other components may be further included instead of excluding other components unless otherwise stated.

An aspect of the present invention provides a compound represented by Formula 1, an isomer thereof, a solvate thereof, a hydrate thereof, or a pharmaceutically acceptable salt thereof.

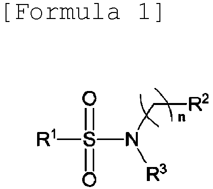

In Formula 1,
n is an integer of 0 to 5,
R¹ is unsubstituted or substituted aryl of C₆₋₁₀, or unsubstituted or substituted heteroaryl of 5 to 14 atoms, containing one or more heteroatoms selected from the group consisting of N, S and O,
the substituted aryl and the substituted heteroaryl are independently substituted with one or more substituents selected from the group consisting of -OH, halogen, unsubstituted or substituted linear or branched C₁₋₁₀ alkyl with one or more halogens, unsubstituted or substituted linear or branched C₁₋₁₀ alkoxy with one or more halogens, unsubstituted or substituted phenyl, -NR^{a}R^{b}, - NHC(=0) (CH₂)ₘNR^{a}R^{b} and phenoxy, where the substituted phenyl is substituted with linear or branched C₁₋₁₀ alkyl, linear or branched C₁₋₁₀ alkoxy or halogen, R^{a} and R^{b} are independently hydrogen or unsubstituted or substituted linear or branched C₁₋₁₀ alkyl with one or more halogens, or may form heterocycloalkyl of 5 to 6 atoms, containing one or more heteroatoms selected from the group consisting of N, O and S, together with a N atom bonded, and m is an integer of 0 to 5, R² is unsubstituted or substituted heterocycloalkyl of 4 to 8 atoms, containing one or more heteroatoms selected from the group consisting of N, S and O,
the substituted heterocycloalkyl is substituted with one or more substituents selected from the group consisting of indole, -(CH₂)ₚC₆₋₁₀ aryl, and heterocycloalkyl of 5 to 6 atoms, containing one or more heteroatoms selected from the group consisting of N, O and S, where the indole and C₆₋₁₀ aryl are unsubstituted or independently substituted with one or more substituents selected from the group consisting of halogen, unsubstituted or substituted linear or branched C₁₋₅ alkyl with one or more halogens, and unsubstituted or substituted linear or branched C₁₋₅ alkoxy with one or more halogens, and p is an integer of 0 to 5; and
R³ is hydrogen or linear or branched C₁₋₅ alkyl; or
if n is 0, R² and R³ may form unsubstituted or substituted heterocycloalkyl of 4 to 8 atoms, containing one or more heteroatoms selected from the group consisting of N, S and O, together with a nitrogen atom bonded, the substituted heterocycloalkyl is substituted with indole or - (CH₂)_{q}C₆₋₁₀ aryl, and q is an integer of 0 to 5.

In Formula 1,
n is an integer of 0 to 4,
R¹ is unsubstituted or substituted aryl of C₆₋₁₀, or unsubstituted or substituted heteroaryl of 5 to 10 atoms, containing one or more heteroatoms selected from the group consisting of N, S and O,
the substituted aryl and the substituted heteroaryl are independently substituted with one or more substituents selected from the group consisting of -OH, halogen, unsubstituted or substituted linear or branched C₁₋₅ alkyl with one or more halogens, unsubstituted or substituted linear or branched C₁₋₅ alkoxy with one or more halogens, unsubstituted or substituted phenyl, -NR^{a}R^{b}, - NHC(=O)(CH₂)ₘNR^{a}R^{b} and phenoxy, where the substituted phenyl is substituted with linear or branched C₁₋₅ alkyl, linear or branched C₁₋₅ alkoxy or halogen, R^{a} and R^{b} are independently hydrogen or unsubstituted or substituted linear or branched C₁₋₅ alkyl with one or more halogens, or may form heterocycloalkyl of 5 to 6 atoms, containing one or more heteroatoms selected from the group consisting of N, O and S, together with a N atom bonded, and m is an integer of 0 to 4, R² is unsubstituted or substituted heterocycloalkyl of 4 to 6 atoms, containing one or more heteroatoms selected from the group consisting of N, S and O,
the substituted heterocycloalkyl is substituted with one or more substituents selected from the group consisting of indole, -(CH₂)ₚ phenyl, and heterocycloalkyl of 5 to 6 atoms, containing one or more O, where the indole and phenyl are unsubstituted or independently substituted with one or more substituents selected from the group consisting of halogen, unsubstituted or substituted linear or branched C₁₋₃ alkyl with one or more halogens, and unsubstituted or substituted linear or branched C₁₋₃ alkoxy with one or more halogens, and p is an integer of 0 to 4; and
R³ is hydrogen or linear or branched C₁₋₃ alkyl; or
if n is 0, R² and R³ may form unsubstituted or substituted heterocycloalkyl of 4 to 6 atoms, containing one or more heteroatoms selected from the group consisting of N, S and O, together with a nitrogen atom bonded, the substituted heterocycloalkyl is substituted with indole or - (CH₂)_{q} phenyl, and q may be an integer of 0 to 4.

In Formula 1,
n is an integer of 0 to 3,
R¹ is unsubstituted or substituted aryl of C₆₋₁₀, or unsubstituted or substituted heteroaryl of 5 to 9 atoms, containing one or more N,
the substituted aryl and the substituted heteroaryl are independently substituted with one or more substituents selected from the group consisting of -OH, halogen, unsubstituted or substituted linear or branched C₁₋₄ alkyl with one or more halogens, unsubstituted or substituted linear or branched C₁₋₄ alkoxy with one or more halogens, unsubstituted or substituted phenyl, -NR^{a}R^{b}, - NHC (=O)(CH₂)ₘNR^{a}R^{b} and phenoxy, where the substituted phenyl is substituted with linear or branched C₁₋₄ alkyl, linear or branched C₁₋₄ alkoxy or halogen, R^{a} and R^{b} are independently hydrogen or unsubstituted or substituted linear or branched C₁₋₄ alkyl with one or more halogens, or may form heterocycloalkyl of 5 to 6 atoms, containing one or more N, together with a N atom bonded, and m is an integer of 0 to 3,
R² is unsubstituted or substituted heterocycloalkyl of 6 atoms, containing one or more N,
the substituted heterocycloalkyl is substituted with one or more substituents selected from the group consisting of indole, -(CH₂)ₚ phenyl, and heterocycloalkyl of 6 atoms, containing one or more O, where the indole and phenyl are unsubstituted or independently substituted with one or more substituents selected from the group consisting of halogen, methyl, ethyl, methoxy, ethoxy and -CF₃, and p is an integer of 0 to 3; and
R³ is hydrogen or methyl; or
if n is 0, R² and R³ may form unsubstituted or substituted heterocycloalkyl of 6 atoms, containing one or more N, together with a nitrogen atom bonded, the substituted heterocycloalkyl is substituted with indole or -(CH₂)_{q} phenyl, and q is an integer of 0 to 3.

In Formula 1,
n is 0, 2, 3 or 4,
R¹ is unsubstituted or substituted phenyl, unsubstituted naphthyl, unsubstituted indole, unsubstituted or substituted pyridinyl, or unsubstituted quinolinyl, the substituted phenyl and the substituted pyridinyl are independently substituted with one or more substituents selected from the group consisting of -OH, halogen, methoxy, butoxy, -OCF₃, -NH₂, -NHnBu, -NHC(=O)(CH₂)₂ piperidinyl, - NHC(=O)(CH₂)₂N(CH₃)₂, unsubstituted or substituted phenyl with methoxy, halogen or methyl, and phenoxy,
R² is substituted piperidinyl or substituted piperazinyl,
the substituted piperidinyl and substituted piperazinyl are independently substituted with one or more substituents selected from the group consisting of indole, -(CH₂)ₚ phenyl, and methyltetrahydropyrazinyl, where the indole and phenyl are unsubstituted or independently substituted with one or more substituents selected from the group consisting of halogen, methyl, methoxy and -CF₃, and p is an integer of 0 to 2; and
R³ is hydrogen; or
if n is 0, R² and R³ may form piperidinyl together with a nitrogen atom bonded, and the piperidinyl may be substituted with indole or -(CH₂)_{q} phenyl.

In Formula 1,
n is 0, 2, 3 or 4;
R¹ is 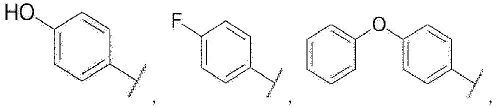 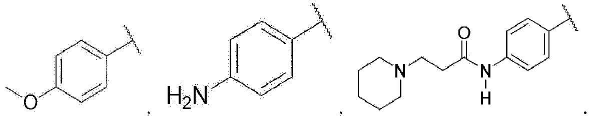 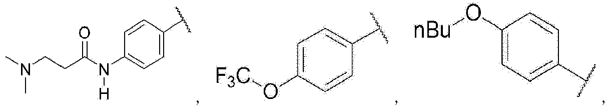 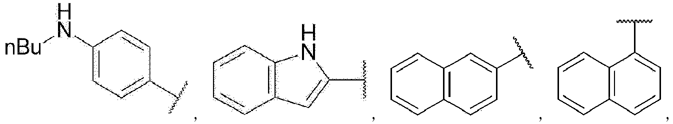 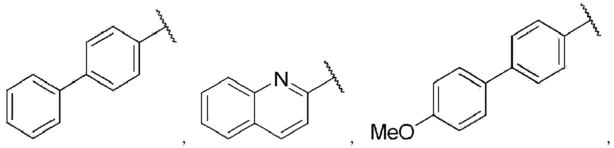 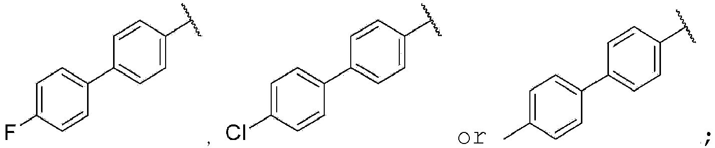
R² is 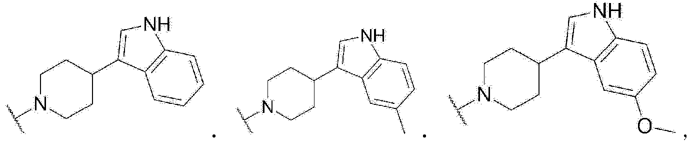 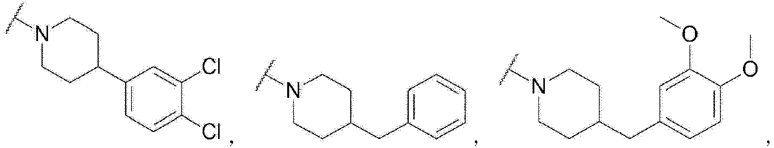 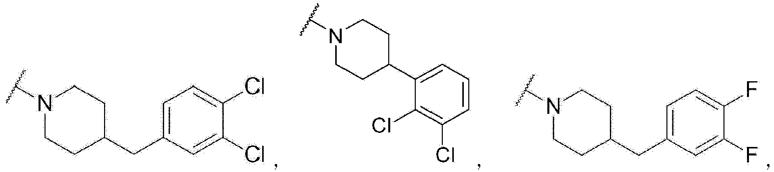 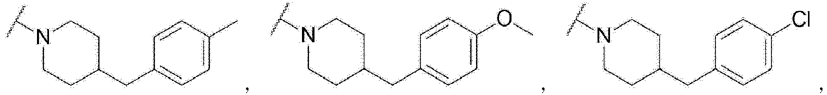 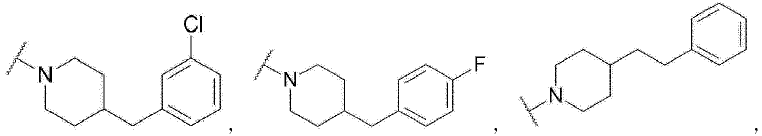 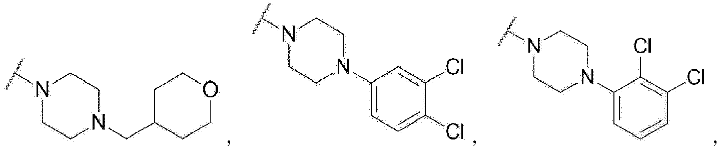 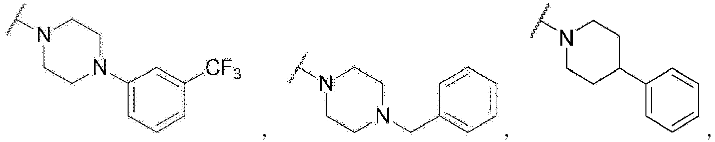 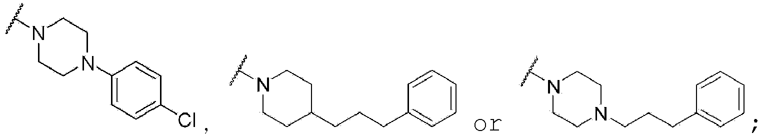 and
R³ is hydrogen; or
if n is 0, -NR²R³ may be

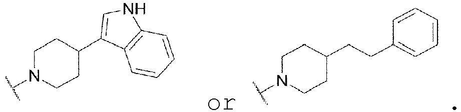

Examples of the compound represented by Formula 1 according to the present invention may include the following compound group:
<1> N-(2-(4-benzylpiperidin-1-yl)ethyl)-4-hydroxybenzenesulfonamide;
<2> N-(2-(4-benzylpiperidin-1-yl)ethyl)-4-fluorobenzenesulfonamide;
<3> N-(2-(4-benzylpiperidin-1-yl)ethyl)-4-phenoxybenzenesulfonamide;
<4> 4-butoxy-N-(2-(4-(3,4-dichlorobenzyl)piperidin-1-yl)ethyl)benzenesulfonamide;
<5> 3-(1-((4-butoxyphenyl)sulfonyl)piperidin-4-yl)-1H-indole;
<6> N-(2-(4-(1H-indol-3-yl)piperidin-1-yl)ethyl)-4-butoxybenzenesulfonamide;
<7> 4-butoxy-N-(2-(4-(5-methyl-1H-indol-3-yl)piperidin-1-yl)ethyl)benzenesulfonamide;
<8> 4-butoxy-N-(2-(4-(5-methoxy-1H-indol-3-yl)piperidin-1-yl)ethyl)benzenesulfonamide;
<9> 1-((4-butoxyphenyl)sulfonyl)-4-phenethylpiperidin;
<10> N-(3-(4-benzylpiperidin-1-yl)propyl)-4-butoxybenzenesulfonamide;
<11> N-(3-(4-(1H-indol-3-yl)piperidin-1-yl)propyl)-4-butoxybenzenesulfonamide;
<12> N-(3-(4-benzylpiperidin-1-yl)propyl)-1H-indol-2-sulfonamide;
<13> N-(3-(4-benzylpiperidin-1-yl)propyl)-4-phenoxybenzenesulfonamide;
<14> N-(3-(4-benzylpiperidin-1-yl)propyl)-4-methoxybenzenesulfonamide;
<15> 4-amino-N-(3-(4-benzylpiperidin-1-yl)propyl)benzenesulfonamide;
<16> N-(3-(4-benzylpiperidin-1-yl)propyl)-4-(butylamino)benzenesulfonamide;
<17> N-(4-(N-(3-(4-benzylpiperidin-1-yl)propyl)sulfamoyl)phenyl)-3-(piperidin-1-yl)propaneamide;
<18> N-(4-(N-(3-(4-benzylpiperidin-1-yl)propyl)sulfamoyl)phenyl)-3-(dimethylamino)propaneamide;
<19> N-(3-(4-benzylpiperidin-1-yl)propyl)-4-butoxybenzenesulfineamide;
<20> 4-butoxy-N-(3-(4-(3,4-dichlorobenzyl)piperidin-1-yl)propyl)benzenesulfonamide;
<21> 4-butoxy-N-(3-(4-(3,4-difluorobenzyl)piperidin-1-yl)propyl)benzenesulfonamide;
<22> 4-butoxy-N-(3-(4-(3,4-dichlorophenyl)piperidin-1-yl)propyl)benzenesulfonamide;
<23> 4-butoxy-N-(3-(4-(3,4-dichlorophenyl)piperazin-1-yl)propyl)benzenesulfonamide;
<24> 4-butoxy-N-(3-(4-(2,3-dichlorophenyl)piperazin-1-yl)propyl)benzenesulfonamide;
<25> 4-butoxy-N-(3-(4-((tetrahydro-2H-pyran-4-yl)methyl)piperazin-1-yl)propyl)benzenesulfonamide;
<26> 4-butoxy-N-(3-(4-(3-(trifluoromethyl)phenyl)piperazin-1-yl)propyl)benzenesulfonamide;
<27> N-(3-(4-benzylpiperazin-1-yl)propyl)-4-butoxybenzenesulfonamide;
<28> 4-butoxy-N-(3-(4-(4-chlorobenzyl)piperidin-1-yl)propyl)benzenesulfonamide;
<29> 4-butoxy-N-(3-(4-(4-methylbenzyl)piperidin-1-yl)propyl)benzenesulfonamide;
<30> 4-butoxy-N-(3-(4-(4-methoxybenzyl)piperidin-1-yl)propyl)benzenesulfonamide;
<31> 4-butoxy-N-(3-(4-(3-chlorobenzyl)piperidin-1-yl)propyl)benzenesulfonamide;
<32> 4-butoxy-N-(3-(4-(4-fluorobenzyl)piperidin-1-yl)propyl)benzenesulfonamide;
<33> 4-butoxy-N-(3-(4-(2,3-dichlorophenyl)piperidin-1-yl)propyl)benzenesulfonamide;
<34> N-(3-(4-benzylpiperidin-1-yl)propyl)-4-(trifluoromethoxy)benzenesulfonamide;
<35> N-(3-(4-benzylpiperidin-1-yl)propyl)-5-butoxypyridin-2-sulfonamide;
<36> N-(2-(4-benzylpiperidin-1-yl)ethyl)-1H-indol-2-sulfonamide;
<37> 4-butoxy-N-(3-(4-(3,4-dimethoxybenzyl)piperidin-1-yl)propyl)benzenesulfonamide;
<38> N-(2-(4-benzylpiperidin-1-yl)ethyl)naphthalen-2-sulfonamide;
<39> N-(2-(4-benzylpiperidin-1-yl)ethyl)naphthalen-1-sulfonamide;
<40> N-(2-(4-benzylpiperidin-1-yl)ethyl)-[1,1'-biphenyl]-4-sulfonamide;
<41> N-(2-(4-benzylpiperidin-1-yl)ethyl)quinolin-2-sulfonamide;
<42> N-(2-(4-phenylpiperidin-1-yl)ethyl)naphthalen-2-sulfonamide;
<43> N-(2-(4-phenylpiperidin-1-yl)ethyl)naphthalen-1-sulfonamide;
<44> N-(2-(4-phenylpiperidin-1-yl)ethyl)-[1,1'-biphenyl]-4-sulfonamide;
<45> N-(2-(4-phenylpiperidin-1-yl)ethyl)quinolin-2-sulfonamide;
<46> N-(2-(4-benzylpiperazin-1-yl)ethyl)naphthalen-2-sulfonamide;
<47> N-(2-(4-benzylpiperazin-1-yl)ethyl)naphthalen-1-sulfonamide;
<48> N-(2-(4-phenylpiperazin-1-yl)ethyl)-[1,1'-biphenyl]-4-sulfonamide;
<49> N-(2-(4-phenylpiperazin-1-yl)ethyl)quinolin-2-sulfonamide;
<50> N-(2-(4-(4-chlorophenyl)piperazin-1-yl)ethyl)naphthalen-2-sulfonamide;
<51> N-(2-(4-(4-chlorophenyl)piperazin-1-yl)ethyl)naphthalen-1-sulfonamide;
<52> N-(2-(4-(4-chlorophenyl)piperazin-1-yl)ethyl)-[1,1'-biphenyl]-4-sulfonamide;
<53> N-(2-(4-(4-chlorophenyl)piperazin-1-yl)ethyl)quinolin-2-sulfonamide;
<54> N-(3-(4-benzylpiperidin-1-yl)propyl)naphthalen-2-sulfonamide;
<55> N-(3-(4-benzylpiperidin-1-yl)propyl)naphthalen-1-sulfonamide;
<56> N-(3-(4-benzylpiperidin-1-yl)propyl)-[1,1'-biphenyl]-4-sulfonamide;
<57> N-(3-(4-benzylpiperidin-1-yl)propyl)quinolin-2-sulfonamide;
<58> N-(3-(4-benzylpiperidin-1-yl)propyl)-4'-fluoro-[1,1'-biphenyl]-4-sulfonamide;
<59> N-(3-(4-benzylpiperidin-1-yl)propyl)-4'-chloro-[1,1'-biphenyl]-4-sulfonamide;
<60> N-(3-(4-benzylpiperidin-1-yl)propyl)-4'-methyl-[1,1'-biphenyl]-4-sulfonamide;
<61> N-(3-(4-benzylpiperidin-1-yl)propyl)-4'-methoxy-[1,1'-biphenyl]-4-sulfonamide;
<62> N-(3-(4-benzylpiperidin-1-yl)propyl)naphthalen-2-sulfonamide;
<63> N-(3-(4-benzylpiperidin-1-yl)propyl)naphthalen-1-sulfonamide;
<64> N-(3-(4-benzylpiperidin-1-yl)propyl)-[1,1'-biphenyl]-4-sulfonamide;
<65> N-(3-(4-phenylpiperidin-1-yl)propyl)quinolin-2-sulfonamide;
<66> N-(2-(4-benzylpiperazin-1-yl)propyl)naphthalen-2-sulfonamide;
<67> N-(2-(4-benzylpiperazin-1-yl)propyl)naphthalen-1-sulfonamide;
<68> N-(3-(4-phenylpiperazin-1-yl)propyl)-[1,1'-biphenyl]-4-sulfonamide;
<69> N-(3-(4-phenylpiperazin-1-yl)propyl)quinolin-2-sulfonamide;
<70> N-(3-(4-(4-chlorophenyl)piperazin-1-yl)propyl)naphthalen-2-sulfonamide;
<71> N-(3-(4-(4-chlorophenyl)piperazin-1-yl)propyl)naphthalen-1-sulfonamide;
<72> N-(3-(4-(4-chlorophenyl)piperazin-1-yl)propyl)-[1,1'-biphenyl]-4-sulfonamide;
<73> N-(3-(4-(4-chlorophenyl)piperazin-1-yl)propyl)quinolin-2-sulfonamide;
<74> 4'-fluoro-N-(3-(4-(3-phenylpropyl)piperidin-1-yl)propyl)-[1,1'-biphenyl]-4-sulfonamide;
<75> N-(3-(4-(3-phenylpropyl)piperidin-1-yl)propyl)-[1,1'-biphenyl]-4-sulfonamide;
<76> N-(3-(4-(3-phenylpropyl)piperazin-1-yl)propyl)-[1,1'-biphenyl]-4-sulfonamide;
<77> N-(4-(4-benzylpiperidin-1-yl)butyl)-[1,1'-biphenyl]-4-sulfonamide;
<78> N-(4-(4-(3-phenylpropyl)piperazin-1-yl)butyl)-[1,1'-biphenyl]-4-sulfonamide; and
<79> N-(4-(4-(3-phenylpropyl)piperidin-1-yl)butyl)-[1,1'-biphenyl]-4-sulfonamide.

The compound represented by Formula 1 of the present invention can be used in the form of a pharmaceutically acceptable salt, and an acid addition salt formed by a pharmaceutically acceptable free acid is useful as the salt. The acid addition salt is obtained from inorganic acids (e.g., hydrochloric acid, nitric acid, phosphoric acid, sulfuric acid, hydrobromic acid, hydroiodic acid, nitrous acid, phosphorous acid, etc.); non-toxic organic acids (*e*.*g*., aliphatic mono- and dicarboxylates, phenyl-substituted alkanoates, hydroxy alkanoates and alkanedioates, aromatic acids, aliphatic and aromatic sulfonic acids, etc.; organic acids (e.g., trifluoroacetic acid, acetate, benzoic acid, citric acid, lactic acid, maleic acid, gluconic acid, methanesulfonic acid, 4-toluenesulfonic acid, tartaric acid, fumaric acid, etc.). These types of pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, maleate, tartrate, methanesulfonate, propanesulfonate, naphthalen-1-sulfonate, naphthalen-2-sulfonate, mandelate, etc.

The acid addition salt according to the present invention may be prepared by a conventional method, for example, may be prepared by dissolving the derivative of Formula 1 in an organic solvent (e.g., methanol, ethanol, acetone, methylene chloride, acetonitrile, etc.), and adding an organic or inorganic acid to filter and dry the precipitate; or may be prepared by distilling the solvent and excess acid under reduced pressure, drying, and crystallizing in an organic solvent.

In addition, a pharmaceutically acceptable metal salt may be prepared using a base. An alkali metal or alkaline earth metal salt is obtained, for example, by dissolving a compound in an excess alkali metal hydroxide or alkaline earth metal hydroxide solution, filtering the undissolved compound salt, and evaporating and drying the filtrate. In this case, it is pharmaceutically suitable to prepare a sodium, potassium, or calcium salt as the metal salt. In addition, the corresponding salt is obtained by reacting an alkali metal or alkaline earth metal salt with a suitable negative salt (e.g., silver nitrate).

Furthermore, the present invention includes not only the compound represented by Formula 1 and a pharmaceutically acceptable salt thereof, but also solvates, optical isomers, hydrates, etc. which can be prepared therefrom.

The term "hydrate" refers to a compound of the present invention, which contains a stoichiometric or non-stoichiometric amount of water bound by a non-covalent intermolecular force, or a salt thereof. The hydrate of the compound represented by Formula 1 of the present invention may contain a stoichiometric or non-stoichiometric amount of water bound by a non-covalent intermolecular force. The hydrate may contain 1 equivalent or more, preferably 1-5 equivalents of water. Such a hydrate may be prepared by crystallizing the compound represented by Formula 1 of the present invention, a isomer thereof, or a pharmaceutically acceptable salt thereof from water or a water-containing solvent.

The term "solvate" refers to the compound of the present invention or a salt thereof that contains a stoichiometric or non-stoichiometric amount of a solvent bound by a non-covalent intermolecular force. Preferred solvents as such include solvents that are volatile, non-toxic, and/or suitable for administration to humans.

The term "isomer" refers to compounds of the present invention or salts thereof that have the same chemical formula or molecular formula but differ structurally or sterically. These isomers include structural isomers (e.g., tautomers, etc.), R or S isomers having an asymmetric carbon center, stereoisomers (*e*.*g*., geometric isomers (*trans, cis*)*,* and optical isomers (enantiomers). All of these isomers and mixtures thereof are also included within the scope of the present invention.

The compound represented by Formula 1 according to the present invention may be prepared, as shown in Reaction 1 below, may be prepared according to a preparation method which includes preparing a compound represented by Formula 1 by reacting a compound represented by Formula 2 with a compound represented by Formula 3.

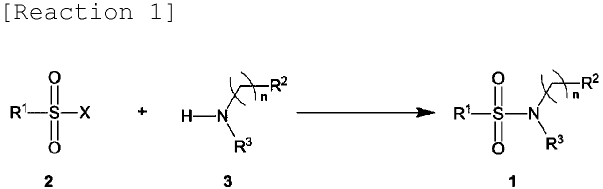

In Reaction 1,
R¹, R², R³ and n are the same as defined in Formula 1; and
X is halogen.

The preparation method of Reaction 1 is a method of reacting the halogen of the compound represented by Formula 2 and the amine of the compound represented by Formula 3 to form a sulfonamide bond and to prepare the compound represented by Formula 1. The preparation method may use reaction conditions well-known in the art and may be performed according to the Examples of the present invention, but this is only an illustration and is not limited thereto.

Another aspect of the present invention provides a pharmaceutical composition for use in preventing or treating mental illness, containing the compound represented by Formula 1, an optical isomer thereof or a pharmaceutically acceptable salt thereof, as an active ingredient.
The compound can inhibit the reuptake of serotonin, norepinephrine, and dopamine.
The mental illness may be at least one selected from the group consisting of bulimia nervosa, mood disorder, depression, atypical depression, depression secondary to pain, major depressive disorder, dysthymic disorder, bipolar disorder, bipolar I disorder, bipolar II disorder, cyclothymic disorder, mood disorder due to a general medical condition, substance-induced mood disorder, pseudo dementia, Ganger syndrome, obsessive-compulsive disorder, panic disorder, panic disorder without agoraphobia, panic disorder with agoraphobia, agoraphobia without history of panic disorder, panic attacks, memory deficit, memory loss, attention deficit hyperactivity disorder, obesity, anxiety, generalized anxiety disorder, eating disorder, Parkinson's disease, Parkinson's symptoms, dementia, aging dementia, senile dementia, Alzheimer's disease, Down syndrome, acquired immunodeficiency syndrome dementia, memory dysfunction in aging, specific phobia, social phobia, social anxiety disorder, post-traumatic stress disorder, acute stress disorder, chronic stress disorder, drug addiction, drug abuse, drug abuse tendency, cocaine abuse, nicotine abuse, tobacco abuse, alcohol addiction, alcoholism, pathological kleptomaniac, withdrawal syndrome due to withdrawal of intoxicating substances, pain, chronic pain, inflammatory pain, neuropathic pain, diabetic neuropathic pain, migraine, tension-type headache, chronic tension-type headache, depression-related pain, fibromyalgia, arthritis, osteoarthritis, rheumatoid arthritis, back pain, cancer pain, irritable bowel pain, irritable bowel syndrome, postoperative pain, postmastectomy pain syndrome (PMPS), poststroke pain, drug-induced neuropathy, diabetic neuropathy, pain sustained by the sympathetic nervous system, trigeminal neuralgia, toothache, facial muscle pain, phantom limb pain, anorexia, premenstrual syndrome, premenstrual dysphoric disorder, late luteal phase syndrome, posttraumatic syndrome, chronic fatigue syndrome, persistent vegetative state, urinary incontinence, stress incontinence, urge incontinence, nocturnal incontinence, sexual dysfunction, premature ejaculation, erectile difficulty, erectile dysfunction, female premature orgasm, restless legs syndrome, periodic limb movement disorder, eating disorder, anorexia nervosa, sleep disorder, pervasive developmental disorder, autism, Asperger's disorder, Rett disorder, childhood disintegrative disorder, learning disorder, motor ability disorder, mutism, trichotillomania, narcolepsy, post-stroke depression, stroke-induced brain injury, stroke-induced nerve injury, Tourette syndrome, tinnitus, tic disorder, body dysmorphic disorder, oppositional defiant disorder, and post-stroke disorder.

The compound represented by Formula 1 according to the present invention is excellent in the reuptake inhibitory effect of serotonin, norepinephrine, and dopamine, and thus can be useful in the treatment of mental illness (see Experimental Example 1 and Table 17).

In the pharmaceutical composition according to the present invention, the compound represented by Formula 1 or a pharmaceutically acceptable salt thereof may be administered in various oral and parenteral dosage forms during clinical administration, and more preferably, a parenteral dosage forms. When formulated, the pharmaceutical composition is prepared using diluents or excipients such as commonly used fillers, extenders, binders, wetting agents, disintegrants, and surfactants. Solid formulations for oral administration include tablets, pills, powders, granules, capsules, etc., and such solid formulations are prepared by mixing at least one excipient, for example, starch, calcium carbonate, sucrose or lactose, gelatin, etc. with one or more compounds. In addition to simple excipients, lubricants such as magnesium stearate and talc are also used. Liquid formulations for oral administration include suspensions, solutions for internal use, emulsions, syrups, etc., and in addition to water and liquid paraffin, which are commonly used simple diluents, various excipients such as wetting agents, sweeteners, fragrances, and preservatives, may be included. Formulations for parenteral administration include sterilized aqueous solutions, non-aqueous solutions, suspensions, and emulsions. As non-aqueous solvents and suspending solvents, propylene glycol, polyethylene glycol, vegetable oils (e.g., olive oil), injectable esters (e.g., ethyl oleate), etc. may be used.

The compound represented by Formula 1 or a pharmaceutically acceptable salt thereof may be administered in various oral and parenteral dosage forms during clinical administration. When formulated, the pharmaceutical composition is prepared using diluents or excipients such as commonly used fillers, extenders, binders, wetting agents, disintegrants, and surfactants. Solid formulations for oral administration include tablets, pills, powders, granules, capsules, etc., and such solid formulations are prepared by mixing at least one excipient, for example, starch, calcium carbonate, sucrose or lactose, gelatin, etc. with one or more compounds. In addition to simple excipients, lubricants such as magnesium stearate and talc are also used. Liquid formulations for oral administration include suspensions, solutions for internal use, emulsions, syrups, etc., and in addition to water and liquid paraffin, which are commonly used simple diluents, various excipients such as wetting agents, sweeteners, fragrances, and preservatives, may be included. Formulations for parenteral administration include sterilized aqueous solutions, non-aqueous solutions, suspensions, and emulsions. As non-aqueous solvents and suspending solvents, propylene glycol, polyethylene glycol, vegetable oils (e.g., olive oil), injectable esters (e.g., ethyl oleate), etc. may be used.

The pharmaceutical composition containing the compound represented by Formula 1 or a pharmaceutically acceptable salt thereof, as an active ingredient may be administered parenterally, and the parenteral administration is performed by subcutaneous injection, intravenous injection, intramuscular injection, or intrathoracic injection.

In this case, to be formulated into a dosage form for parenteral administration, the compound represented by Formula 1 or a pharmaceutically acceptable salt thereof may be mixed in water along with a stabilizer or buffer to prepare a solution or suspension, which may be prepared in unit dosage form in an ampoule or vial. The composition may be sterilized and/or contain preservatives, stabilizers, wetting agents or emulsification accelerators, salts and/or buffers for the control of osmotic pressure, and other therapeutically useful substances, and may be formulated according to the conventional method such as mixing, granulation, or coating method.

Dosage forms for oral administration include, for example, tablets, pills, hard/soft capsules, liquids, suspensions, emulsifiers, syrups, granules, elixirs, troches, etc., and these dosage forms contain diluents (e.g., lactose, dextrose, sucrose, mannitol, sorbitol, cellulose, and/or glycine), lubricants (e.g., silica, talc, stearic acid and a magnesium or calcium salt thereof, and/or polyethylene glycol) in addition to active ingredients. Tablets may contain binders (e.g., magnesium aluminum silicate, starch paste, gelatin, methylcellulose, sodium carboxymethylcellulose and/or polyvinylpyrrolidine); optionally contain disintegrants (e.g., starch, agar, alginic acid or a sodium salt thereof) or effervescent mixtures, and/or absorbents, colorants, flavors, and sweeteners.

The pharmaceutical composition for use in preventing or treating mental illness containing the compound represented by Formula 1, an optical isomer thereof or pharmaceutically acceptable salt thereof, as an active ingredient may be administered as an individual therapeutic agent or used in combination with other therapeutic agents in use.

Still another aspect of the present invention provides a triple reuptake inhibitor of serotonin, norepinephrine, and dopamine for use in preventing or treating mental illness, containing the compound represented by Formula 1 above, an isomer thereof, a solvate thereof, a hydrate thereof, or a pharmaceutically acceptable salt thereof, as an active ingredient.

Yet another aspect of the present invention provides a health functional food composition for use in preventing or improving mental illness containing the compound represented by Formula 1 above, an isomer thereof, a solvate thereof, a hydrate thereof, or a pharmaceutically acceptable salt thereof, as an active ingredient.

Further, another aspect of the present invention provides a method for use in preventing or treating mental illness, the method including administering a pharmaceutical composition or health functional food composition containing the compound represented by Formula 1 above, an isomer thereof, a solvate thereof, a hydrate thereof, or a pharmaceutically acceptable salt thereof, as an active ingredient to a subject in need thereof.

Yet still another aspect of the present invention provides a use of a pharmaceutical composition or health functional food composition containing the compound represented by Formula 1 above, an isomer thereof, a solvate thereof, a hydrate thereof, or a pharmaceutically acceptable salt thereof, as an active ingredient in the prevention or treatment of mental illness.

### MODES FOR CARRYING OUT THE INVENTION

Hereinafter, the present invention will be described in detail by Examples and Experimental Examples.

However, the following Examples and Experimental Examples are merely illustrative of the present invention, and the contents of the present invention are not limited to the following Examples and Experimental Examples.

### <Preparation Example 1> Preparation of 2-(4-benzylpiperidin-1-yl)ethaneamine 2HCl salt

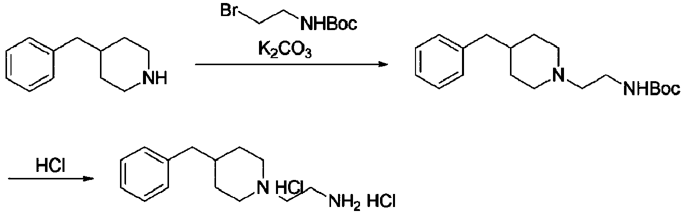

Step 1: 4-Benzylpiperidine (1 eq) and K₂CO₃ (1.5 eq) were dissolved in DMF, followed by stirring at room temperature for 5 minutes. To the reaction product, tert-butyl(2-bromoethyl) carbamate (1.2 eq) was added dropwisely, followed by stirring at 60°C for 12 hours. To the reaction product, tert-butyl(2-bromoethyl) carbamate (1.2 eq) was added dropwisely once more, followed by stirring at 60°C for 12 hours. After finishing the reaction, a 1 N HCl aqueous solution was added dropwisely and subjected to extraction with dichloromethane. An organic layer was washed with H₂O and brine in order, and dried over anhydrous Na₂SO₄, and the solvent was removed under a reduced pressure. Then, the reaction mixture was separated and purified by MPLC to obtain tert-butyl(2-(4-benzylpiperidin-1-yl)ethyl)carbamate.

Step 2: Tert-butyl(2-(4-benzylpiperidin-1-yl)ethyl)carbamate was directly treated with a 4 M HCl dioxane solution, followed by stirring at room temperature for 20 minutes. After finishing the reaction, an excessive amount of HCl was removed under a reduced pressure. A 2-(4-benzylpiperidin-1-yl)ethaneamine 2HCl salt was obtained as a white solid.

Compounds of Preparation Example 2 and Preparation Example 3 were obtained by the same method as Preparation Example 1 except for using R listed in Table 1 instead of 4-benzylpiperirine in Step 1.

**[Table 1]**

| **Preparation Example** | **R** | **Chemical structure** |
|---|---|---|
| 2 | 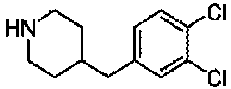 | 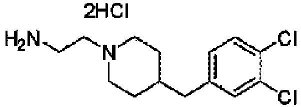 |
| 3 | 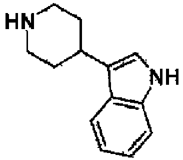 | 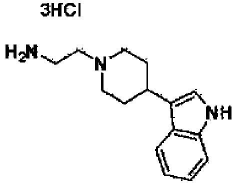 |

### <Preparation Example 4> Preparation of 2-(4-(5-methyl-1H-indol-3-yl)piperidin-1-yl)ethan-1-amine

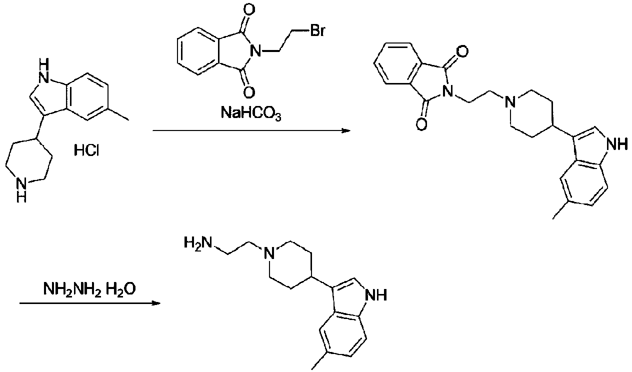

Step 1: A 5-methyl-3-(piperidin-4-yl)-1H-indole HCl salt (1 eq), 2-(2-bromoethyl)isoindolidin-1,3-dione (1 eq) and NaHCO₃ (1.1 eq) were dissolved in DMF, followed by stirring at 70°C for 3 hours. After finishing the reaction, ice was added, and a solid obtained by filtering was separated and purified by MPLC to obtain 2-(2-(4-(5-methyl-1H-indol-3-yl)piperidin-1-yl)ethyl)isoindolidin-1,3-dione.

Step 2: The 2-(2-(4-(5-methyl-1H-indol-3-yl)piperidin-1-yl)ethyl)isoindolidin-1,3-dione (1 eq) synthesized in Step 1 was dissolved in ethanol, a hydrazine hydrate (1.4 eq) was added thereto dropwisely, followed by refluxing and stirring for 2 hours. After cooling to room temperature, the reaction product was filtered, and the solvent in a filtrate collected was removed under a reduced pressure. A solid compound was dissolved in a 1 M NaOH aqueous solution and subjected to extraction with ethyl acetate, an organic layer was washed with H₂O and brine in order and dried over anhydrous Na₂SO₄, and the solvent was removed under a reduced pressure. 2-(4-(5-Methyl-1H-indol-3-yl)piperidin-1-yl)ethan-1-amine was obtained without additional purification.

Compounds of Preparation Example 5 and Preparation Example 6 were obtained by the same method as Preparation Example 4 except for using R listed in Table 2 instead of the 5-methyl-3-(piperidin-4-yl)-1H-indole HCl salt.

**[Table 2]**

| **Preparation Example** | **R** | **Chemical structure** |
|---|---|---|
| 5 | 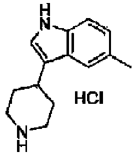 | 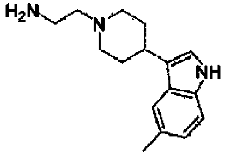 |
| 6 | 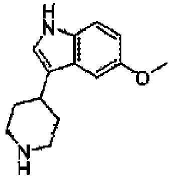 | 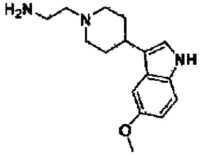 |

### <Preparation Example 7> Preparation of 3-(4-benzylpiperidin-1-yl)propan-1-amine

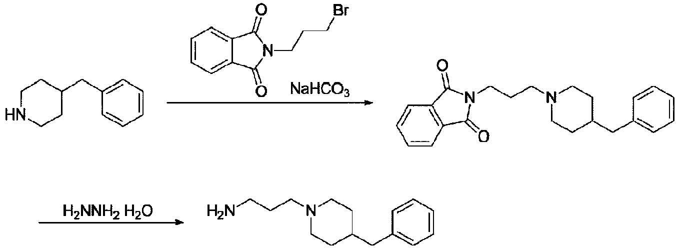

Step 1: 4-Benzylpiperidine (1 eq), N-(3-bromopropyl)phthalimide (1 eq) and NaHCO₃ (1.1 eq) were dissolved in DMF, followed by stirring at 70°C for 3 hours. After finishing the reaction, ice was added, and a solid obtained by filtering was separated and purified by MPLC to obtain 2-(3-(4-benzylpiperidin-1-yl)propyl)isoindolin-1,3-dione.

Step 2: The 2-(3-(4-benzylpiperidin-1-yl)propyl)isoindolin-1,3-dione (1 eq) synthesized in Step 1 was dissolved in ethanol, and a hydrazine hydrate (1.4 eq) was added thereto dropwisely, followed by refluxing and stirring for 2 hours. After cooling to room temperature, the reaction product was filtered, and the solvent in a filtrate collected was removed under a reduced pressure. A solid compound was dissolved in a 1 M NaOH aqueous solution and subjected to extraction with ethyl acetate, an organic layer was washed with H₂O and brine in order and dried over anhydrous Na₂SO₄, and the solvent was removed under a reduced pressure. 3-(4-Benzylpiperidin-1-yl)propan-1-amine was obtained without additional purification.

Compounds of Preparation Example 8 and Preparation Example 9 were obtained by the same method as Preparation Example 7 except for using R listed in Table 3 instead of the 4-benzylpiperidine.

**[Table 3]**

| **Preparation Example** | **R** | **Chemical structure** |
|---|---|---|
| 8 | 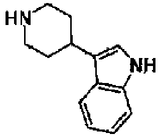 | 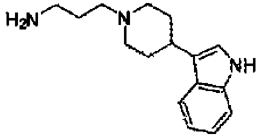 |
| 9 | 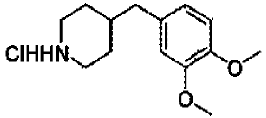 | 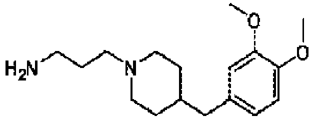 |

### <Example 1> Preparation of N-(2-(4-benzylpiperidin-1-yl)ethyl)-4-hydroxybenzenesulfonamide

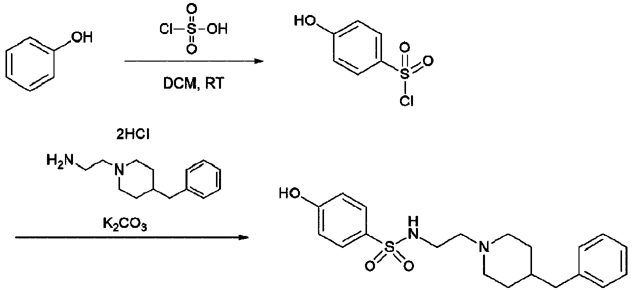

Step 1: Phenol (1 eq) was dissolved in DCM, and chlorosulfonic acid (4 eq) was added dropwisely at 0°C, followed by stirring at room temperature for 3 hours. After finishing the reaction, ice was added. The reaction solution was extracted with dichloromethane and neutralized using a saturated sodium carbonate aqueous solution. An organic layer was washed with H₂O and brine in order, and dried over anhydrous Na₂SO₄, and the solvent was removed under a reduced pressure. 4-(Hydroxybenzen-1-sulfonyl chloride was obtained without separate purification.

Step 2: The 4-(hydroxybenzen-1-sulfonyl chloride (1 eq) obtained in Step 1 was dissolved in DCM, and a 2-(4-benzylpiperidin-1-yl)ethaneamine 2HCl salt (1.5 eq) and K₂CO₃ (1.5 eq) were added thereto dropwisely. Stirring was performed at room temperature for 3 hours. After finishing the reaction, the resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 1.

The compounds of Example 2 and Example 3 were obtained by the same method as Example 1 except for using R listed in Table 4 instead of the phenol in Step 1.

**[Table 4]**

| **Example** | **R** | **Chemical structure of Example** |
|---|---|---|
| 2 | 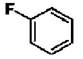 | 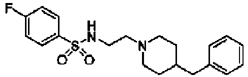 |
| 3 | 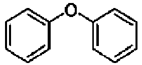 | 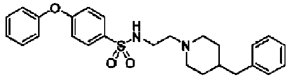 |

### <Example 4> Preparation of 4-butoxy-N-(2-(4-(3,4-dichlorobenzyl)piperidin-1-yl)ethyl)benzenesulfonamide

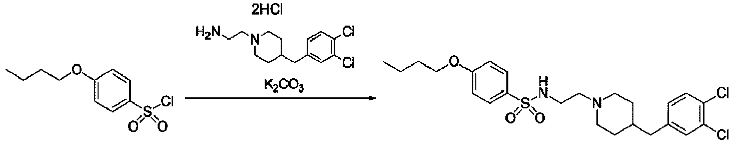

4-Butoxy benzenesulfonyl chloride (1 eq) was dissolved in DCM, and a 2-(4-(3,4-dichlorobenzylpiperidin-1-yl) ethaneamine 2HCl salt (2 eq) and K₂CO₃ (2 eq) were added thereto dropwisely. Stirring was performed at room temperature for 3 to 6 hours. After finishing the reaction, the resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 4.

The compounds of Example 5 to Example 9 were obtained by the same method as Example 4 except for using R listed in Table 5 instead of the 2-(4-(3,4-dichlorobenzylpiperidin-1-yl)ethaneamine 2HCl salt in Step 1.

**[Table 5]**

| **Example** | **R** | **Chemical structure of Example** |
|---|---|---|
| 5 | 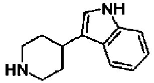 | 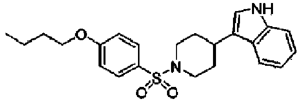 |
| 6 | 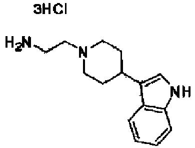 | 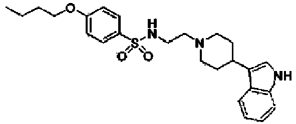 |
| 7 | 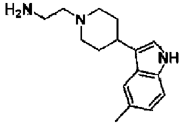 | 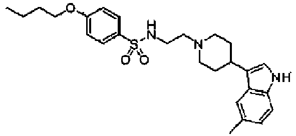 |
| 8 | 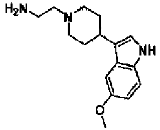 | 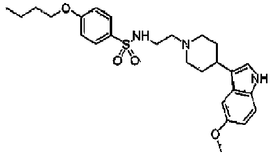 |
| 9 | 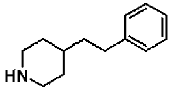 | 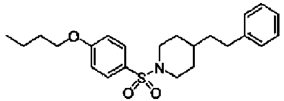 |

### <Example 10> Preparation of N-(3-(4-benzylpiperidin-1-yl)propyl)-4-butoxybenzenesulfonamide

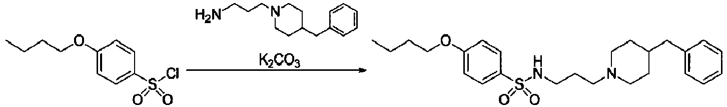

4-Butoxy benzenesulfonyl chloride (1 eq) was dissolved in DCM, and a 3-(4-benzylpiperidin-1-yl)propan-1-amine (2 eq) and K₂CO₃ (2 eq) were added thereto dropwisely. Stirring was performed at room temperature for 3 to 6 hours. After finishing the reaction, the resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain the compound of Example 10.

The compounds of Example 11 and Example 12 were obtained by the same method as Example 10 except for using R listed in Table 6 instead of the 3-(4-benzylpiperidin-1-yl)propan-1-amine.

**[Table 6]**

| **Example** | **R** | **Chemical structure of Example** |
|---|---|---|
| 11 | 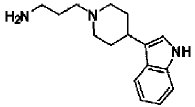 | 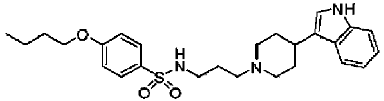 |
| 12 | 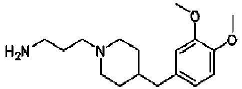 | 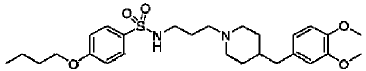 |

The compounds of Example 13 and Example 14 were obtained by the same method as Example 10 except for using R listed in Table 7 instead of the 4-butoxybenzenesulfonyl chloride.

**[Table 7]**

| **Example** | **R** | **Chemical structure of Example** |
|---|---|---|
| 13 | 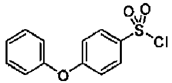 | 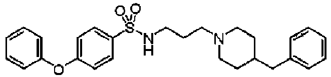 |
| 14 | 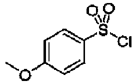 | 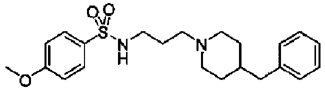 |

### <Example 15> Preparation of 4-amino-N-(3-(4-benzylpiperidin-1-yl)propyl)benzenesulfonamide

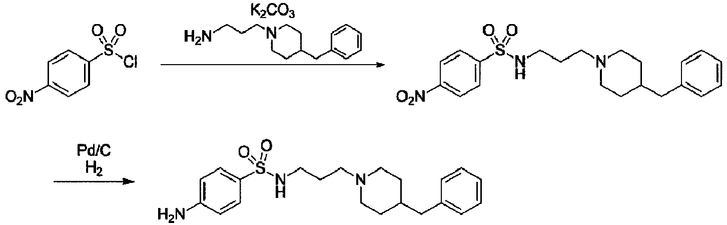

Step 1: 4-Nitrobenzenesulfonyl chloride (1 eq) was dissolved in DCM, and a 3-(4-benzylpiperidin-1-yl)propan-1-amine (1.1 eq) and K₂CO₃ (1.5 eq) were added thereto dropwisely. Stirring was performed at room temperature for 12 hours. After finishing the reaction, the resultant was extracted with dichloromethane, an organic layer was washed with H₂O and brine in order, and dried over anhydrous Na₂SO₄, and the solvent was removed under a reduced pressure. Then, the reaction mixture was separated and purified by MPLC to obtain N-(3-(4-benzylpiperidin-1-yl)propyl)-4-nitrobenzenesulfonamide.

Step 2: The N-(3-(4-benzylpiperidin-1-yl)propyl)-4-nitrobenzenesulfonamide (1 eq) synthesized in Step 1 was dissolved in methanol, and Pd/C (10% wt) was added dropwisely, followed by stirring under a hydrogen stream for 3 hours. After finishing the reaction, celite filtering was performed, and the solvent was removed under a reduced pressure. Then, the reaction mixture was separated and purified by MPLC to obtain the compound of Example 15.

### <Example 16> Preparation of N-(3-(4-benzylpiperidin-1-yl)propyl)-4-(butylamino)benzenesulfonamide

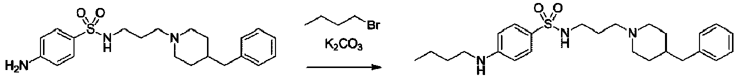

4-Amino-N-(3-(4-benzylpiperidin-1-yl)propyl)benzenesulfonamide (DN205914) (1 eq) and K₂CO₃ (2 eq) were dissolved in DMF, followed by stirring for 5 minutes. To the reaction mixture, 1-bromobutane (1.2 eq) was added dropwisely, followed by stirring at room temperature for 6 hours. After finishing the reaction, water was added, and the resultant was extracted with ethyl acetate. An organic layer was washed with H₂O and brine in order, and dried over anhydrous Na₂SO₄, and the solvent was removed under a reduced pressure. The reaction mixture was separated and purified by MPLC to obtain the compound of Example 16.

The compounds of Example 17 and Example 18 were obtained by the same method as Example 16 except for using R listed in Table 8 instead of the 1-bromobutane.

**[Table 8]**

| **Example** | **R** | **Chemical structure of Example** |
|---|---|---|
| 17 | 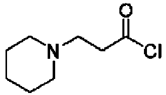 | 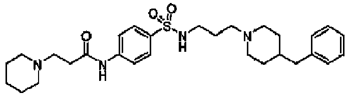 |
| 18 | 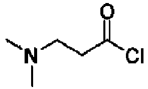 | 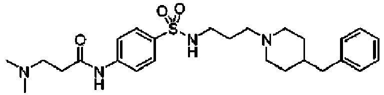 |

### <Example 19> Preparation of N-(3-(4-benzylpiperidin-1-yl)propyl)-4-butoxybenzenesulfonamide

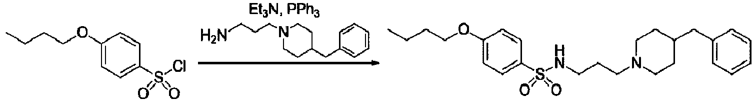

4-Butoxybenzenesulfonyl chloride (1 eq) was dissolved in DCM, and Et₃N (10 eq) was added dropwisely at 0°C. A mixture solution of triphenylphosphine (PPh₃) (1 eq) and 3-(4-benzylpiperidin-1-yl)propan-1-amine (1 eq) dissolved in DCM, was slowly added dropwisely to the reactant using a syringe pump over 1 hour. After finishing the dropwise addition, the product was checked using TLC, and a saturated NaHCO₃ aqueous solution was added to terminate the reaction. After finishing the reaction, the resultant was extracted with dichloromethane. An organic layer was washed with a saturated NaHCO₃ aqueous solution and brine in order, and dried over anhydrous Na₂SO₄, and the solvent was removed under a reduced pressure. Then, the reaction mixture was separated and purified by MPLC to obtain the compound of Example 19.

### <Example 20> Preparation of 4-butoxy-N-(3-(4-(3,4-dichlorobenzyl)piperidin-1-yl)propyl)benzenesulfonamide

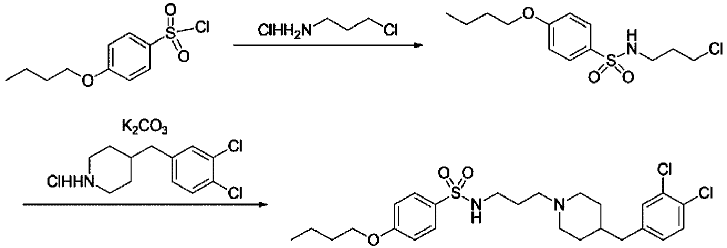

Step 1: 4-Butoxybenzenesulfonyl chloride (1 eq) and a 3-chloropropan-1-amine HCl salt (1.2 eq) were dissolved in DMF, and triethylamine (TEA) (4 eq) was added thereto dropwisely. Stirring was performed at 35°C for 12 hours. After finishing the reaction, the resultant was extracted with ethyl acetate, an organic layer was washed with H₂O and brine in order, and dried over anhydrous Na₂SO₄, and the solvent was removed under a reduced pressure. 4-Butoxy-N-(3-chloropropyl)benzenesulfonamide was obtained without purification.

Step 2: The 4-butoxy-N-(3-chloropropyl)benzenesulfonamide (1 eq) synthesized in Step 1 was dissolved in DMF, and a 4-(3,4-dichlorobenzyl)piperidine HCl salt (1.5 eq) and K₂CO₃ (4 eq) were added thereto dropwisely, followed by stirring at 70°C for 12 hours. After finishing the reaction, the resultant was extracted with ethyl acetate, an organic layer was washed with H₂O and brine in order, and dried over anhydrous Na₂SO₄, and the solvent was removed under a reduced pressure. The reaction mixture was separated and purified by MPLC to obtain the compound of Example 20.

The compounds of Example 21 to Example 33 were obtained by the same method as Example 20 except for using R listed in Table 9 instead of the 4-(3,4-dichlorobenzyl)piperidine HCl salt in Step 2.

**[Table 9]**

| **Example** | **R** | **Chemical structure of Example** |
|---|---|---|
| 21 | 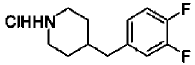 | 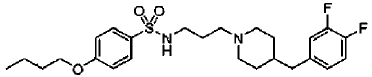 |
| 22 | 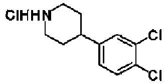 | 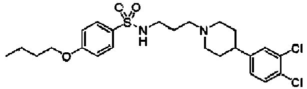 |
| 23 | 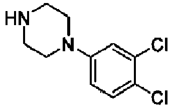 | 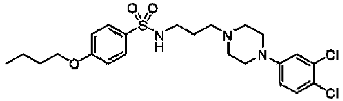 |
| 24 | 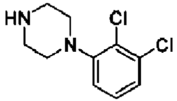 | 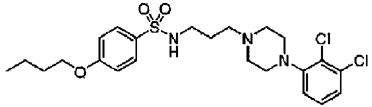 |
| 25 | 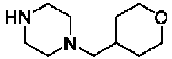 | 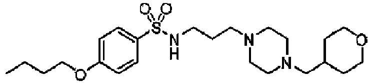 |
| 26 | 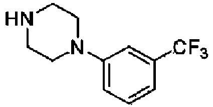 | 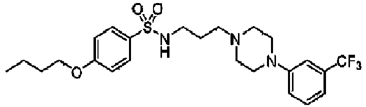 |
| 27 | 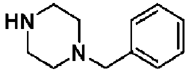 | 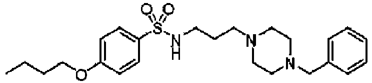 |
| 28 | 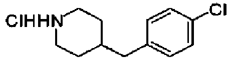 | 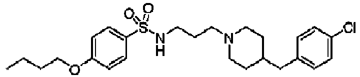 |
| 29 | 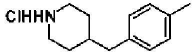 | 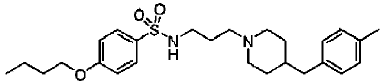 |
| 30 | 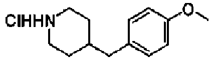 | 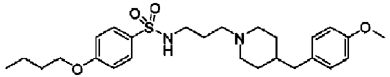 |
| 31 | 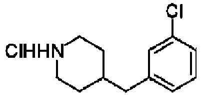 | 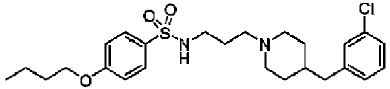 |
| 32 | 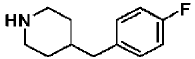 | 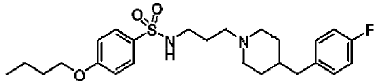 |
| 33 | 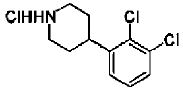 | 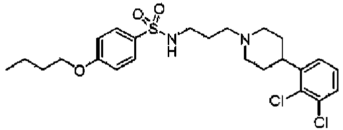 |

The compound of Example 34 was obtained by the same method as Example 20 except for using R₁ listed in Table 10 instead of the 4-butoxybenzenesulfonyl chloride in Step 1 and using R₂ listed in Table 10 instead of the 4-(3,4-dichlorobenzyl)piperidine HCl salt in Step 2.

**[Table 10]**

| **Example** | **R₁** | **R₂** | **Chemical structure of Example** |
|---|---|---|---|
| 34 | 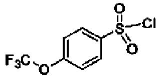 | 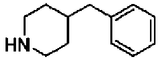 | 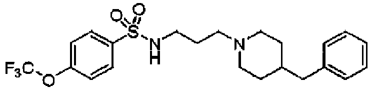 |

### <Example 35> Preparation of N-(3-(4-benzylpiperidin-1-yl)propyl)-5-butoxypyridin-2-sulfonamide

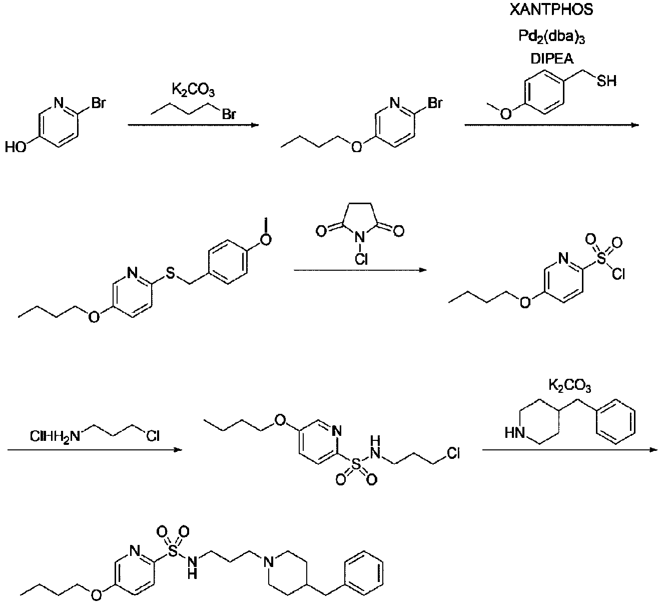

Step 1: 6-Bromopyridin-3-ol (1 eq) was dissolved in DMF, and 1-bromobutane (1.5 eq) and K₂CO₃ (2 eq) were added thereto dropwisely, followed by stirring at 80°C for 2 hours. After finishing the reaction, water was added, and the resultant was extracted with ethyl acetate. An organic layer was washed with H₂O and brine in order, and dried over anhydrous Na₂SO₄, and the solvent was removed under a reduced pressure. The reaction mixture was separated and purified by MPLC to obtain 2-bromo-5-butoxypyridine.

Step 2: The 2-bromo-5-butoxypyridine (1 eq) synthesized in Step 1 was dissolved in dioxane, and (4-methoxyphenyl)methanethiol (1.1 eq) and DIPEA (3 eq) were added thereto dropwisely at room temperature. After degassing and charging a nitrogen gas, Pd₂(dba)₃ (0.025 eq) and XPhos (0.05 eq) were added. The reaction mixture was reacted using Biotage microwave at 150°C for 30 minutes. After finishing the reaction, the resultant was extracted with ethyl acetate, an organic layer was washed with H₂O and brine in order, and dried over anhydrous Na₂SO₄, and the solvent was removed under a reduced pressure. 5-Butoxy-2-((4-methoxybenzyl)thio)pyridine was obtained without purification.

Step 3: The 5-butoxy-2-((4-methoxybenzyl)thio)pyridine (1 eq) synthesized in Step 2 was dissolved in a solution of acetic acid: water (1: 1), and N-chlorosuccinimide (4 eq) was added thereto dropwisely, followed by stirring at room temperature for 1 hour. After finishing the reaction, the resultant was extracted with ethyl acetate, an organic layer was washed with H₂O and brine in order, and dried over anhydrous Na₂SO₄, and the solvent was removed under a reduced pressure. 5-Butoxypyridin-2-sulfonyl chloride was obtained without purification.

Step 4: The 5-butoxypyridin-2-sulfonyl chloride (1 eq) synthesized in Step 3 was dissolved in DMF, and a 3-chloropropan-1-amine HCl salt (1.2 eq) and TEA (4 eq) were added thereto dropwisely, followed by stirring at room temperature for 12 hours. After finishing the reaction, celite filtering was performed, and the solvent was removed under a reduced pressure. Then, the reaction mixture was extracted with ethyl acetate, an organic layer was washed with H₂O and brine in order, and dried over anhydrous Na₂SO₄, and the solvent was removed under a reduced pressure. 5-Butoxy-N-(3-chloropropyl)pyridin-2-sulfonamide was obtained without purification.

Step 5: The 5-butoxy-N-(3-chloropropyl)pyridin-2-sulfonamide (1 eq) synthesized in Step 4 was dissolved in DMF, and 4-benzylpiperidine (1.5 eq) and K₂CO₃ (3 eq) were added thereto dropwisely, followed by stirring at 70°C for 12 hours. After finishing the reaction, the resultant was extracted with ethyl acetate, an organic layer was washed with H₂O and brine in order, and dried over anhydrous Na₂SO₄, and the solvent was removed under a reduced pressure. The reaction mixture was separated and purified by MPLC to obtain the compound of Example 35.

### <Example 36> Preparation of N-(2-(4-benzylpiperidin-1-yl)ethyl)-1H-indol-2-sulfonamide

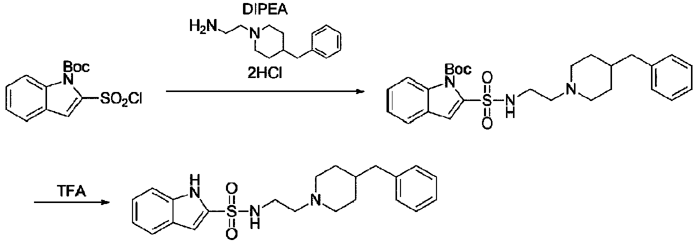

Step 1: Tert-butyl 2-(chlorosulfonyl)-1H-indol-1-carboxylate (1 eq) was dissolved in DMF, and a 2-(4-(3,4-dichlorobenzylpiperidin-1-yl)ethaneamine 2HCl salt (1.5 eq) and DIPEA (7 eq) were added thereto dropwisely. Stirring was performed at room temperature for 1 hour. After finishing the reaction, water was added dropwisely, and the resultant was extracted with ethyl acetate. An organic layer was washed with H₂O and brine in order, and dried over anhydrous MgSO₄, and the solvent was removed under a reduced pressure. Then, the reaction mixture was separated and purified by MPLC to obtain tert-butyl 2-(N-(2-(4-benzylpiperidin-1-yl)ethyl)sulfamoyl)-1H-indol-1-carboxylate.

Step 2: The tert-butyl 2-(N-(2-(4-benzylpiperidin-1-yl)ethyl)sulfamoyl)-1H-indol-1-carboxylate (1 eq) obtained in Step 1 was dissolved in DCM: trifluoroacetic acid (TFA) (1: 1), followed by stirring at room temperature for 30 minutes. After finishing the reaction, the solvent was removed under a reduced pressure. The compound thus dried was dissolved in THF and subjected to stirring at room temperature for 5 minutes. Ethyl acetate was added thereto dropwisely, and an organic layer was washed with a saturated NaHCO₃ aqueous solution and dried over Na₂SO₄. The solvent was removed under a reduced pressure. The compound of Example 36 was obtained without a separate purification process.

The compound of Example 37 was obtained by the same method as Example 36 except for using R listed in Table 11 instead of the 2-(4-(3,4-dichlorobenzenepiperidin-1-yl)ethaneamine 2HCl salt.

**[Table 11]**

| **Example** | **R** | **Structure** |
|---|---|---|
| 37 | 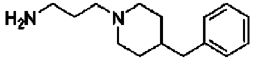 | 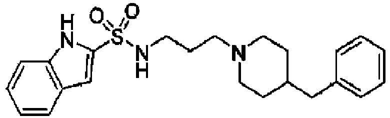 |

### <Example 38> Preparation of N-(2-(4-benzylpiperidin-1-yl)ethyl)naphthalen-2-sulfonamide

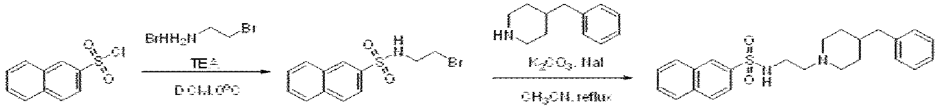

Step 1: Naphthalen-2-sulfonyl chloride (1 eq) and 2-bromoethylamine hydrobromide (1.15 eq) were dissolved in anhydrous dichloromethane, and triethylamine (2.4 eq) was added dropwisely over 10 minutes at 0°C under a nitrogen stream. After stirring at 0°C for 10 minutes, dichloromethane was added for dilution. An organic layer was washed with a 2 M hydrochloric acid aqueous solution and brine in order, and dried over MgSO₄, and the solvent was removed under a reduced pressure. Then, N-(2-bromoethyl)naphthalen-2-sulfonamide was obtained without separate purification.

Step 2: The N-(2-bromoethyl)naphthalen-2-sulfonamide (1 eq) obtained in Step 1, a-benzylpiperidine (1.5 eq), K₂CO₃ (2 eq) and a catalytic amount of sodium iodide were dissolved in acetonitrile, followed by refluxing and stirring until the reaction was finished. After finishing the reaction, filtering was performed using a filter, and the solvent was removed under a reduced pressure. Then, the reaction mixture was separated and purified by MPLC to obtain the compound of Example 38.

The compounds of Example 39 to 53 were obtained by the same method as Example 38 except for using R₁ listed in Table 12 instead of the naphthalen-2-sulfonyl chloride in Step 1 and using R₂ listed in Table 12 instead of the 4-benzylpiperidine in Step 2.

**[Table 12]**

| **Example** | **R₁** | **R₂** | **Chemical structure of Examole** |
|---|---|---|---|
| 39 | 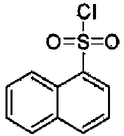 | 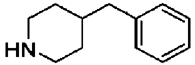 | 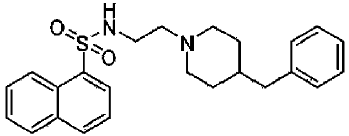 |
| 40 | 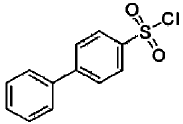 | 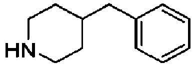 | 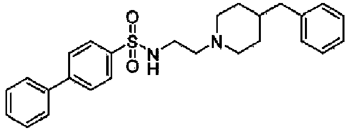 |
| 41 | 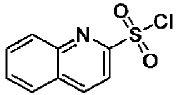 | 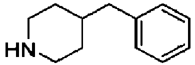 | 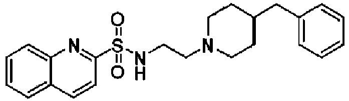 |
| 42 | 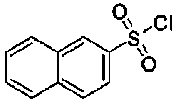 | 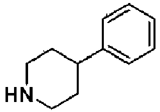 | 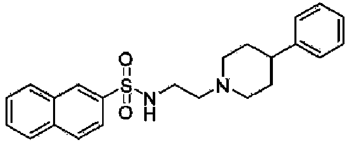 |
| 43 | 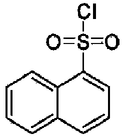 | 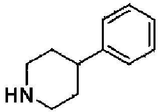 | 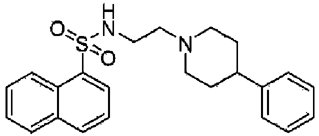 |
| 44 | 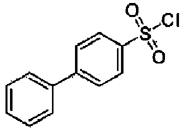 | 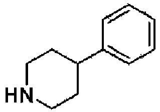 | 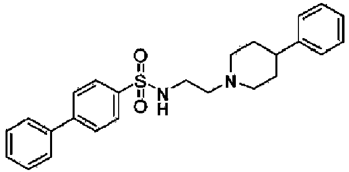 |
| 45 | 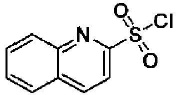 | 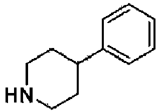 | 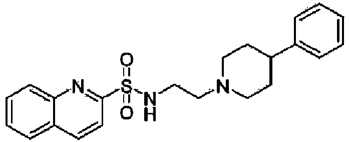 |
| 46 | 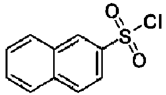 | 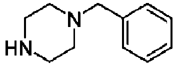 | 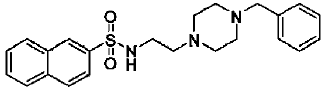 |
| 47 | 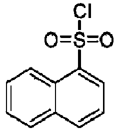 | 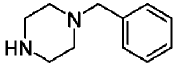 | 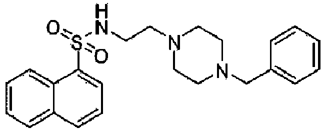 |
| 48 | 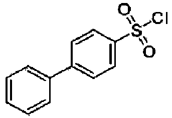 | 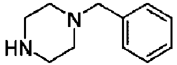 | 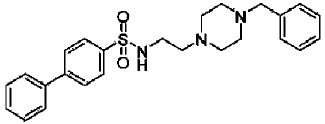 |
| 49 | 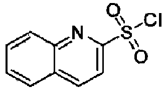 | 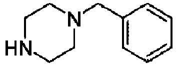 | 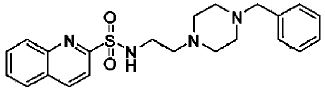 |
| 50 | 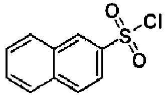 | 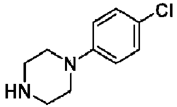 | 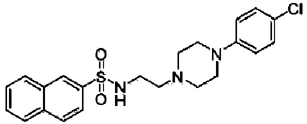 |
| 51 | 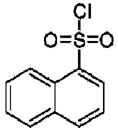 | 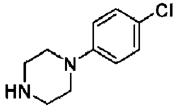 | 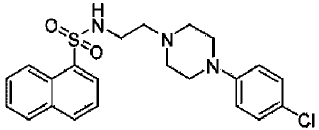 |
| 52 | 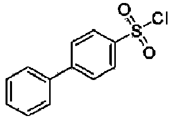 | 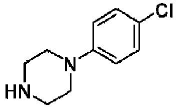 | 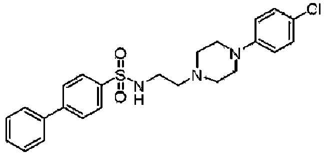 |
| 53 | 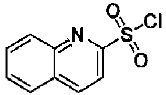 | 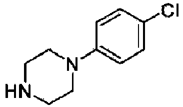 | 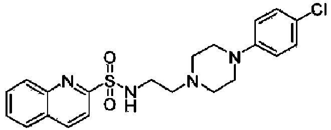 |

### <Example 54> Preparation of N-(3-(4-benzylpiperidin-1-yl)propyl)naphthalen-2-sulfonamide

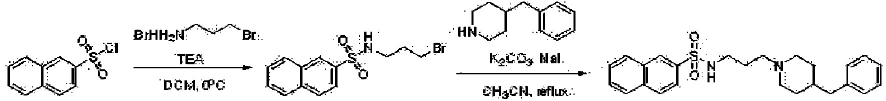

Step 1: Naphthalen-2-sulfonyl chloride (1 eq) and 3-bromopropylamine hydrobromide (1.15 eq) were dissolved in anhydrous dichloromethane, and triethylamine (2.4 eq) was added dropwisely over 10 minutes at 0°C under a nitrogen stream. After stirring at 0°C for 10 minutes, dichloromethane was added for dilution. An organic layer was washed with a 2 M hydrochloric acid aqueous solution and brine in order, and dried over MgSO₄, and the solvent was removed under a reduced pressure. Then, N-(3-bromopropyl)naphthalen-2-sulfonamide was obtained without separate purification.

Step 2: The N-(3-bromopropyl)naphthalen-2-sulfonamide (1 eq) obtained in Step 1, 4-benzylpiperidine (1.5 eq), K₂CO₃ (2 eq) and a catalytic amount of sodium iodide were dissolved in acetonitrile, followed by refluxing and stirring until the reaction was finished. After finishing the reaction, filtering was performed using a filter, and the solvent was removed under a reduced pressure. Then, the reaction mixture was separated and purified by MPLC to obtain the compound of Example 54.

The compounds of Example 55 to 76 were obtained by the same method as Example 54 except for using R₁ listed in Table 13 instead of the naphthalen-2-sulfonyl chloride in Step 1 and using R₂ listed in Table 13 instead of the 4-benzylpiperidine in Step 2.

**[Table 13]**

| **Example** | **R₁** | **R₂** | **Chemical structure of Example** |
|---|---|---|---|
| 55 | 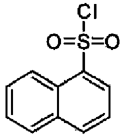 | 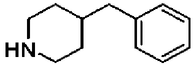 | 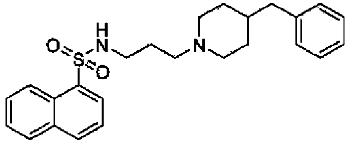 |
| 56 | 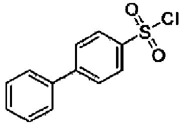 | 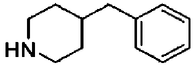 | 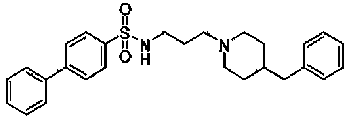 |
| 57 | 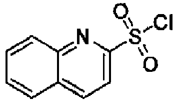 | 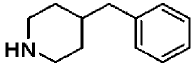 | 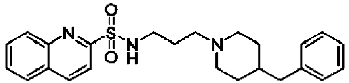 |
| 58 | 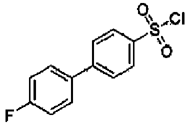 | 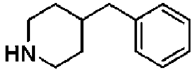 | 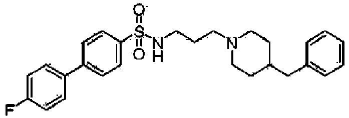 |
| 59 | 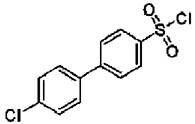 | 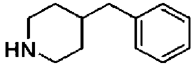 | 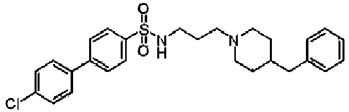 |
| 60 | 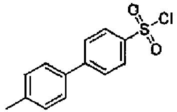 | 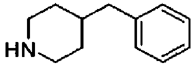 | 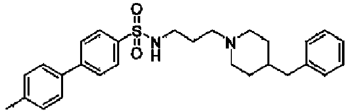 |
| 61 | 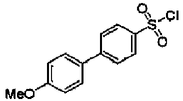 | 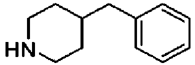 | 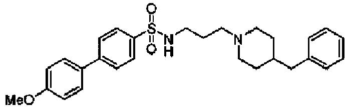 |
| 62 | 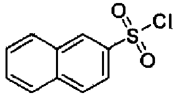 | 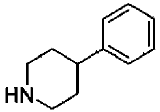 | 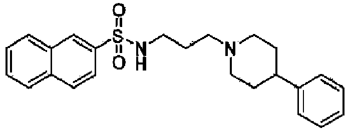 |
| 63 | 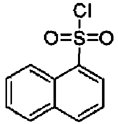 | 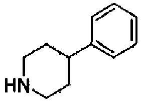 | 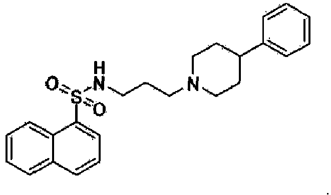 |
| 64 | 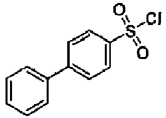 | 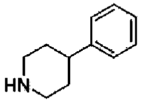 | 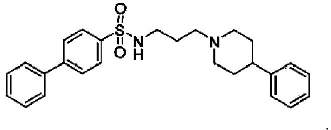 |
| 65 | 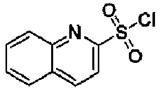 | 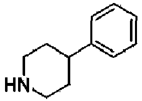 | 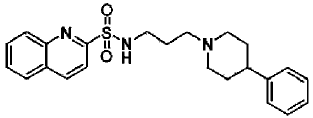 |
| 66 | 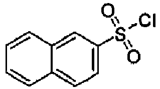 | 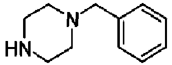 | 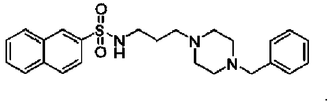 |
| 67 | 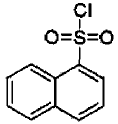 | 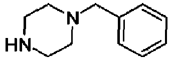 | 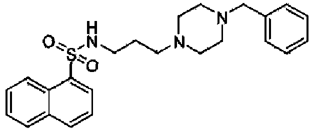 |
| 68 | 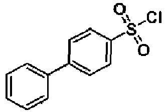 | 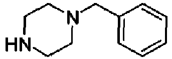 | 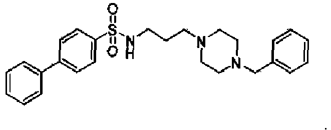 |
| 69 | 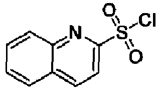 | 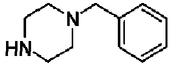 | 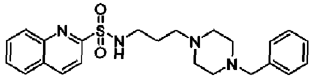 |

### <Example 77> Preparation of N-(4-(4-benzylpiperidin-1-yl)butyl)-[1,1'-biphenyl]-4-sulfonamide

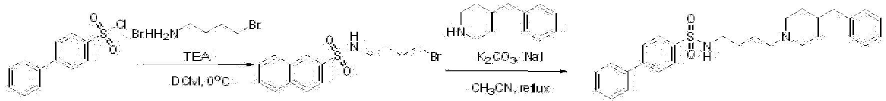

Step 1: [1,1'-Biphenyl]-4-sulfonyl chloride (1 eq) and 4-chlorobutan-1-amine hydrochloride (1.15 eq) were dissolved in anhydrous dichloromethane, and triethylamine (2.4 eq) was added dropwisely over 10 minutes at 0°C under a nitrogen stream. After stirring at 0°C for 10 minutes, dichloromethane was added for dilution. An organic layer was washed with a 2 M hydrochloric acid aqueous solution and brine in order, and dried over MgSO₄, and the solvent was removed under a reduced pressure. Then, N-(4-bromobutyl)naphthalen-2-sulfonamide was obtained without separate purification.

Step 2: The N-(4-bromobutyl)naphthalen-2-sulfonamide (1 eq) obtained in Step 1, 4-benzylpiperidine (1.5 eq), K₂CO₃ (2 eq) and a catalytic amount of sodium iodide were dissolved in acetonitrile, followed by refluxing and stirring until the reaction was finished. After finishing the reaction, filtering was performed using a filter, and the solvent was removed under a reduced pressure. Then, the reaction mixture was separated and purified by MPLC to obtain the compound of Example 77.

The compounds of Examples 78 to 79 were obtained by the same method as Example 77 except for using R listed in Table 14 instead of the 4-benzylpiperidine in Step 2.

**[Table 14]**

| **Example** | **R** | **Chemical Structure of Example** |
|---|---|---|
| 78 | 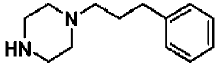 | 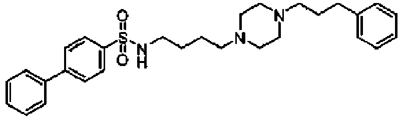 |
| 79 | 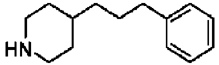 | 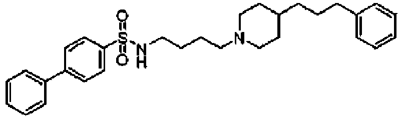 |

The compound names, NMR analysis results and Mass analysis results of Example 1 to Example 79 are summarized and shown in Tables 15 and 16.

**[Table 15]**

| Examp le | Compound name | NMR | Mass |
|---|---|---|---|
| 1 | N- (2- (4-benzylpip eridin-1-yl)ethyl) -4-hydroxybe nzenesulf onamide | ¹H NMR (400 MHz, MeOD) δ 7.72 - 7.66 (m, 2H), 7.26 (t, J = 7.4 Hz, 2H), 7.19 - 7.12 (m, 3H), 6.94 - 6.89 (m, 2H), 2.97 (t, J = 7.0 Hz, 2H), 2.83 (d, J = 11.5 Hz, 2H), 2.54 (d, J = 6.9 Hz, 2H), 2.44 (t, J = 7.0 Hz, 2H), 1.98 (t, J = 11.0 Hz, 2H), 1.61 (d, J = 13.3 Hz, 2H), 1.56 - 1.49 (m, 1H), 1.31 - 1.24 (m, 2H). | 375.05 (M+1) |
| 2 | N-(2-(4-benzylpip eridin-1-yl)ethyl) -4-fluoroben zenesulfo namide | ¹H NMR (400 MHz, CDCl₃) δ 7.77 (d, J = 8.9 Hz, 2H), 7.33 (d, J = 8.2 Hz, 1H), 7.21 (d, J = 1.9 Hz, 1H), 6.95 (d, J = 8.9 Hz, 3H), 5.14 (s, 1H), 4.01 (t, J = 6.5 Hz, 2H), 2.94 (t, J = 5.7 Hz, 2H), 2.57 (d, J = 11.5 Hz, 2H), 2.46 (d, J = 7.0 Hz, 2H), 2.37 - 2.30 (m, 2H), 1.87 - 1.73 (m, 4H), 1.57 - 1.46 (m, 5H), 1.16 (dt, J = 20.2, 7.6 Hz, 2H), 0.98 (t, J = 7.4 Hz, 3H). | 499.10 (M) |
| 3 | N-(2-(4-benzylpip eridin-1-yl)ethyl) -4-phenoxybe nzenesulf onamide | ¹H NMR (400 MHz, CDCl₃) δ 7.67 (d, J = 8.1 Hz, 1H), 7.44 (d, J = 8.3 Hz, 1H), 7.37 - 7.32 (m, 1H), 7.28 (d, J = 7.1 Hz, 1H), 7.19 (t, J = 7.5 Hz, 2H), 7.10 (d, J = 8.1 Hz, 3H), 3.23 - 3.15 (m, 2H), 2.75 (d, J = 11.7 Hz, 2H), 2.51 (d, J = 6.9 Hz, 2H), 2.48 - 2.42 (m, 2H), 1.97 - 1.85 (m, 2H), 1.57 (d, J = 14.1 Hz, 2H), 1.54 - 1.44 (m, 1H), 1.27 (dt, J = 20.5, 10.4 Hz, 2H). | 398.05 (M+1) |
| 4 | 4-butoxy-N-(2-(4-(3,4-dichlorob enzyl) pip eridin-1-yl)ethyl) benzenesu lfonamide | ¹H NMR (400 MHz, CDCl₃) δ 7.91 - 7.85 (m, 2H), 7.29 (d, J = 7.1 Hz, 2H), 7.22 - 7.15 (m, 3H), 7.12 (d, J = 6.9 Hz, 2H), 2.98 - 2.91 (m, 2H), 2.57 (d, J = 11.4 Hz, 2H), 2.51 (d, J = 7.0 Hz, 2H), 2.38 - 2.31 (m, 2H), 1.84 (t, J = 10.8 Hz, 2H), 1.56 (d, J = 13.0 Hz, 2H), 1.50 - 1.44 (m, 1H), 1.21 - 1.10 (m, 2H). | 377.07 (M+1) |
| 5 | 3-(1-((4-butoxyphe nyl)sulfo nyl)piper idin-4-yl)-1H-indole | ¹H NMR (400 MHz, CDCl₃) δ 7.95 (s, 1H), 7.74 (d, J = 8.9 Hz, 2H), 7.51 (d, J = 7.5 Hz, 1H), 7.36 (d, J = 8.1 Hz, 1H), 7.18 (t, J = 7.6 Hz, 1H), 7.08 (dd, J = 11.0, 4.0 Hz, 1H), 7.01 (d, J = 8.9 Hz, 2H), 6.95 (d, J = 2.1 Hz, 1H), 4.04 (t, J = 6.5 Hz, 2H), 3.91 (d, J = 11.6 Hz, 2H), 2.76 (t, J = 12.0 Hz, 1H), 2.44 (dd, J = 11.9, 9.8 Hz, 2H), 2.09 (d, J = 11.3 Hz, 2H), 1.94 - 1.84 (m, 2H), 1.80 (dd, J = 14.7, 6.8 Hz, 2H), 1.56 - 1.50 (m, 2H), 1.00 (t, J = 7.4 Hz, 3H). | 413.11 (M+1) |
| 6 | N- (2- (4-(1H-indol-3-yl)piperi din-1-yl)ethyl) -4-butoxyben zenesulfo namide | ¹H NMR (400 MHz, CDCl₃) δ (s,1H), 7.79 (d, J = 8.8 Hz, 2H), 7.61 (d, J = 7.8 Hz, 1H), 7.38 (d, J = 8.1 Hz, 1H), 7.22 - 7.17 (m, 1H), 7.11 (t, J = 7.5 Hz, 1H), 7.01 - 6.97 (m, 1H), 6.95 (d, J = 8.9 Hz, 2H), 3.99 (t, J = 6.5 Hz, 2H), 3.01 - 2.94 (m, 2H), 2.84 - 2.74 (m, 1H), 2.71 (d, J = 11.3 Hz, 2H), 2.50 - 2.39 (m, 2H), 2.09 (t, J = 11.5 Hz, 2H), 1.98 (d, J = 12.6 Hz, 2H), 1.81 - 1.72 (m, 2H), 1.72 - 1.62 (m, 2H), 1.47 (dd, J = 15.0, 7.5 Hz, 2H), 0.96 (t, J = 7.4 Hz, 3H). | 456.13 (M+1) |
| 7 | 4-butoxy-N- (2- (4-(5-methyl-1H-indol-3-yl)piperi din-1-yl)ethyl) benzenesu lfonamide | ¹H NMR (400 MHz, MeOD) δ 7.83 (d, J = 8.8 Hz, 2H), 7.45 (t, J = 7.9 Hz, 2H), 7.29 - 7.23 (m, 3H), 7.16 (dd, J = 12.2, 7.2 Hz, 3H), 7.09 (d, J = 8.7 Hz, 4H), 3.00 (t, J = 7.0 Hz, 2H), 2.82 (d, J = 11.5 Hz, 2H), 2.54 (d, J = 6.8 Hz, 2H), 2.44 (t, J = 7.0 Hz, 2H), 1.96 (t, J = 11.7 Hz, 2H), 1.60 (t, J = 11.6 Hz, 2H), 1.56 - 1.50 (m, 1H), 1.30 - 1.19 (m, 2H). | 451.05 (M+1) |
| 8 | 4-butoxy-N-(2-(4-(5-methoxy-1H-indol-3-yl) piperi din-1-yl) ethyl) benzenesu lfonamide | ¹H NMR (400 MHz, CDCl₃) δ 7.87 (s, 1H), 7.79 (d, J = 8.8 Hz, 2H), 7.38 (s, 1H), 7.27 (s, 1H), 7.05 - 6.98 (m, 1H), 6.95 (d, J = 8.8 Hz, 3H), 3.99 (t, J = 6.5 Hz, 2H), 3.03 - 2.94 (m, 2H), 2.79 - 2.74 (m, 1H), 2.70 (d, J = 11.4 Hz, 2H), 2.46 (s, 3H), 2.45 - 2.42 (m, 2H), 2.12 - 2.03 (m, 2H), 2.02 - 1.92 (m, 2H), 1.76 (dd, J = 14.8, 6.8 Hz, 2H), 1.67 (dd, J = 23.5, 14.0 Hz, 2H), 1.50 - 1.43 (m, 2H), 0.96 (t, J = 7.4 Hz, 3H). | 470.04 (M+1) |
| 9 | 1-((4-butoxyphe nyl)sulfo nyl)-4-phenethyl piperidine | ¹H NMR (400 MHz, CDCl₃) δ 7.87 (s, 1H), 7.79 (d, J = 8.7 Hz, 2H), 7.28 (s, 1H), 7.02 (d, J = 2.1 Hz, 1H), 6.95 (d, J = 8.8 Hz, 3H), 6.86 (dd, J = 8.7, 2.3 Hz, 1H), 3.99 (t, J = 6.5 Hz, 2H), 3.87 (s, 3H), 2.99 (t, J = 5.7 Hz, 2H), 2.71 (d, J = 11.9 Hz, 3H), 2.45 (t, J = 5.7 Hz, 2H), 2.09 (t, J = 11.1 Hz, 2H), 1.98 (d, J = 12.3 Hz, 2H), 1.81 - 1.72 (m, 2H), 1.71 - 1.63 (m, 2H), 1.50 - 1.45 (m, 2H), 0.97 (t, J = 7.4 Hz, 3H). | 486.03 (M+1) |
| 10 | N- (3- (4-benzylpip eridin-1-yl)propyl ) -4-butoxyben zenesulfo namide | ¹H NMR (400 MHz, MeOD) δ 7.69 (d, J = 8.8 Hz, 2H), 7.24 (t, J = 7.6 Hz, 2H), 7.15 (d, J = 7.7 Hz, 3H), 7.10 (d, J = 8.8 Hz, 2H), 4.09 (t, J = 6.4 Hz, 2H), 3.72 (d, J = 12.0 Hz, 2H), 2.64 - 2.57 (m, 2H), 2.21 (t, J = 11.7 Hz, 2H), 1.80 (dd, J = 14.3, 7.3 Hz, 4H), 1.54 (dd, J = 14.7, 7.6 Hz, 4H), 1.28 - 1.21 (m, 2H), 1.02 (t, J = 7.4 Hz, 3H). | 402.02 (M+1) |
| 11 | N- (3- (4-(1H-indol-3-yl)piperi din-1-yl)propyl ) -4-butoxyben zenesulfo namide | ¹H NMR (400 MHz, CDCl₃) δ 7.67 (d, J = 8.1 Hz, 2H), 7.48 (d, J = 8.4 Hz, 2H), 7.35 - 7.27 (m, 3H), 7.23 - 7.15 (m, 2H), 7.11 (d, J = 7.5 Hz, 2H), 7.08 (s, 1H), 3.31 (d, J = 11.4 Hz, 2H), 3.14 (t, J = 5.7 Hz, 2H), 2.86 (t, J = 6.4 Hz, 2H), 2.56 (d, J = 5.9 Hz, 2H), 2.33 (s, 2H), 1.91 - 1.85 (m, 2H), 1.77 (d, J = 11.3 Hz, 2H), 1.63 - 1.58 (m, 3H). | 412.03 (M+1) |
| 12 | N-(3-(4-benzylpip eridin-1-yl)propyl )-1H-indol-2-sulfonamide | ¹H NMR (400 MHz, CDCl₃) δ 7.78 (d, J = 8.7 Hz, 2H), 7.29 (t, J = 7.5 Hz, 2H), 7.20 (t, J = 7.2 Hz, 1H), 7.15 (d, J = 7.5 Hz, 2H), 6.96 (d, J = 8.7 Hz, 2H), 4.01 (t, J = 6.5 Hz, 2H), 3.12 - 3.03 (m, 4H), 2.57 (d, J = 6.6 Hz, 4H), 2.04 (s, 2H), 1.82 - 1.75 (m, 2H), 1.72 (d, J = 12.8 Hz, 4H), 1.59 - 1.45 (m, 5H), 0.99 (t, J = 7.4 Hz, 3H). | 445.10 (M+1) |
| 13 | N-(3-(4-benzylpip eridin-1-yl)propyl )-4-phenoxybe nzenesulfonamide | ¹H NMR (400 MHz, MeOD) δ 7.80 (d, J = 8.8 Hz, 2H), 7.59 (d, J = 7.8 Hz, 1H), 7.33 (d, J = 8.1 Hz, 1H), 7.13 - 7.01 (m, 4H), 6.99 (t, J = 7.5 Hz, 1H), 4.07 (t, J = 6.4 Hz, 2H), 3.06 (d, J = 9.7 Hz, 2H), 2.93 (t, J = 6.6 Hz, 2H), 2.86 (d, J = 11.5 Hz, 1H), 2.49 (s, 2H), 2.22 (d, J = 7.2 Hz, 2H), 2.07 (d, J = 11.2 Hz, 2H), 1.91 - 1.81 (m, 2H), 1.78 (dd, J = 14.6, 6.4 Hz, 2H), 1.74 - 1.66 (m, 2H), 1.58 - 1.46 (m, 2H), 1.00 (t, J = 7.4 Hz, 3H). | 470.10 (M+1) |
| 14 | N-(3-(4-benzylpip eridin-1-yl)propyl )-4-methoxybe nzenesulfonamide | ¹H NMR (400 MHz, CDCl₃) δ 7.80 (d, J = 8.6 Hz, 2H), 7.41 (t, J = 7.8 Hz, 2H), 7.30 - 7.26 (m, 2H), 7.24 - 7.17 (m, 2H), 7.15 (d, J = 7.4 Hz, 2H), 7.07 (d, J = 8.4 Hz, 2H), 7.04 (d, J = 8.7 Hz, 2H), 3.06 (t, J = 5.5 Hz, 2H), 2.85 (d, J = 11.4 Hz, 2H), 2.54 (d, J = 7.2 Hz, 2H), 2.37 (m, J = 5.5 Hz, 2H), 1.82 (t, J = 11.4 Hz, 2H), 1.66 (d, J = 14.4 Hz, 2H), 1.63 - 1.58 (m, 2H), 1.56 - 1.49 (m, 1H), 1.33 - 1.23 (m, 2H). | 465.04 (M+1) |
| 15 | 4-amino-N-(3-(4-benzylpip eridin-1-yl)propyl )benzenes ulfonamid e | ¹H NMR (400 MHz, CDCl₃) δ 7.79 (d, N-(3-(4-benzylpip eridin-1-yl)propyl )benzenes ulfonamid e | 402.95 |
| 16 | N-(3-(4-benzylpip eridin-1-yl)propyl )-4-(butylami no)benzen esulfonamide | ¹H NMR (400 MHz, CDCl₃) δ 7.77 (d, J = 8.6 Hz, 2H), 7.35 (d, J = 8.1 Hz, 1H), 7.26 (d, J = 8.4 Hz, 1H), 6.97 (t, J = 9.1 Hz, 3H), 4.02 (t, J = 6.5 Hz, 2H), 3.02 (t, J = 11.0 Hz, 2H), 2.85 (d, J = 11.4 Hz, 2H), 2.50 (d, J = 7.2 Hz, 2H), 2.35 (t, J = 5.6 Hz, 2H), 1.87 - 1.69 (m, 4H), 1.65 (s, 1H), 1.63 - 1.56 (m, 3H), 1.49 (dt, J = 14.5, 7.3 Hz, 3H), 1.30 (dd, J = 12.3, 3.1 Hz, 2H), 0.99 (t, J = 7.4 Hz, 3H). | 514.45 (M+1) |
| 17 | N-(4-(N-(3-(4-benzylpip eridin-1-yl)propyl )sulfamoy 1) phenyl) -3-(piperidi n-1-yl)propaneamide | ¹H NMR (400 MHz, CDCl₃) δ 7.77 (d, J = 8.7 Hz, 2H), 7.06 (dd, J = 18.6, 8.4 Hz, 1H), 6.95 (t, J = 9.0 Hz, 3H), 6.85 (s, 1H), 4.01 (t, J = 6.5 Hz, 2H), 3.02 (t, J = 11.2 Hz, 2H), 2.90 (d, J = 11.5 Hz, 2H), 2.50 (d, J = 7.2 Hz, 2H), 2.39 (t, J = 5.8 Hz, 2H), 1.85 (t, J = 10.8 Hz, 2H), 1.78 (dd, J = 14.7, 6.8 Hz, 2H), 1.68 - 1.59 (d, J = 4.1 Hz, 4H), 1.50 (dq, J = 14.1, 7.1 Hz, 3H), 1.36 - 1.26 (m, 2H), 0.99 (t, J = 7.4 Hz, 3H). | 481.50 (M+1) |
| 18 | N-(4-(N-(3-(4-benzylpip eridin-1-yl)propyl )sulfamoy l)phenyl) -3-(dimethyl amino)propaneamide | ¹H NMR (400 MHz, CDCl₃) δ 7.78 (d, *J* = 8.7 Hz, 2H), 7.38 (d, *J* = 8.3 Hz, 1H), 7.30 (d, *J* = 1.7 Hz, 1H), 7.08 (dd, *J* = 8.3, 1.8 Hz, 1H), 6.96 (d, *J* = 8.8 Hz, 2H), 4.01 (t, *J* = 6.5 Hz, 2H), 3.10 - 3.04 (m, 2H), 3.00 (d, *J* = 11.5 Hz, 2H), 2.43 (t, *J =* 5.8 Hz, 2H), 1.99 (t, *J* = 11.0 Hz, 2H), 1.84 (d, *J* = 13.3 Hz, 2H), 1.82 - 1.75 (m, 2H), 1.75 - 1.67 (m, 2H), 1.64 (dd, *J* = 11.2, 5.6 Hz, 2H), 1.50 (dd, *J* = 15.0, 7.5 Hz, 2H), 1.34 - 1.28 (m, 1H), 0.98 (t, *J =* 7.4 Hz, 3H). | 500.40 (M+1) |
| 19 | N-(3-(4-benzylpip eridin-1-yl)propyl )-4-butoxybenzenesulfi neamide | ¹H NMR (400 MHz, CDCl₃) δ 7.76 (d, *J* = 8.8 Hz, 2H), 7.29 (s, 1H), 6.96 (dd, *J* = 5.8, 2.8 Hz, 3H), 6.74 (dd, *J* = 9.0, 2.7 Hz, 1H), 4.01 (t, *J* = 6.4 Hz, 2H), 3.22 - 3.14 (m, 3H), 9.22 (t, *J* = 5.7 Hz, 2H), 2.56 (s, 3H), 2.48 (s, 1H), 2.06 - 1.99 (m, 2H), 1.82-1.76 (m, 2H), 1.72-1.66 (m, 2H), 1.53-1.44 (m. 4 H), 0.99 (t, *J* = 7.4 Hz, 3H). | 500.10 (M) |
| 20 | 4-butoxy-N-(3-(4-(3,4-dichlorob enzyl)pip eridin-1-yl)propyl )benzenes ulfonamide | ¹H NMR (400 MHz, CDCl₃) δ 7.78 (d, J = 8.4 Hz, 2H), 7.23 - 7.11 (m, 2H), 7.03 - 6.88 (m, 3H), 4.01 (t, *J* = 6.4 Hz, 2H), 3.15 - 2.95 (m, 5H), 2.61 (s, 2H), 2.50 (t, *J* = 5.6 Hz, 2H), 1.84 - 1.73 (m, 2H), 1.71 - 1.62 (m, 2H), 1.55 - 1.45 (m, 2H), 1.36 - 1.22 (m, 3H), 1.48 (t, *J* = 7.4 Hz, 3H). | 500.01 (M) |
| 21 | 4-butoxy-N-(3-(4-(3,4-difluorob enzyl)pip eridin-1-yl)propyl )benzenes ulfonamide | ¹H NMR (400 MHz, CDCl₃) δ 7.77 (d, *J* = 8.5 Hz, 2H), 6.96 (d, J = 8.6 Hz, 2H), 4.07 - 3.91 (m, 4H), 3.38 (t, *J* = 11.6 Hz, 2H), 3.03 (t, *J* = 5.5 Hz, 2H), 2.60 - 2.28 (m, 7H), 2.22 (d, *J* = 7.1 Hz, 2H), 2.07 - 1.98 (m, 1H), 1.84 - 1.70 (m, 3H), 1.69 - 1.58 (m, 4H), 1.54 - 1.45 (m, 2H), 1.35 - 1.19 (m, 5H), 0.99 (t, J = 7.4 Hz, 3H). | 454.15 (M+1) |
| 22 | 4-butoxy-N-(3-(4-(3,4-dichlorop henyl)pip eridin-1-yl)propyl ) benzenes ulfonamide | ¹H NMR (400 MHz, CDCl₃) δ 7.77 (d, *J* = 8.5 Hz, 2H), 7.36 (t, *J* = 7.9 Hz, 1H), 7.14 - 7.03 (m, 3H), 6.96 (d, *J* = 8.6 Hz, 2H), 4.01 (t, *J* = 6.4 Hz, 2H), 3.29 - 3.23 (m, 3H), 3.08 (t, *J* = 5.7 Hz, 2H), 2.68 - 2.58 (m, 3H), 2.57 - 2.46 (m, 2H), 1.85 - 1.66 (m, 5H), 1.55 - 1.45 (m, 3H), 0.99 (t, *J* = 7.4 Hz, 3H). | 500.09 (M+1) |
| 23 | 4-butoxy-N-(3-(4-(3,4-dichlorop henyl)pip erazin-1-yl)propyl )benzenes ulfonamide | ¹H NMR (400 MHz, CDCl₃) δ 7.76 (d, *J* = 8.6 Hz, 2H), 7.36 - 7.30 (m, 4H), 7.30 - 7.23 (m, 1H), 6.95 (d, *J* = 8.6 Hz, 2H), 4.01 (t, *J* = 6.5 Hz, 2H), 3.52 (s, 2H), 3.03 (t, *J* = 5.6 Hz, 2H), 2.60 - 2.27 (m, 8H), 1.84 - 1.74 (m, 2H), 1.66 - 1.57 (m, 2H), 1.54 - 1.45 (m, 2H), 1.33 - 1.21 (m, 2H), 0.98 (t, J = 7.4 Hz, 3H). | 446.15 |
| 24 | 4-butoxy-N-(3-(4-(2,3-dichlorop henyl) pip erazin-1-yl)propyl )benzenes ulfonamide | ¹H NMR (400 MHz, CDCl₃) δ 7.63 (d, J = 8.4 Hz, 2H), 7.38 (s, 1H), 7.29 (t, J = 7.6 Hz, 2H), 7.20 (t, J = 7.4 Hz, 1H), 7.15 (d, J = 7.6 Hz, 2H), 6.69 (d, J = 8.4 Hz, 2H), 4.07 (s, 2H), 3.01 (t, J = 5.5 Hz, 2H), 2.83 (d, J = 11.4 Hz, 2H), 2.54 (d, J = 7.2 Hz, 2H), 2.33 (t, J = 5.6 Hz, 2H), 1.79 (t, J = 11.4 Hz, 2H), 1.65 (d, J = 12.8 Hz, 2H), 1.58 (dt, J = 11.2, 5.7 Hz, 2H), 1.54 - 1.45 (m, 1H), 1.28 (qd, J = 12.6, 3.3 Hz, 2H). | 388.08 (M+1) |
| 25 | 4-butoxy-N-(3-(4-((tetrahy dro-2H-pyran-4-yl)methyl )piperazi n-1-yl)propyl )benzenes ulfonamide | ¹H NMR (400 MHz, CDCl₃) δ 7.56 (d, J = 8.4 Hz, 2H), 7.29 - 7.26 (m, 2H), 7.18 (t, J = 7.3 Hz, 1H), 7.14 (d, J = 7.5 Hz, 2H), 6.66 (d, J = 8.4 Hz, 2H), 4.06 (s, 2H), 3.07 (dt, J = 10.3, 8.0 Hz, 4H), 2.84 (d, J = 10.5 Hz, 2H), 2.52 (d, J = 7.0 Hz, 2H), 2.27 (d, J = 6.9 Hz, 2H), 1.83 (t, J = 11.2 Hz, 2H), 1.75 - 1.68 (m, 2H), 1.62 (d, J = 12.7 Hz, 2H), 1.52 - 1.45 (m, 2H), 1.32 - 1.23 (m, 3H), 0.88 (t, J = 7.3 Hz, 3H). | 444.16 (M+1) |
| 26 | 4-butoxy-N-(3-(4-(3-(trifluor omethyl)p henyl)pip erazin-1-yl)propyl ) benzenes ulfonamide | ¹H NMR (400 MHz, CDCl₃) δ 7.77 (d, J = 8.7 Hz, 2H), 6.96 (d, J = 8.7 Hz, 2H), 6.80 (d, J = 7.9 Hz, 1H), 6.69 (d, J = 10.0 Hz, 2H), 4.01 (t, J = 6.5 Hz, 2H), 3.88 (d, J = 8.5 Hz, 6H), 3.02 (t, J = 10.9 Hz, 2H), 2.85 (d, J = 11.2 Hz, 2H), 2.49 (d, J = 7.2 Hz, 2H), 2.35 (t, J = 10.9 Hz, 2H), 1.87 - 1.73 (m, 4H), 1.67 (d, J = 12.6 Hz, 2H), 1.62 - 1.56 (m, 2H), 1.49 (dt, J = 14.6, 7.4 Hz, 3H), 1.34 - 1.26 (m, | 505.16 (M+1) |
| | | 2H), 0.99 (t, J = 7.4 Hz, 3H). | |
| 27 | N-(3-(4-benzylpip erazin-1-yl)propyl )-4-butoxyben zenesulfo namide | ¹H NMR (400 MHz, CDCl₃) δ 7.77 (d, J = 8.7 Hz, 2H), 7.26 (s, 2H), 7.24 (s, 1H), 7.08 (d, J = 8.1 Hz, 2H), 6.96 (d, J = 8.7 Hz, 2H), 4.01 (t, J = 6.4 Hz, 2H), 3.02 (t, J = 10.9 Hz, 2H), 2.86 (d, J = 10.9 Hz, 2H), 2.52 (d, J = 7.2 Hz, 2H), 2.36 (t, J = 10.4 Hz, 2H), 1.86 - 1.73 (m, 4H), 1.65 - 1.58 (m, 4H), 1.49 (dt, J = 14.5, 7.4 Hz, 3H), 1.31 (d, J = 11.9 Hz, 2H), 0.99 (t, J = 7.4 Hz, 3H) . | 479.11 (M) |
| 28 | 4-butoxy-N-(3-(4-(4-chloroben zyl)piper idin-1-yl)propyl )benzenes ulfonamid e | ¹H NMR (400 MHz, CDCl₃) δ 7.77 (d, J = 8.7 Hz, 2H), 7.10 (d, J = 7.8 Hz, 2H), 7.04 (d, J = 7.8 Hz, 2H), 6.96 (d, J = 8.7 Hz, 3H), 4.01 (t, J = 6.5 Hz, 2H), 3.02 (t, J = 11.0 Hz, 2H), 2.83 (d, J = 11.4 Hz, 2H), 2.51 (d, J = 7.2 Hz, 2H), 2.34 (d, J = 5.9 Hz, 2H), 2.33 (s, 3H), 1.80 (dd, J = 14.1, 7.3 Hz, 4H), 1.66 (d, J = 12.9 Hz, 2H), 1.58 (dt, J = 11.7, 6.0 Hz, 2H), 1.49 (dt, J = 14.7, 7.4 Hz, 2H), 1.33 - 1.27 (m, 2H), 0.99 (t, J = 7.4 Hz, 3H). | 459.18 (M+1) |
| 29 | 4-butoxy-N- (3- (4-(4-methylben zyl)piper idin-1-yl)propyl )benzenes ulfonamid e | ¹H NMR (400 MHz, CDCl₃) δ 7.77 (d, J = 8.6 Hz, 2H), 7.07 (d, J = 8.2 Hz, 2H), 6.96 (d, J = 8.6 Hz, 2H), 6.83 (d, J = 8.2 Hz, 2H), 4.01 (t, J = 6.5 Hz, 2H), 3.79 (s, 3H), 3.02 (t, J = 10.9 Hz, 2H), 2.83 (d, J = 11.2 Hz, 2H), 2.49 (d, J = 7.2 Hz, 2H), 2.34 (t, J = 10.9 Hz, 2H), 1.83 - 1.77 (m, 4H), 1.65 (d, J = 12.7 Hz, 2H), 1.58 (dt, J = 11.6, 5.8 Hz, 2H), 1.49 (dt, J = 12.4, 6.4 Hz, 2H), 1.44 (d, J = 3.6 Hz, 1H), 1.33 - 1.20 (m, 2H), 0.99 (t, J = 7.4 Hz, 3H). | 475.20 (M+1) |
| 30 | 4-butoxy-N-(3-(4-(4-*J* = 8.6 Hz, 2H), 7.36 - 7.30 (m, 4H), 7.30 - 7.23 (m, 1H), 6.95 (d, *J* = 8.6 Hz, 2H), 4.01 (t, *J* = 6.5 Hz, 2H), 3.52 (s, 2H), 3.03 (t, *J* = 5.6 Hz, 2H), 2.60 - 2.27 (m, 8H), 1.84 - 1.74 (m, 2H), 1.66 - 1.57 (m, 2H), 1.54 - 1.45 (m, 2H), 1.33 - 1.21 (m, 2H), 0.98 (t, J = 7.4 Hz, 3H). | ¹H NMR (400 MHz, CDCl₃) δ 7.77 (d, *J* = 8.7 Hz, 2H), 7.24 - 7.15 (m, 2H), 7.14 (s, 1H), 7.02 (d, *J* = 7.1 Hz, 1H), 6.96 (d, *J* = 8.7 Hz, 2H), 4.02 (t, *J* = 6.5 Hz, 2H), 3.05 - 2.96 (m, 2H), 2.92 (d, *J* = 8.6 Hz, 2H), 2.53 (d, *J* = 7.2 Hz, 2H), 2.42 (d, *J* = 0.6 Hz, 1H), 2.01 (dd, *J* = 12.1, 6.4 Hz, 2H), 1.84 (s, 2H), 1.78 (dd, *J* = 14.6, 6.8 Hz, 2H), 1.67 (s, 1H), 1.64 (s, 2H), 1.57 - 1.43 (m, 3H), 0.99 (t, *J* = 7.4 Hz, 3H). | 479.14 (M) |
| 31 | 4-butoxy-N-(3-(4-(3-chloroben zyl) piper idin-1-yl)propyl )benzenes ulfonamid e | ¹H NMR (400 MHz, MeOD) δ 7.67 (s, 4H), 7.15 (t, J = 7.4 Hz, 2H), 7.05 (t, J = 8.8 Hz, 3H), 2.77 (t, J = 6.8 Hz, 2H), 2.72 (d, J = 11.5 Hz, 2H), 2.63 (t, J = 7.1 Hz, 2H), 2.49 (t, J = 7.1 Hz, 2H), 2.42 (d, J = 6.9 Hz, 6H), 2.22 - 2.16 (m, 2H), 1.93 (d, J = 8.7 Hz, 2H), 1.76 (t, J = 12.2 Hz, 2H), 1.45 - 1.37 (m, 8H), 1.48 - 1.40 (m, 3H), 1.19 - 1.08 (m, 2H). | 527.24 (M+1) |
| 32 | 4-butoxy-N-(3-(4-(4-fluoroben zyl)piper idin-1-yl)propyl )benzenes ulfonamid e | ¹H NMR (400 MHz, CDCl₃) δ 7.77 (d, J = 8.8 Hz, 2H), 7.09 (dd, J = 8.2, 5.7 Hz, 2H), 6.97 (t, J = 8.5 Hz, 2H), 4.01 (t, J = 6.5 Hz, 2H), 3.02 (t, J = 11.2 Hz, 2H), 2.84 (d, J = 11.5 Hz, 2H), 2.51 (d, J = 7.2 Hz, 2H), 2.34 (t, J = 11.2 Hz, 2H), 1.85 - 1.73 (m, 4H), 1.65 (s, 1H), 1.63 - 1.54 (m, 3H), 1.54 - 1.45 (m, 2H), 1.44 (s, 1H), 1.33 - 1.19 (m, 2H), 0.99 (t, J = 7.4 Hz, 3H). | 463.05 (M+1) |
| 33 | 4-butoxy-N-(3-(4-(2,3-dichlorop henyl)pip eridin-1-yl)propyl )benzenes ulfonamid e | ¹H NMR (400 MHz, CDCl₃) δ 7.78 (d, J = 8.8 Hz, 2H), 7.33 (dd, J = 7.5, 1.6 Hz, 1H), 7.26 - 7.17 (m, 2H), 6.95 (d, J = 8.8 Hz, 2H), 4.01 (t, J = 6.5 Hz, 2H), 3.12 - 3.04 (m, 2H), 3.01 (d, J = 11.6 Hz, 2H), 2.45 (t, J = 13.4 Hz, 2H), 2.06 (t, J = 11.2 Hz, 2H), 1.89 (d, J = 12.7 Hz, 2H), 1.82 - 1.74 (m, 2H), 1.71 - 1.61 (m, 4H), 1.55 - 1.43 (m, 2H), 1.35 - 1.27 (m, 1H), 0.98 (t, J = 7.4 Hz, 3H). | 500.60 (M+1) |
| 34 | N-(3-(4-benzylpip eridin-1-yl)propyl )-4-(trifluor omethoxy) benzenesu lfonamide | ¹H NMR (400 MHz, CDCl₃) δ 7.90 (d, J = 8.7 Hz, 2H), 7.34 (d, J = 8.4 Hz, 2H), 7.29 (t, J = 7.4 Hz, 2H), 7.20 (t, J = 7.3 Hz, 1H), 7.15 (d, J = 7.3 Hz, 2H), 3.07 (t, J = 11.0 Hz, 2H), 2.86 (d, J = 11.4 Hz, 2H), 2.56 (d, J = 7.2 Hz, 2H), 2.39 (t, J = 7.4 Hz, 2H), 1.84 (t, J = 11.4 Hz, 2H), 1.68 (d, J = 13.6 Hz, 2H), 1.62 (dd, J = 10.6, 5.5 Hz, 2H), 1.58 - 1.47 (m, 1H), 1.31 (d, J = 11.7 Hz, 2H). | 457.00 (M+1) |
| 35 | N-(3-(4-benzylpip eridin-1-yl)propyl )-5-butoxypyr idin-2-sulfonami de | ¹H NMR (400 MHz, CDCl₃) δ 7.60 (d, *J* = 8.6 Hz, 2H), 7.32 - 7.27 (m, 2H), 7.23 - 7.16 (m, 1H), 7.11 (d, *J* = 7.4 Hz, 2H), 6.99 (d, *J* = 8.6 Hz, 2H), 4.00 (t, *J* = 6.4 Hz, 2H), 3.20 - 3.11 (m, 1H), 3.10 - 2.96 (m, 2H), 2.93 - 2.85 (m, 1H), 2.53 - 2.46 (m, 3H), 2.00 - 1.86 (m, 2H), 1.83 - 1.71 (m, 4H), 1.63 (t, *J* = 11.6 Hz, 2H), 1.54 - 1.45 (m, 3H), 1.35 - 1.25 (m, 3H), 0.98 (t, *J* = 7.4 Hz, 3H). | 429.25 (M+1) |
| 36 | N-(2-(4-benzylpip eridin-1-yl)ethyl) -1H-indol-2-sulfonami de | ¹H NMR (400 MHz, MeOD) δ 8.34 (d, J = 2.6 Hz, 1H), 7.94 (d, J = 8.7 Hz, 1H), 7.53 (dd, J = 8.7, 2.8 Hz, 1H), 7.27 (t, J = 7.4 Hz, 2H), 7.17 (t, J = 8.3 Hz, 3H), 4.15 (t, J = 6.4 Hz, 2H), 3.02 (t, J = 6.7 Hz, 2H), 2.89 (d, J = 11.5 Hz, 2H), 2.55 (d, J = 7.0 Hz, 2H), 2.36 (t, J = 15 Hz, 2H), 1.92 (t, J = 11.7 Hz, 2H), 1.86 - 1.77 (m, 2H), 1.69 - 1.59 (m, 4H), 1.54 (dq, J = 14.8, 7.6 Hz, 4H), 1.26 (d, J = 11.7 Hz, 1H), 1.02 (t, J = 7.4 Hz, 3H). | 446.25 (M+1) |
| 37 | 4-butoxy-N-(3-(4-(3,4-dimethoxy benzyl) pi peridin-1-yl)propyl | ¹H NMR (400 MHz, CDCl₃) δ 11.44 (s, 1H), 7.78 (d, J = 8.7 Hz, 2H), 7.64 (d, J = 8.7 Hz, 2H), 7.29 (t, J = 7.4 Hz, 2H), 7.19 (t, J = 7.4 Hz, 1H), 7.15 (d, J = 7.0 Hz, 2H), 3.03 (t, J = 5.6 Hz, 2H), 2.86 (d, J = 11.6 Hz, 2H), 2.66 (t, J = 5.8 Hz, 2H), 2.56 - 2.51 (m, 4H), 2.40 | 487.18 (M+1) |
| | )benzenes ulfonamid e | (s, 6H), 2.35 (t, J = 5.6 Hz, 2H), 1.82 (t, J = 11.0 Hz, 2H), 1.67 (d, J = 13.2 Hz, 2H), 1.62 - 1.56 (m, 2H), 1.55 - 1.48 (m, 1H), 1.35 - 1.25 (m, 2H) | |

**[Table 16]**

| Examp le | Compound name | NMR |
|---|---|---|
| 38 | N-(2-(4-benzylpipe ridin-1-yl)ethyl)n aphthalen-2-sulfonamide | ¹H NMR (500 MHz, CDCl₃) : δ 8.45-8.44 (m, 1H), 7.98-7.96 (m, 2H), 7.95-7.90 (m, 1H), 7.84-7.82 (m, 1H), 7.64-7.60 (m, 1H), 7.29-7.26 (m, 3H, overlapped with CDCl₃), 7.20-7.17 (m, 1H), 7.12-7.10 (m, 2H), 3.00-2.97 (m, 2H), 2.52-2.50 (m, 4H), 2.33-2.31 (m, 2H), 1.80-1.74 (m, 2H), 1.51-1.40 (m, 3H), 1.20-1.10 (m, 2H); ¹³C NMR (125 MHz, CDCl₃): δ 140.5, 136.3, 134.8, 132.1, 129.4, 129.2, 129.1, 128.7, 128.5, 128.2, 127.9, 127.5, 125.8, 122.3, 55.7, 53.2, 43.1, 39.4, 37.8, 32.1. |
| 39 | N-(2-(4-benzylpipe ridin-1-yl) ethyl) n aphthalen-1-sulfonamide | ¹H NMR (500 MHz, CDCl₃): δ 8.71-8.69 (m, 1H), 8.28-8.26 (m, 1H), 8.07-8.00 (m, 1H), 7.97-7.95 (m, 1H), 7.72-7.67 (m, 1H), 7.64-7.61 (m, 1H), 7.56-7.53 (m, 1H), 7.30-7.27 (m, 2H), 7.22-7.18 (m, 1H), 7.10-7.08 (m, 2H), 2.89-2.87 (m, 2H), 2.43 (d, J = 6.7 Hz, 2H), 2.23 (d, J = 11.5 Hz, 2H), 2.15-2.14 (m, 2H), 1.65-1.60 (m, 2H), 1.35-1.27 (m, 3H), 0.90-0.82 (m, 2H) ¹³C NMR (125 MHz, CDCl₃): δ 140.4, 134.2, 134.1, 134.0, 129.7, 129.1, 129.0, 128.2, 128.1, 126.8, 126.7,124.3, 124.0, 55.3, 52.9, 43.0, 39.6, 37.5, 31.7. |
| 40 | N-(2-(4-benzylpipe ridin-1-yl)ethyl)-[1,1'-biphenyl] - 4-sulfonamide | ¹H NMR (500 MHz, CDCl₃): δ 8.47-8.30 (m, 1H), 7.99-7.90 (m, 3H), 7.85-7.82 (m, 1H), 7.66-7.60 (m, 2H), 7.31-7.27 (m, 2H), 7.22-7.14 (m, 3H), 3.10-3.07 (m, 2H), 2.83 (d, J = 11.0 Hz, 2H), 2.56 (d, J = 17.3 Hz, 2H), 2.33-2.30 (m, 2H), 1.82-1.77 (m, 2H), 1.67 (d, J = 12.8 Hz, 2H), 1.60-1.56 (m, 3H), 1.36-1.28 (m, 2H); ¹³C NMR (125 MHz, CDCl₃) : δ 140.6, 137.0, 134.6, 132.2, 129.3, 129.1, 129.0, 128.5, |
| | | 128.2, 128.1, 127.9, 127.4, 125.8, 122.4, 58.6, 53.8, 44.7, 42.9, 38.0, 32.3, 23.7. |
| 41 | N-(2-(4-benzylpipe ridin-1-yl)ethyl)q uinolin-2-sulfonamide | ¹H NMR (500 MHz, CDCl₃) : δ 9.05-9.00 (m, 1H), 8.47-8.40 (m, 1H), 8.27-8.25 (m, 1H), 8.04-8.02 (m, 1H), 7.62-7.59 (m, 1H), 7.55-7.52 (m, 1H), 7.28-7.07 (m, 5H), 6.73 ((s, 1H), 2.96-2.94 (m, 2H), 2.44 (d, J = 6.5, 2H), 2.32 (d, J = 11.7, 2H), 2.26-2.23 (m, 2H), 1.67-1.62 (m, 2H), 1.37-1.32 (m, 3H), 0.92-0.84 (m, 2H); ¹³C NMR (125 MHz, CDCl₃) : δ 151.1, 143.4, 140.4, 136.0, 135.9, 133.3, 131.2, 129.1, 128.7, 128.2, 125.8, 125.6, 122.1, 56.3, 53.3, 43.1, 40.6, 37.5, 31.9. |
| 42 | N-(2-(4-phenylpipe ridin-1-yl)ethyl)n aphthalen-2-sulfonamide | ¹H NMR (500 MHz, CDCl₃) : δ 8.50-8.49 (m, 1H), 7.99-7.97 (m, 2H), 7.92-7.88 (m, 2H), 7.67-7.60 (m, 2H), 7.34-7.31 (m, 2H), 7.24-7.20 (m, 3H), 5.50 ((s, 1H), 3.07-3.05 (m, 2H), 2.69 (d, J = 11.6, 2H), 2.46-2.40 (m, 3H), 2.01-2.68 (m, 2H), 1.75-1.62 (m, 4H); ¹³C NMR (125 MHz, CDCl₃) : δ 146.0, 136.3, 134.7, 132.1, 129.4, 129.2, 128.8, 128.6, 128.5, 127.9, 127.6, 126.8, 126.2, 122.3, 55.8, 53.6, 42.4, 39.5, 33.3. |
| 43 | N-(2-(4-phenylpipe ridin-1-yl) ethyl) n aphthalen-1-sulfonamide | ¹H NMR (500 MHz, CDCl₃): δ 8.78-8.76 (m, 1H), 8.32-8.30 (m, 1H), 8.07-8.05 (m, 1H), 7.95-7.93 (m, 1H), 7.74-7.71 (m, 1H), 7.60-7.54 (m, 2H), 7.35-7.15 (m, 5H), 5.67 (s, 1H), 2.96-2.94 (m, 2H), 2.38-2.22 (m, 5H), 1.85-1.80 (m, 2H), 1.56-1.54 (m, 2H), 1.38-1.27 (m, 2H); ¹³C NMR (125 MHz, CDCl₃) : δ 146.0, 134.2, 134.17, 134.1, 129.7, 129.2, 128.4, 128.3, 128.1, 126.9, 126.8, 126.2, 124.4, 124.2, 55.5, 53.4, 48.3, 39.8, 33.1. |
| 44 | N-(2-(4-phenylpipe ridin-1-yl)ethyl)-[1,1'-biphenyl]-4-sulfonamide | ¹H NMR (500 MHz, CDCl₃): δ 7.96 (d, J = 8.1, 2H), 7.71 (d, J = 8.0, 2H), 7.57 (d, J = 7.5, 2H), 7.46-7.37 (m, 3H), 7.30-7.28 (m, 2H), 7.22-7.18 (m, 3H), 5.41 (s, 1H), 3.07-3.04 (m, 2H), 2.71 (d, J = 11.75 Hz, 2H), 2.45-2.42 (m, 3H), 2.02-1.98 (m, 2H), 1.76-1.61 (m, 4H); ¹³C NMR (125 MHz, CDCl₃): δ 146.0, 145.5, 139.2, 138.3, 129.1, 128.52, 128.5, 127.7, 127.6, 127.3, 126.8, 126.3, 55.9, |
| | | 53.7, 42.5, 39.6, 33.4. |
| 45 | N-(2-(4-phenylpipe ridin-1-yl) ethyl) q uinolin-2-sulfonamide | ¹H NMR (500 MHz, CDCl₃): δ 9.08-9.07 (m, 1H), 8.44-8.42 (m, 1H), 8.26-8.24 (m, 1H), 8.05-8.03 (m, 1H), 7.64-7.61 (m, 1H), 7.52-7.50 (m, 1H), 7.33-7.29 (m, 2H), 7.22-7.13 (m, 3H), 6.78-6.76 (m, 1H), 3.03-3.00 (m, 2H), 2.47 (d, J = 11.55 Hz, 2H), 2.36-2.29 (m, 3H), 1.87-1.82 (m, 2H), 1.60-1.57 (m, 2H), 1.41-1.33 (m, 2H); ¹³C NMR (125 MHz, CDCl₃) : δ 151.1, 148.2, 143.4, 137.0, 135.9, 133.3, 131.2, 128.7, 128.4, 126.7, 126.2, 125.7, 122.2, 56.4, 53.7, 42.3, 40.6, 33.2. |
| 46 | N-(2-(4-benzylpipe razin-1-yl)ethyl)n aphthalen-2-sulfonamide | ¹H NMR (500 MHz, CDCl₃) : δ 8.44-8.43 (m, 1H), 7.99-7.89 (m, 4H), 7.83-7.81 (m, 1H), 7.66-7.60 (m, 2H), 7.32-7.23 (m, 5H, overlapped with CDCl₃)), 3.47 (s, 2H), 3.01-3.00 (m, 2H), 2.38-2.24 (m, 10H); ¹³C NMR (125 MHz, CDCl₃) : δ 137.9, 136.3, 134.7, 132.1, 129.4, 129.2, 129.1, 128.8, 128.5, 128.2, 127.9, 127.6, 127.1, 122.3, 62.9, 55.4, 52.9, 52.4, 39.2. |
| 47 | N-(2-(4-benzylpipe razin-1-yl) ethyl) n aphthalen-1-sulfonamid e | ¹H NMR (500 MHz, CDCl₃) : δ 8.67-8.65 (m, 1H), 8.27-8.25 (m, 1H), 8.04-8.03 (m, 1H), 7.93-7.91 (m, 1H), 7.68-7.65 (m, 1H), 7.60-7.57 (m, 1H), 7.54-7.51 (m, 1H), 7.31-7.23 (m, 5H, overlapped with CDCl₃)), 3.39 (s, 2H), 2.89-2.87 (m, 2H), 2.18-2.00 (m, 10H); ¹³C NMR (125 MHz, CDCl₃) : δ 137.7, 134.1, 134.0, 133.9, 124.3, 124.1, 129.7, 129.1, 129.0, 128.3, 128.1, 128.0, 127.0, 126.8, 62.8, 55.0, 52.5, 52.1, 39.3. |
| 48 | N- (2- (4-phenylpipe razin-1-yl)ethyl)-[1,1'-biphenyl]-4-sulfonamid e | ¹H NMR (500 MHz, CDCl₃): δ 7.95-7.92 (m, 2H), 7.73-7.70 (m, 2H), 7.60-7.58 (m, 2H), 7.50-7.41 (m, 3H), 7.33-7.24 (m, 5H, overlapped with CDCl₃), 3.49 (s, 2H), 3.04-3.02 (m, 2H), 2.43-2.30 (m, 10H); ¹³C NMR (125 MHz, CDCl₃) : δ 144.5, 139.2, 138.1, 137.9, 129.1, 129.0, 128.5, 128.2, 127.7, 127.6, 127.3, 127.1, 63.0, 55.4, 52.9, 52.4, 39.2. |
| 49 | N-(2-(4-phenylpipe razin-1-yl)ethyl)q uinolin-2-sulfonamide | ¹H NMR (500 MHz, CDCl₃) : δ 9.01 (dd, J = 4.5, 1.5 Hz, 1H), 8.43 (dd, J = 7.0, 1.5 Hz, 1H), 8.27-8.25 (m, 1H), 8.04-8.02 (m,1H), 7.65-7.62 (m, 1H), 7.54-7.52 (m, 1H), 7.33-7.24 ((m, 5H), 6.71 (t, J = 5.5 Hz, 1H), 3.43 (s, 2H), 2.99-2.96 (m, 2H), 2.33-2.10 (m, 10H); ¹³C NMR (125 MHz, CDCl₃) : δ 151.1, 143.4, 137.7, 137.0, 136.0, 133.1, 131.2, 129.1, 128.6, 128.1, 127.0, 125.6, 122.0, 62.9, 56.0, 52.6, 52.5, 40.3. |
| 50 | N-(2-(4-(4-chlorophen yl)piperaz in-1-yl)ethyl)n aphthalen-2-sulfonamide | ¹H NMR (500 MHz, CDCl₃): δ 8.46-8.45 (m, 1H), 8.00-7.83 (m, 4H), 7.86-7.83 (m, 2H), 7.68-7.61 (m, 2H), 7.21-7.18 (m, 2H), 3.07-3.02 (m, 6H), 2.47-2.45 (m, 2H), 2.39 (t, J = 5.0 Hz, 4H); ¹³C NMR (125 MHz, CDCl₃) : δ 149.6, 136.3, 134.8, 132.1, 129.5, 129.2, 128.9, 128.8, 128.6, 127.9, 127.7, 124.8, 124.2, 117.3, 55.6, 52.3, 49.0, 39.2. |
| 51 | N-(2-(4-(4-chlorophen yl)piperaz in-1-yl)ethyl)n aphthalen-1-sulfonamide | ¹H NMR (500 MHz, CDCl₃) : δ 8.70-8.67 (m, 1H), 8.31-8.30 (m, 1H), 8.11-8.09 (m, 1H), 8.00-7.97 (m, 1H), 7.70-7.66 (m, 1H), 7.62-7.57 (m, 2H), 7.21-7.19 (m, 2H), 6.74-6.71 (m, 2H), 5.51 (s, 1H), 2.96 (s, 2H), 2.81 (t, J = 5Hz, 4H), 2.28-2.26 (m, 2H), 2.16 (t, J = 5 Hz, 4H); ¹³C NMR (125 MHz, CDCl₃): δ 149.6, 134.24, 134.23, 134.0, 129.9, 129.2, 128.9, 128.4, 128.1, 126.9, 124.7, 124.3, 124.2, 117.2, 55.2, 52.1, 48.8, 39.4, 30.9. |
| 52 | N-(2-(4-(4-chlorophen yl)piperaz in-1-yl) ethyl) - [1,1'-biphenyl]-4-sulfonamide | ¹H NMR (500 MHz, CDCl₃): δ 7.96-7.95 (m, 2H), 7.75-7.72 (m, 2H), 7.62-7.58 (m, 2H), 7.51-7.48 (m, 2H), 7.45-7.43 (m, 1H), 7.21-7.18 (m, 2H), 6.80-6.77 (m, 2H), 3.10-3.05 (m, 6H), 2.51-2.48 (m, 2H), 2.45-2.43 (m, 4H); ¹³C NMR (125 MHz, CDCl₃) : δ 149.6, 145.6, 139.2, 138.2, 129.1, 129.0, 128.6, 127.7, 127.6, 127.3, 124.8, 117.3, 55.7, 52.4, 49.1, 39.3. |
| 53 | N-(2-(4-(4-chlorophen yl) piperaz in-1-yl) ethyl)q | ¹H NMR (500 MHz, CDCl₃): δ 9.03-9.02 (m, 1H), 8.45 (dd, J = 7.1, 5.0 Hz, 1H), 8.29-8.27 (m, 1H), 8.07-8.05 (m, 1H), 7.68-7.65 (m, 1H), 7.54-7.51 (m, 1H), 7.20-7.17 (m, 2H), 6.76-6.72 (m, 3H), 3.06-3.03 (m, 2H), 2.86-2.84 (m, 4H), |
| | uinolin-2-sulfonamid e | 2.42-2.40 (m, 2H), 2.26-2.24 (m, 4H); ¹³C NMR (125 MHz, CDCl₃): δ 151.1, 149.7, 143.5, 136.9, 136.0, 133.2, 131.3, 129.0, 128.7, 125.8, 124.7, 122.2, 117.2, 56.2, 52.4, 48.9, 40.3. |
| 54 | N-(3-(4-benzylpipe ridin-1-yl)propyl) naphthalen -2-sulfonamide | ¹H NMR (500 MHz, CDCl₃) : δ 8.47-8.30 (m, 1H), 7.99-7.90 (m, 3H), 7.85-7.82 (m, 1H), 7.66-7.60 (m, 2H), 7.31-7.27 (m, 2H), 7.22-7.14 (m, 3H), 3.10-3.07 (m, 2H), 2.83 (d, J = 11.0 Hz, 2H), 2.56 (d, J = 17.3 Hz, 2H), 2.33-2.30 (m, 2H), 1.82-1.77 (m, 2H), 1.67 (d, J = 12.8 Hz, 2H), 1.60-1.56 (m, 3H), 1.36-1.28 (m, 2H); ¹³C NMR (125 MHz, CDCl₃) : δ 140.6, 137.0, 134.6, 132.2, 129.3, 129.1, 129.0, 128.5, 128.2, 128.1, 127.9, 127.4, 125.8, 122.4, 58.6, 53.8, 44.7, 42.9, 38.0, 32.3, 23.7. |
| 55 | N-(3-(4-benzylpipe ridin-1-yl)propyl) naphthalen -1-sulfonamide | ¹H NMR (500 MHz, CDCl₃) : δ 8.74-8.72 (m, 1H), 8.29-8.27 (m, 1H), 8.07-8.05 (m, 1H), 7.97-7.95 (m, 1H), 7.67-7.53 (m, 3H), 7.33-7.18 (m, 5H), 2.97-2.95 (m, 2H), 2.86 (d, J = 11.9 Hz, 2H), 2.60 (d, J = 7.2 Hz, 2H), 2.29-2.26 (m, 2H), 1.80-1.76 (m, 2H), 1.70 (d, J = 12.7 Hz, 2H), 1.60-1.51 (m, 3H), 1.41-1.34 (m, 2H); ¹³C NMR (125 MHz, CDCl₃): δ 140.5, 134.6, 134.2, 133.7, 129.6, 129.0, 128.9, 128.2, 128.1, 127.8, 126.6, 125.8, 124.4, 124.1, 58.4, 53.7, 44.3, 42.9, 37.9, 32.1, 23.6. |
| 56 | N-(3-(4-benzylpipe ridin-1-yl)propyl) -[1,1'-biphenyl]-4-sulfonamide | ¹H NMR (500 MHz, CDCl₃): δ 7.97-7.94 (m, 2H), 7.74-7.73 (m, 2H), 7.64-7.63 (m, 2H), 7.51-7.41 (m, 3H), 7.31-7.16 (m, 5H), 3.13-3.10 (m, 2H), 2.88 (d, J = 13.0 Hz, 2H), 2.57 (d, J = 7.2 Hz, 2H), 2.40-2.37 (m, 2H), 1.87-1.82 (m, 2H), 1.70-1.50 (m, 5H), 1.37-1.29 (m, 2H); ¹³C NMR (125 MHz, CDCl₃) : δ 145.0, 140.0, 139.2, 138.8, 129.0, 128.9, 128.3, 128.1, 127.4, 127.3, 127.2, 125.7, 58.3, 53.7, 44.3, 42.8, 37.8, 32.1, 23.8. |
| 57 | N-(3-(4-benzylpipe ridin-1-yl)propyl) quinolin-2-sulfonamide | ¹H NMR (500 MHz, CDCl₃): δ 9.00-8.98 (m, 1H), 8.42-8.40 (m, 1H), 8.26-8.24 (m, 1H), 8.03-8.01 (m, 1H), 7.60-7.58 (m, 1H), 7.53-7.50 (m, 1H), 7.26-7.09 (m, 5H), 6.90 ((s, 1H), 2.90 (t, J = 6.5 Hz, 2H), 2.74 (d, J = 11.8, 2H), 2.49 (d, J =7.1, 2H), 2.23-2.20 (m, 2H), 1.75-1.70 (m, 2H), 1.63-1.41 (m, 5H), 1.29-1.21 (m, 2H); ¹³C NMR (125 MHz, CDCl₃) : δ 151.1, 143.4, 140.6, 137.0, 135.9, 133.3, 131.4, 129.1, 129.0, 128.2, 125.8, 125.6, 122.2, 56.6, 53.8, 43.1, 42.7, 37.9, 32.1, 29.2, 26.2. |
| 58 | N-(3-(4-benzylpipe ridin-1-yl)propyl) -4'-fluoro-[1,1'-biphenyl]-4-sulfonamide | ¹H NMR (500 MHz, CDCl₃): δ 7.95-7.92 (m, 2H), 7.70-7.67 (m, 2H), 7.61-7.57 (m, 2H), 7.31-7.16 (m, 7H), 3.11 (t, J = 5.5 Hz, 2H), 2.87 (d, J = 11.5 Hz, 2H), 2.57 (d, J = 7.5 Hz, 2H) 2.39-2.37 (m, 2H), 1.86-1.81 (m, 2H), 1.70-1.52 (m, 5H), 1.36-1.25 (m, 2H); ¹³C NMR (125 MHz, CDCl₃): δ 164.0, 162.1, 144.1, 140.6, 138.9, 135.54, 135.52, 129.0 (t, J = 30 HZ), 128.2, 127.5 (d, J = 18.7 Hz), 125.8, 116.0 (d, J = 21.1 Hz), 58.5, 53.8, 44.6, 43.0, 38.0, 32.3, 23.9 |
| 59 | N-(3-(4-benzylpipe ridin-1-yl)propyl) -4'-chloro-[1,1'-biphenyl]-4-sulfonamide | ¹H NMR (500 MHz, CDCl₃): δ 7.94-7.91 (m, 2H), 7.70-7.68 (m, 2H), 7.57-7.54 (m, 2H), 7.32-7.27 (m, 2H), 7.22-7.15 (m, 3H), 3.10 (t, J = 5.5 Hz, 2H), 2.88 (d, J = 11.5 Hz, 2H), 2.57 (d, J = 7.5 Hz, 2H) 2.40-2.38 (m, 2H), 1.87-1.82 (m, 2H), 1.70-1.51 (m, 5H), 1.36-1.24 (m, 2H); ¹³C NMR (125 MHz, CDCl₃) : δ 143.8, 140.6, 139.3, 137.8, 134.6, 129.2, 129.1, 128.5, 128.2, 127.6, 127.4, 125.9, 58.6, 53.9, 44.6, 42.9, 37.9, 32.3, 23.8. |
| 60 | N-(3-(4-benzylpipe ridin-1-yl)propyl) -4'-methyl-[1,1'-biphenyl] - 4-sulfonamide | ¹H NMR (500 MHz, CDCl₃) : δ 7.92-7.89 (m, 2H), 7.72-7.70 (m, 2H), 7.54-7.52 (m, 2H), 7.31-7.29 (m, 4H), 7.22-7.15 (m, 3H), 3.11-3.09 (m, 2H), 2.86 (d, J = 11.5 Hz, 2H), 2.57 (d, J = 7.5 Hz, 2H) 2.43 (s, 3H), 2.37 (t, J = 5.5 Hz, 2H), 1.85-1.80 (m, 2H), 1.70-1.63 (m, 5H), 1.36-1.28 (m, 2H); ¹³C NMR (125 MHz, CDCl₃): δ 145.1, 140.6, 138.5, 138.4, 136.5, 129.7, 129.1, 128.2, 127.5, 127.3, 127.1, 125.8, 58.6, 53.9, 44.7, 43.0, 38.0, 32.3, 23.8, 21.2. |
| 61 | N-(3-(4-benzylpipe ridin-1-yl)propyl) | ¹H NMR (500 MHz, CDCl₃): δ 7.91-7.88 (m, 2H), 7.70-7.67 (m, 2H), 7.59-7.56 (m, 2H), 7.31-7.26 (m, 2H), 7.22-7.15 (m, 3H), 7.03-7.00 (m, 2H), 3.88 (s, 3H), |
| | -4'-methoxy-[1,1'-biphenyl]-4-sulfonamide | 3.11-3.09 (m, 2H), 2.87 (d, J = 11.5 Hz, 2H), 2.57 (d, J = 7.0 Hz, 2H), 2.37 (t, J = 5.5 Hz, 2H), 1.85-1.80 (m, 2H), 1.69-1.51 (m, 5H), 1.35-1.25 (m, 2H); ¹³C NMR (125 MHz, CDCl₃) : δ 160.0, 144.7, 140.6, 138.1, 131.8, 129.1, 128.4, 128.2, 127.5, 126.9, 125.8, 114.5, 58.6, 55.4, 53.9, 44.6, 32.2, 23.8. |
| 62 | N-(3-(4-benzylpipe ridin-1-yl)propyl) naphthalen -2-sulfonamide | ¹H NMR (500 MHz, CDCl₃) : δ 8.44 (d, J = 1.25 Hz, 1H), 7.95-7.85 (m, 4H), 7.63-7.56 (m, 2H), 7.32-7.18 (m, 5H), 3.13-3.10 (m, 2H), 2.96 (d, J = 11.9, 2H), 2.52-2.46 (m, 1H), 2.40-2.37 (m, 2H), 2.03-1.95 (m, 2H), 1.87-1.75 (m, 4H), 1.64-1.60 (m, 2H); ¹³C NMR (125 MHz, CDCl₃) : δ 145.8, 137.0, 134.7, 132.2, 129.34, 129.3, 129.1, 128.6, 128.5, 128.2, 127.9, 122.5, 126.9, 126.3, 122.4, 58.4, 54.2, 44.4, 42.4, 33.5, 24.0. |
| 63 | N-(3-(4-benzylpipe ridin-1-yl)propyl) naphthalen -1-sulfonamide | ¹H NMR (500 MHz, CDCl₃): δ 8.79-8.77 (m, 1H), 8.31-8.30 (m, 1H), 8.07-8.05 (m, 1H), 7.96-7.94 (m, 1H), 7.65-7.53 (m, 3H), 7.37-7.22 (m, 5H), 3.02-3.00 (m, 4H), 2.57-2.51 (m, 1H), 2.37-2.35 (m, 2H), 2.06-1.82 (m, 6H), 1.66-1.61 (m, 2H); ¹³C NMR (125 MHz, CDCl₃) : δ 145.7, 134.6, 134.1, 133.7, 129.5, 128.9, 128.3, 128.1, 127.7, 126.7, 126.6, 126.1, 124.3, 124.1, 58.2, 54.0, 44.1, 42.3, 33.3, 23.7. |
| 64 | N-(3-(4-benzylpipe ridin-1-yl)propyl) -[1,1'-biphenyl] - 4-sulfonamide | ¹H NMR (500 MHz, CDCl₃) : δ 7.94 (d, J = 8.1, 2H), 7.69 (d, J = 8.1, 2H), 7.58 (d, J = 7.6, 2H), 7.45-7.36 (m, 3H), 7.30-7.16(m, 5H), 3.13-3.11 (m, 2H), 2.99 (d, J = 3.5 Hz, 2H), 2.51-2.41 (m, 3H), 2.01-1.62 (m, 8H); ¹³C NMR (125 MHz, CDCl₃) : δ 145.9, 145.2, 139.2, 138.9, 129.1, 128.5, 128.4, 127.6, 127.5, 127.3, 126.9, 126.3, 58.2, 54.2, 44.3, 42.5, 33.5, 24.3. |
| 65 | N-(3-(4-phenylpipe ridin-1-yl)propyl) quinolin-2-sulfonamid e | ¹H NMR (500 MHz, CDCl₃) : δ 9.01-9.00 (m, 1H), 8.44-8.42 (m, 1H), 8.27-8.25 (m, 1H), 8.04-8.02 (m, 1H), 7.64-7.61 (m, 1H), 7.53-7.51 (m, 1H), 7.28-7.15 (m, 5H), 6.84 (s, 1H), 2.94 (t, J = 6.5 H, 2H), 2.88 (d, J = 2.9 Hz, 2H), 2.47-2.41 (m, 1H), 2.31 (t, J = 6.95 Hz, 2H), 1.95-1.90 (m, 2H), 1.80-1.64 (m, 6H); ¹³C NMR (125 MHz, CDCl₃) : δ 151.2, 146.2, 143.4, 137.0, 136.0, 133.2, 131.5, 128.8, 128.4, 126.8, 126.1, 125.7, 122.2, 56.4, 54.3, 42.6, 42.5, 33.4, 26.4. |
| 66 | N-(2-(4-benzylpipe razin-1-yl)propyl) naphthalen -2-sulfonamide | ¹H NMR (500 MHz, CDCl₃): δ 8.44-8.43 (m, 1H), 7.97-7.90 (m, 4H), 7.85-7.84 (m, 1H), 7.66-7.56 (m, 2H), 7.34-7.25 (m, 5H, overlapped with CDCl₃)), 3.53 (s, 2H), 3.11-3.09 (m, 2H), 2.50-2.37 (m, 10H), 1.63-1.59 (m, 2H); ¹³C NMR (125 MHz, CDCl₃) : δ 138.1, 137.0, 134.7, 132.2, 129.3, 129.2, 129.1, 128.6, 128.2, 127.9, 127.5, 127.1, 122.4, 62.9, 58.2, 53.2, 53.1, 44.5, 23.7. |
| 67 | N-(2-(4-benzylpipe razin-1-yl)propyl) naphthalen -1-sulfonamide | ¹H NMR (500 MHz, CDCl₃) : δ 8.71-8.70 (m, 1H), 8.26-8.25 (m, 1H), 8.05-8.03 (m, 1H), 7.95-7.93 (m, 1H), 7.62-7.51 (m, 3H), 7.36-7.25 (m, 5H), 3.55 (s, 2H), 2.94 (t, J = 5.5 Hz, 2H), 2.53-2.30 (m, 10H), 1.61-1.56 (m, 2H); ¹³C NMR (125 MHz, CDCl₃): δ 138.1, 134.6, 134.2, 133.8, 129.7, 129.1, 129.0, 128.2, 127.9, 127.0, 126.6, 124.5, 124.1, 62.9, 58.1, 53.1, 53.0, 44.3, 23.5. |
| 68 | N-(3-(4-phenylpipe razin-1-yl)propyl) -[1,1'-biphenyl]-4-sulfonamide | ¹H NMR (500 MHz, CDCl₃): δ 7.94-7.91 (m, 2H), 7.73-7.71 (m, 2H), 7.63-7.60 (m, 2H), 7.51-7.41 (m, 3H), 7.33-7.41 (m, 5H, overlapped with CDCl₃), 3.53 (s, 2H), 3.11-3.10 (m, 2H), 2.49-2.42 (m, 10H), 1.67-1.62 (m, 2H); ¹³C NMR (125 MHz, CDCl₃) : δ 145.2, 139.4, 138.8, 138.2, 129.1, 129.0, 128.4, 128.2, 127.6, 127.5, 127.3, 127.1, 62.7, 58.2, 53.2, 53.1, 44.5, 27.8. |
| 69 | N-(3-(4-phenylpipe razin-1-yl)propyl) quinolin-2-sulfonamide | ¹H NMR (500 MHz, CDCl₃) : δ 8.99 (dd, J = 4.25, 1.5 Hz, 1H), 8.42 (dd, J = 7.25, 1.5 Hz, 1H), 8.28-8.26 (m, 1H), 8.04 (d, J = 8Hz, 1H) 1H), 7.64-7.61 (m, 1H), 7.55-7.52 (m, 1H), 7.30-7.22 ((m, 5H), 6.82 (s, 1H), 3.49 (s, 2H), 2.92 (t, J = 6.5 Hz, 2H), 2.44-2.27 (m, 10H), 1.65-1.60 (m, 2H); ¹³C NMR (125 MHz, CDCl₃) : δ 151.0, 143.2, 137.9, 136.8, 135.7, 133.1, 131.2, 129.2, 128.6, 128.0, 126.8, 125.9, 122.0, 62.8, |
| | | 56.0, 53.0, 52.9, 52.8, 42.4, 25.9. |
| 70 | N-(3-(4-(4-chlorophen yl)piperaz in-1-yl)propyl) naphthalen -2-sulfonamide | ¹H NMR (500 MHz, CDCl₃): δ 8.42 (d, J = 3 Hz, 1H), 7.97-7.90 (m, 2H), 7.83-7.80 (m, 1H), 7.66-7.60 (m, 3H), 7.23-7.20 (m, 2H), 6.84-6.81(M, 2H) 3.18-3.12 (m, 6H), 2.55 (t, J = 5.0 Hz, 4H), 2.46-2.44 (m, 2H), 1.71-1.66 (m, 2H); ¹³C NMR (125 MHz, CDCl₃): δ 149.6, 136.9, 134.7, 132.2, 129.4, 129.1, 129.0, 128.6, 128.2, 127.9, 127.5, 124.8, 122.3, 117.4, 57.9, 53.0, 49.1, 44.1, 30.9, 24.1. |
| 71 | N-(3-(4-(4-chlorophen yl)piperaz in-1-yl)propyl) naphthalen -1-sulfonamide | ¹H NMR (500 MHz, CDCl₃) : δ 8.66 (d, J = 10 Hz, 1H), 8.27-8.25 (m, 1H), 8.06 (d, J = 8Hz, 1H), 7.96-7.94 (m, 1H), 7.62-7.58 (m, 3H), 7.24-7.21 (m, 2H), 6.87-6.84 (m, 2H), 3.20 (t, J = 5Hz, 4H), 3.01-2.99 (m, 2H), 2.53 (t, J = 5 Hz, 4H), 2.40-2.38 (m, 2H), 1.68-1.64 (m, 2H); ¹³C NMR (125 MHz, CDCl₃) : δ 149.6, 134.6, 134.3, 134.0, 129.8, 129.1, 129.0, 128.2, 128.0, 126.7, 124.9, 124.4, 124.2, 117.4, 58.0, 53.0, 49.2, 44.1, 30.9, 24.0. |
| 72 | N-(3-(4-(4-chlorophen yl)piperaz in-1-yl)propyl) -[1,1'-biphenyl]-4-sulfonamide | ¹H NMR (500 MHz, CDCl₃): δ 7.93-7.91 (m, 2H), 7.74-7.71 (m, 2H), 7.62-7.60 (m, 2H), 7.51-7.47 (m, 2H), 7.45-7.41 (m, 1H), 7.23-7.20 (m, 2H), 6.86-6.82 (m, 2H), 3.19-3.15 (m, 6H), 2.58 (t, J = 5Hz, 4H), 2.51-2.49 (m, 2H), 1.75-1.70 (m, 2H), ; ¹³C NMR (125 MHz, CDCl₃): δ 149.6, 145.3, 139.3, 138.8, 129.1, 129.0, 128.5, 127.6, 127.5, 127.3, 124.8, 117.4, 57.8, 53.0, 49.2, 44.0, 24.3. |
| 73 | N-(3-(4-(4-chlorophen yl)piperaz in-1-yl)propyl) quinolin-2-sulfonamide | ¹H NMR (500 MHz, CDCl₃): δ 9.00-8.99 (m, 1H), 8.45 (dd, J = 7.1, 5.0 Hz, 1H), 8.29-8.27 (m, 1H), 8.05 (d, J = 8.5 Hz,1H), 7.67-7.64 (m, 1H), 7.56-7.53 (m, 1H), 7.20-7.17 (m, 2H), 6.83-6.80 (m, 2H), 6.82 (s, 1H), 3.13-3.11 (m, 4H), 2.96 (s, 2H), 2.49 (t, J = 5Hz, 4H) 2.39-2.36 (m, 2H), 1.72-1.67 (m, 2H); ¹³C NMR (125 MHz, CDCl₃) : δ 151.1, 149.8, 143.4, 137.0, 136.0, 133.2, 131.4, 128.9, 128.8, 125.8, 124.5, 122.2, 117.2, 56.0, 53.0, 49.0, 42.3, 26.3. |
| 74 | 4'-fluoro-N-(3-(4-(3-phenylprop yl)piperid in-1-yl)propyl) -[1,1'-biphenyl]-4-sulfonamid e | ¹H NMR (500 MHz, CDCl₃): δ 7.95-7.89 (m, 2H), 7.67-7.65 (m, 2H), 7.60-7.56 (m, 2H), 7.30-7.26 (m, 4H), 7.20-7.15 (m, 4H, overlapped with CDCl₃), 3.11-3.08 (m, 2H), 2.86 (d, J = 11.5 Hz, 2H), 2.61-2.58 (m, 2H), 2.37 (t, J = 5.5 Hz, 2H), 1.85-1.60 (m, 8H), 1.32-1.23 (m, 5H); ¹³C NMR (125 MHz, CDCl₃): δ 144.0, 142.6, 138.9, 128.9 (d, J = 35 Hz), 128.29 (d, J = 45Hz), 127.5 (d, J = 85 Hz), 125.6, 115.9 (d, J = 85 Hz), 58.7, 53.9, 44.6, 36.1, 35.7, 32.4, 28.8, 23.7. |
| 75 | N-(3-(4-(3-phenylprop yl)piperid in-1-yl)propyl) -[1,1'-biphenyl]-4-sulfonamid e | ¹H NMR (500 MHz, CDCl₃) : δ 7.93-7.91 (m, 2H), 7.74-7.71 (m, 2H), 7.64-7.62 (m, 2H), 7.51-7.47 (m, 2H), 7.44-7.41 (m, 1H), 7.31-7.17 (m, 5H, overlapped with CDCl₃), 3.11 (t, J = 5.5 Hz, 2H), 2.87 (d, J = 11.0 Hz, 2H), 2.62-2.59 (m, 2H), 2.38 (t, J = 5.5 Hz, 2H), 1.86 (d, J = 11 Hz, 2H), 1.73-1.61 (m, 6H), 1.34-1.25 (m, 5H); ¹³C NMR (125 MHz, CDCl₃): δ 145.1, 142.7, 139.4, 138.9, 129.0, 128.4, 128.3, 127.6, 127.5, 127.3, 125.6, 58.7, 54.0, 44.7, 36.1, 36.0, 35.7, 32.5, 28.8, 23.7. |
| 76 | N-(3-(4-(3-phenylprop yl)piperaz in-1-yl)propyl) -[1,1'-biphenyl]-4-sulfonamide | ¹H NMR (500 MHz, CDCl₃): δ 7.94-7.92 (m, 2H), 7.74-7.71 (m, 2H), 7.63-7.61 (m, 2H), 7.50-7.40 (m, 4H), 7.30-7.17 (m, 3H, overlapped with CDCl₃), 3.12-3.10 (m, 2H), 2.66-2.40 (m, 14H), 1.86-1.80 (m, 2H), 1.68-1.64 (m, 2H); ¹³C NMR (125 MHz, CDCl₃) : δ 145.2, 142.0, 139.3, 138.8, 129.1, 128.5, 128.4, 128.3, 127.6, 127.5, 127.3, 125.8, 58.0, 57.8, 53.2, 53.0, 42.2, 33.7, 28.6, 23.9. |
| 77 | N-(4-(4-benzylpipe ridin-1-yl)butyl)-[1,1'-biphenyl]-4-sulfonamide | ¹H NMR (500 MHz, CDCl₃) : δ 7.95-7.92 (m, 2H), 7.74-7.71 (m, 2H), 7.64-7.62 (m, 2H), 7.51-7.47 (m, 2H), 7.44-7.41 (m, 1H), 7.30-7.26 (m, 2H), 7.21-7.15 (m, 3H), 3.02-3.00 (m, 2H), 2.93 (d, J = 11.5 Hz, 2H), 2.58 (d, J = 6.5 Hz, 2H) 2.29-2.27 (m, 2H), 1.91-1.86 (m, 2H), 1.67-1.44 (m, 7H), 1.33-1.24 (m, 2H); ¹³C NMR (125 MHz, CDCl₃) : δ 144.9, 140.7, 139.8, 139.5, 129.1, 129.0, 128.3, 128.2, 127.5, 127.4, 127.3, 125.8, 58.4, 53.7, 43.2, 43.0, 38.0, 31.5, 28.9, 25.2, 22.7. |
| 78 | N-(4-(4-(3-phenylprop yl)piperaz in-1-yl)butyl)-[1,1'-biphenyl]-4-sulfonamide | ¹H NMR (500 MHz, CDCl₃): δ 7.93-7.90 (m, 2H), 7.74-7.68 (m, 2H), 7.62-7.60 (m, 2H), 7.50-7.47 (m, 2H), 7.44-7.40 (m, 1H), 7.30-7.26 (m, 1H, overlapped with CDCl₃), 7.20-7.18 (m, 4H), 3.01-2.99 (m, 2H), 2.66-2.35 (m, 16H), 1.88-1.82 (m, 4H) ; ¹³C NMR (125 MHz, CDCl₃): δ 167.8, 140.9, 138.2, 129.2, 128.7, 128.6, 128.2, 127.1, 127.0, 63.1, 57.6, 53.2, 53.1, 53.0, 48.7, 27.4, 23.6. |
| 79 | N-(4-(4-(3-phenylprop yl)piperid in-1-yl)butyl)-[1,1'-biphenyl]-4-sulfonamide | ¹H NMR (500 MHz, CDCl₃) : δ 7.92-7.89 (m, 2H), 7.69-7.67 (m, 2H), 7.62-7.60 (m, 2H), 7.50-7.47 (m, 2H), 7.43-7.41 (m, 1H), 7.30-7.27 (m, 2H), 7.19-7.16 (m, 3H), 3.01-2.99 (m, 2H), 2.94 (d, J = 12.0 Hz, 2H), 2.60 (t, J = 5.5 Hz, 2H), 2.30 (t, J = 5.5 Hz, 2H), 1.94-1.89 (d, J = 11 Hz, 2H), 1.71-1.56 (m, 8H), 1.44-1.31 (m, 5H); ¹³C NMR (125 MHz, CDCl₃): δ 144.8, 142.7, 139.8, 139.5, 129.0, 128.4, 128.3, 128.2, 127.5, 127.4, 127.3, 125.6, 58.4, 53.8. 43.2, 36.2, 36.1, 35.7, 31.6, 28.9, 28.8, 25.1. |

### <Experimental Example 1> Evaluation of Triple Reuptake Inhibitory Effect

In order to evaluate the reuptake inhibitory effect of the compounds of the Examples according to the present invention on serotonin, norepinephrine and dopamine, the experiments below were performed, and the results are shown in Table 17.

To evaluate the reuptake inhibition effect on dopamine, the dopamine transporter cDNA was transfected using the HEK-293 cell line, and then sub-seeded in a 24 well plate the next day. After removing the medium, each compound of the Examples or Comparative Examples diluted to 1 µM in uptake buffer was added to a concentration of 200 µl/well and cultured at 37°C for 15 minutes. 100 µl of uptake buffer containing 20-30 nM [3H]-DA was added and cultured at 37°C for 5 minutes. The reaction solution was quickly removed and washed three times with 1 ml ice-cold uptake buffer. After dissolving the resultant in 1% SDS (0.5 ml), the degree of binding of radioisotope-labeled dopamine to the transporter was measured.

For norepinephrine (NE) and serotonin (5-HT), the degree of binding of radioisotope-labeled norepinephrine or serotonin to the transporter was measured in the same manner as above, except that cells transfected with NE or 5-HT transporter cDNA were used and and [3H]-NE or [3H]-5-HT was used as a substrate.

In addition, when evaluating the reuptake inhibitory effect on dopamine, norepinephrine, and serotonin, the same method was performed without treating each compound of the Examples or Comparative Examples as a control, the degree of binding of radioisotope-labeled dopamine, norepinephrine, and serotonin to the transporter was measured, and by using this as a reference (100%), the triple reuptake inhibitory effect was evaluated as a relative ratio (Ratio%). That is, in the control group, the binding of dopamine, norepinephrine, and serotonin to transporters is not inhibited, but when each compound of the Examples is treated and thus the binding is inhibited, the Ratio% value decreases.

Meanwhile, the Ratio% values were compared, and in the case of the compounds of Examples 6, 10, 13, 14, 17, 20, 21, 22, 30, 31, 32, 34, 36, 38, 39, 40, 54, 55, 56, 57, 58 and 73 with excellent reuptake inhibitory effects on dopamine, norepinephrine and serotonin, the concentration at which the Ratio% is 50% was calculated using a total of 6 concentrations (1 nM, 5 nM, 10 nM, 50 nM, 100 nM, and 1 µM) and expressed as IC₅₀.

**[Table 17]**

| Example | Triple reuptake inhibition (Ratio%) | | | Triple reuptake inhibition (IC50, µM) | | |
|---|---|---|---|---|---|---|
| | SERT | NET | DAT | SERT | NET | DAT |
| 1 | 84.98 | 77.88 | 65.88 | | | |
| 2 | 100.21 | 61.32 | 73.55 | | | |
| 3 | 62.10 | 56.36 | 50.09 | | | |
| 4 | 76.57 | 80.47 | 88.71 | | | |
| 5 | 106.55 | 94.03 | 100.55 | | | |
| 6 | 29.04 | 81.97 | 54.56 | 0.457 | | 1.99 |
| 7 | 93.29 | 88.38 | 99.63 | | | |
| 8 | 98.57 | 79.30 | 90.67 | | | |
| 9 | 101.11 | 96.32 | 109.25 | | | |
| 10 | 24.45 | 13.02 | 41.80 | 0.483 | 0.168 | 0.612 |
| 11 | 18.04 | 73.79 | 73.16 | | | |
| 12 | 62.27 | 74.48 | 20.69 | | | |
| 13 | 41.46 | 11.04 | 25.55 | 1.08 | 0.112 | 0.872 |
| 14 | 55.67 | 22.30 | 39.90 | 1.75 | 0.173 | 1.256 |
| 15 | 77.80 | 62.09 | 93.13 | | | |
| 16 | 74.25 | 53.45 | 93.59 | | | |
| 17 | 9.28 | 58.66 | 40.96 | 0.057 | 1.557 | 5.175 |
| 18 | | 66.54 | 65.67 | | | |
| 19 | 67.86 | 64.78 | 57.52 | | | |
| 20 | 40.46 | 30.29 | 56.29 | 0.504 | 0.221 | 2.156 |
| 21 | 38.74 | 26.67 | 57.35 | 0.455 | 0.946 | 4.72 |
| 22 | 38.07 | 36.85 | 31.02 | 0.609 | 0.550 | 1.506 |
| 23 | 41.32 | 83.63 | 99.56 | | | |
| 24 | 60.27 | 58.70 | 99.30 | | | |
| 25 | 84.44 | 102.91 | 111.51 | | | |
| 26 | 50.98 | 93.31 | 97.83 | | | |
| 27 | 76.16 | 36.89 | 88.17 | | | |
| 28 | 26.63 | 64.36 | 94.53 | | | |
| 29 | 39.39 | 54.04 | 89.50 | | | |
| 30 | 23.18 | 37.58 | | 0.538 | 1.704 | 11.62 |
| 31 | 14.27 | 18.93 | | 0.216 | 0.041 | 1.054 |
| 32 | 30.80 | 20.42 | 45.64 | 0.386 | 0.749 | 2.137 |
| 33 | 59.74 | 68.93 | 66.67 | | | |
| 34 | 57.25 | 20.03 | 16.35 | 2.592 | 0.612 | 0.942 |
| 35 | 53.08 | 51.99 | 16.12 | | | |
| 36 | 72.85 | 79.12 | 30.48 | | | 0.843 |
| 37 | 57.76 | 93.37 | 99.98 | | | |
| 38 | 34.20 | 21.54 | 56.59 | | | 0.4928 |
| 39 | 15.77 | 18.46 | 31.26 | | | 1.156 |
| 40 | 33.23 | 33.45 | 41.80 | 3.084 | 1.05 | 3.921 |
| 41 | 64.15 | 34.97 | 80.21 | | | |
| 42 | 64.15 | 34.97 | 80.21 | | | |
| 43 | 64.15 | 34.97 | 80.21 | | | |
| 44 | 15.55 | 14.04 | 37.93 | | | |
| 45 | 56.73 | 45.04 | 89.88 | | | |
| 46 | 49.64 | 87.27 | 83.08 | | | |
| 47 | 49.64 | 87.27 | 83.08 | | | |
| 48 | 49.64 | 87.27 | 83.08 | | | |
| 49 | 45.16 | 87.27 | 86.50 | | | |
| 50 | 45.16 | 87.27 | 86.50 | | | |
| 51 | 47.19 | 64.45 | 68.53 | | | |
| 52 | 47.19 | 64.45 | 68.53 | | | |
| 53 | 47.19 | 64.45 | 68.53 | | | |
| 54 | 46.95 | 36.68 | 45.75 | 1.936 | 1.253 | 0.948 |
| 55 | 46.18 | 31.83 | 35.21 | 1.45 | 1.30 | 1.553 |
| 56 | 56.50 | 68.65 | 61.90 | 0.7497 | 0.6411 | 0.4204 |
| 57 | 53.66 | 30.54 | 13.09 | 0.3446 | 1.354 | 4.481 |
| 58 | 50.76 | 44.79 | 55.51 | 1.841 | 0.4289 | 0.6468 |
| 59 | 29.78 | 44.79 | 15.16 | | | |
| 60 | 17.76 | 44.79 | 9.15 | | | |
| 61 | 30.78 | 44.79 | 29.62 | | | |
| 62 | 64.15 | 34.97 | 80.21 | | | |
| 63 | 64.15 | 34.97 | 80.21 | | | |
| 64 | 56.73 | 45.04 | 89.88 | | | |
| 65 | 56.73 | 45.04 | 89.88 | | | |
| 66 | 49.64 | 87.27 | 83.08 | | | |
| 67 | 49.64 | 87.27 | 83.08 | | | |
| 68 | 49.64 | 87.27 | 83.08 | | | |
| 69 | 45.16 | 87.27 | 86.50 | | | |
| 70 | 45.16 | 87.27 | 86.5 | | | |
| 71 | 47.19 | 64.45 | 68.53 | | | |
| 72 | 47.19 | 64.45 | 68.53 | | | |
| 73 | 47.19 | 64.45 | 68.53 | 0.1015 | | |
| 74 | 61.48 | 44.79 | 76.23 | | | |
| 75 | 61.48 | 44.79 | 76.23 | | | |
| 76 | 44.91 | 44.79 | 19.97 | | | |
| 77 | 40.45 | 44.79 | 25.71 | | | |
| 78 | 32.90 | 44.79 | 25.52 | | | |
| 79 | 21.60 | 30.25 | 20.60 | | | |

In Table 16 above, SERT means a serotonin transporter, NET means a norepinephrine transporter, and DAT means a dopamine transporter.

As shown in Table 16 above,
it can be seen that the compounds of the Examples of the present invention can inhibit reuptake of serotonin, norepinephrine, and dopamine.

Therefore, the compound represented by Formula 1 according to the present invention is excellent in the triple reuptake inhibitory effect of serotonin, norepinephrine, and dopamine, and thus can be useful in the treatment of mental illness.

### INDUSTRIAL APPLICABILITY

The compound represented by Formula 1 according to the present invention is excellent in the triple reuptake inhibitory effect of serotonin, norepinephrine, and dopamine, and thus can be useful in the treatment of mental illness.

## Claims

1. A compound represented by the following Formula 1, an isomer thereof, a solvate thereof, a hydrate thereof, or a pharmaceutically acceptable salt thereof: 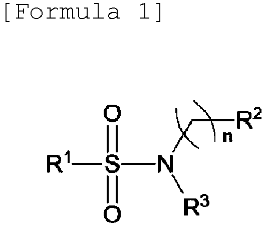 in Formula 1,
n is an integer of 0 to 5,
R¹ is unsubstituted or substituted aryl of C₆₋₁₀, or unsubstituted or substituted heteroaryl of 5 to 14 atoms, containing one or more heteroatoms selected from the group consisting of N, S and O,
the substituted aryl and the substituted heteroaryl are independently substituted with one or more substituents selected from the group consisting of -OH, halogen, unsubstituted or substituted linear or branched C₁₋₁₀ alkyl with one or more halogens, unsubstituted or substituted linear or branched C₁₋₁₀ alkoxy with one or more halogens, unsubstituted or substituted phenyl, -NR^{a}R^{b}, - NHC(=O)(CH₂)ₘNR^{a}R^{b} and phenoxy, where the substituted phenyl is substituted with linear or branched C₁₋₁₀ alkyl, linear or branched C₁₋₁₀ alkoxy or halogen, R^{a} and R^{b} are independently hydrogen or unsubstituted or substituted linear or branched C₁₋₁₀ alkyl with one or more halogens, or may form heterocycloalkyl of 5 to 6 atoms, containing one or more heteroatoms selected from the group consisting of N, O and S, together with a N atom bonded, and m is an integer of 0 to 5,
R² is unsubstituted or substituted heterocycloalkyl of 4 to 8 atoms, containing one or more heteroatoms selected from the group consisting of N, S and O,
the substituted heterocycloalkyl is substituted with one or more substituents selected from the group consisting of indole, -(CH₂)ₚC₆₋₁₀ aryl, and heterocycloalkyl of 5 to 6 atoms, containing one or more heteroatoms selected from the group consisting of N, O and S, where the indole and C₆₋₁₀ aryl are unsubstituted or independently substituted with one or more substituents selected from the group consisting of halogen, unsubstituted or substituted linear or branched C₁₋₅ alkyl with one or more halogens, and unsubstituted or substituted linear or branched C₁₋₅ alkoxy with one or more halogens, and p is an integer of 0 to 5; and
R³ is hydrogen or linear or branched C₁₋₅ alkyl; or
if n is 0, R² and R³ may form unsubstituted or substituted heterocycloalkyl of 4 to 8 atoms, containing one or more heteroatoms selected from the group consisting of N, S and O, together with a nitrogen atom bonded, the substituted heterocycloalkyl is substituted with indole or - (CH₂)_{q}C₆₋₁₀ aryl, and q is an integer of 0 to 5.

2. The compound, an isomer thereof, a solvate thereof, a hydrate thereof, or a pharmaceutically acceptable salt thereof according to claim 1, wherein
n is an integer of 0 to 4,
R₁ is unsubstituted or substituted aryl of C₆₋₁₀, or unsubstituted or substituted heteroaryl of 5 to 10 atoms, containing one or more heteroatoms selected from the group consisting of N, S and O,
the substituted aryl and the substituted heteroaryl are independently substituted with one or more substituents selected from the group consisting of -OH, halogen, unsubstituted or substituted linear or branched C₁₋₅ alkyl with one or more halogens, unsubstituted or substituted linear or branched C₁₋₅ alkoxy with one or more halogens, unsubstituted or substituted phenyl, -NR^{a}R^{b}, - NHC(=O) (CH₂)ₘNR^{a}R^{b} and phenoxy, where the substituted phenyl is substituted with linear or branched C₁₋₅ alkyl, linear or branched C₁₋₅ alkoxy or halogen, R^{a} and R^{b} are independently hydrogen or unsubstituted or substituted linear or branched C₁₋₅ alkyl with one or more halogens, or may form heterocycloalkyl of 5 to 6 atoms, containing one or more heteroatoms selected from the group consisting of N, O and S, together with a N atom bonded, and m is an integer of 0 to 4,
R² is unsubstituted or substituted heterocycloalkyl of 4 to 6 atoms, containing one or more heteroatoms selected from the group consisting of N, S and O,
the substituted heterocycloalkyl is substituted with one or more substituents selected from the group consisting of indole, -(CH₂)ₚ phenyl, and heterocycloalkyl of 5 to 6 atoms, containing one or more O, where the indole and phenyl are unsubstituted or independently substituted with one or more substituents selected from the group consisting of halogen, unsubstituted or substituted linear or branched C₁₋₃ alkyl with one or more halogens, and unsubstituted or substituted linear or branched C₁₋₃ alkoxy with one or more halogens, and p is an integer of 0 to 4; and
R³ is hydrogen or linear or branched C₁₋₃ alkyl; or
if n is 0, R² and R³ may form unsubstituted or substituted heterocycloalkyl of 4 to 6 atoms, containing one or more heteroatoms selected from the group consisting of N, S and O, together with a nitrogen atom bonded, the substituted heterocycloalkyl is substituted with indole or - (CH₂)_{q} phenyl, and q is an integer of 0 to 4.

3. The compound, an isomer thereof, a solvate thereof, a hydrate thereof, or a pharmaceutically acceptable salt thereof according to claim 1, wherein
n is an integer of 0 to 3,
R¹ is unsubstituted or substituted aryl of C₆₋₁₀, or unsubstituted or substituted heteroaryl of 5 to 9 atoms, containing one or more N,
the substituted aryl and the substituted heteroaryl are independently substituted with one or more substituents selected from the group consisting of -OH, halogen, unsubstituted or substituted linear or branched C₁₋₄ alkyl with one or more halogens, unsubstituted or substituted linear or branched C₁₋₄ alkoxy with one or more halogens, unsubstituted or substituted phenyl, -NR^{a}R^{b}, - NHC(=O) (CH₂)ₘNR^{a}R^{b} and phenoxy, where the substituted phenyl is substituted with linear or branched C₁₋₄ alkyl, linear or branched C₁₋₄ alkoxy or halogen, R^{a} and R^{b} are independently hydrogen or unsubstituted or substituted linear or branched C₁₋₄ alkyl with one or more halogens, or may form heterocycloalkyl of 5 to 6 atoms, containing one or more N, together with a N atom bonded, and m is an integer of 0 to 3,
R² is unsubstituted or substituted heterocycloalkyl of 6 atoms, containing one or more N,
the substituted heterocycloalkyl is substituted with one or more substituents selected from the group consisting of indole, -(CH₂)ₚ phenyl, and heterocycloalkyl of 6 atoms, containing one or more O, where the indole and phenyl are unsubstituted or independently substituted with one or more substituents selected from the group consisting of halogen, methyl, ethyl, methoxy, ethoxy and -CF₃, and p is an integer of 0 to 3; and
R³ is hydrogen or methyl; or
if n is 0, R² and R³ may form unsubstituted or substituted heterocycloalkyl of 6 atoms, containing one or more N, together with a nitrogen atom bonded, the substituted heterocycloalkyl is substituted with indole or -(CH₂)_{q} phenyl, and q is an integer of 0 to 3.

4. The compound, an isomer thereof, a solvate thereof, a hydrate thereof, or a pharmaceutically acceptable salt thereof according to claim 1, wherein
n is 0, 2, 3 or 4,
R₁ is unsubstituted or substituted phenyl, unsubstituted naphthyl, unsubstituted indole, unsubstituted or substituted pyridinyl, or unsubstituted quinolinyl,
the substituted phenyl and the substituted pyridinyl are independently substituted with one or more substituents selected from the group consisting of -OH, halogen, methoxy, butoxy, -OCF₃, -NH₂, -NHnBu, -NHC(=O)(CH₂)₂ piperidinyl, - NHC(=O) (CH₂)₂N(CH₃)₂, unsubstituted or substituted phenyl with methoxy, halogen or methyl, and phenoxy,
R² is substituted piperidinyl or substituted piperazinyl,
the substituted piperidinyl and substituted piperazinyl are independently substituted with one or more substituents selected from the group consisting of indole, -(CH₂)ₚ phenyl, and methyltetrahydropyrazinyl, where the indole and phenyl are unsubstituted or independently substituted with one or more substituents selected from the group consisting of halogen, methyl, methoxy and -CF₃, and p is an integer of 0 to 2; and
R³ is hydrogen; or
if n is 0, R² and R³ may form piperidinyl together with a nitrogen atom bonded, and the piperidinyl is substituted with indole or -(CH₂)_{q} phenyl.

5. The compound, an isomer thereof, a solvate thereof, a hydrate thereof, or a pharmaceutically acceptable salt thereof according to claim 1, wherein
n is 0, 2, 3 or 4;
R¹ is 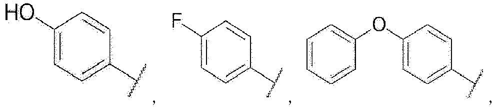 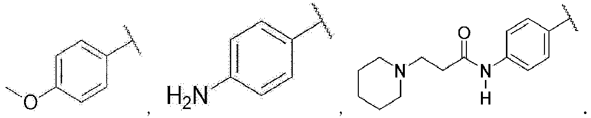 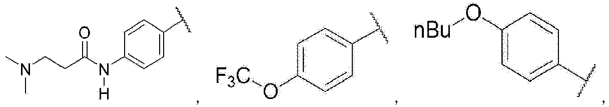 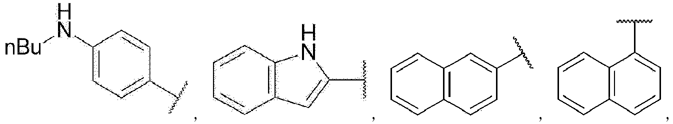 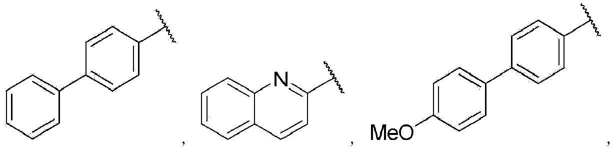 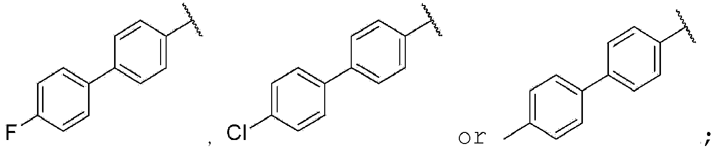
R² is 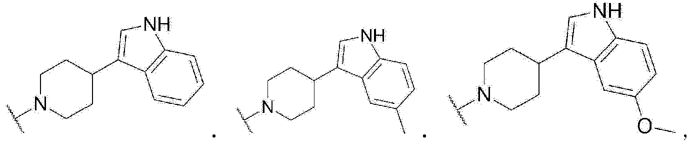 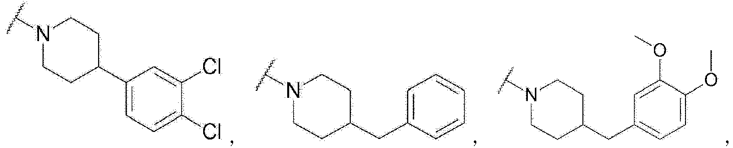 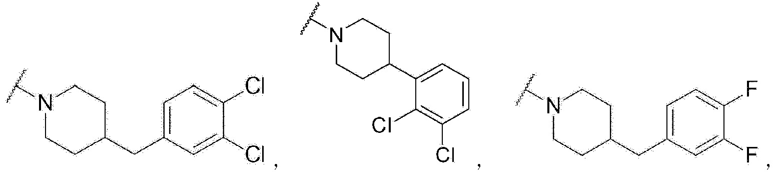 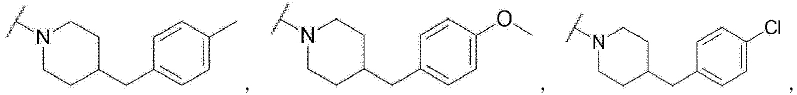 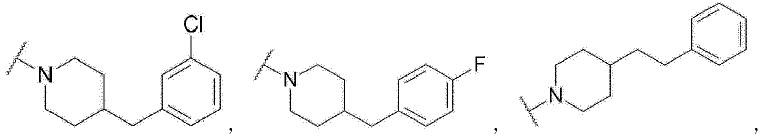 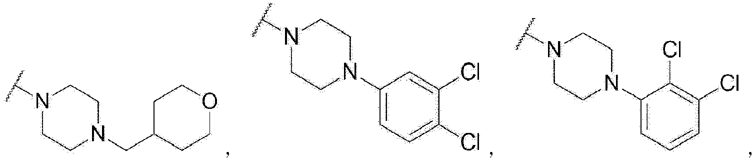 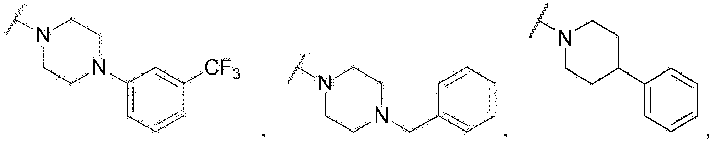 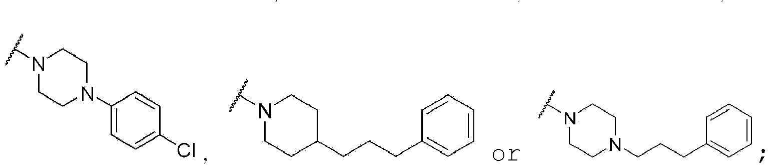 and
R³ is hydrogen; or
if n is 0, -NR²R³ is

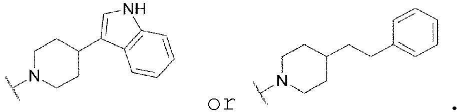

6. The compound, an isomer thereof, a solvate thereof, a hydrate thereof, or a pharmaceutically acceptable salt thereof according to claim 1, wherein the compound represented by Formula 1 is any one selected from the group consisting of the following compound group:
<1> N-(2-(4-benzylpiperidin-1-yl)ethyl)-4-hydroxybenzenesulfonamide;
<2> N-(2-(4-benzylpiperidin-1-yl)ethyl)-4-fluorobenzenesulfonamide;
<3> N-(2-(4-benzylpiperidin-1-yl)ethyl)-4-phenoxybenzenesulfonamide;
<4> 4-butoxy-N-(2-(4-(3,4-dichlorobenzyl)piperidin-1-yl)ethyl)benzenesulfonamide;
<5> 3-(1-((4-butoxyphenyl)sulfonyl)piperidin-4-yl)-1H-indole;
<6> N-(2-(4-(1H-indol-3-yl)piperidin-1-yl)ethyl)-4-butoxybenzenesulfonamide;
<7> 4-butoxy-N-(2-(4-(5-methyl-1H-indol-3-yl)piperidin-1-yl)ethyl)benzenesulfonamide;
<8> 4-butoxy-N-(2-(4-(5-methoxy-1H-indol-3-yl)piperidin-1-yl)ethyl)benzenesulfonamide;
<9> 1-((4-butoxyphenyl)sulfonyl)-4-phenethylpiperidin;
<10> N-(3-(4-benzylpiperidin-1-yl)propyl)-4-butoxybenzenesulfonamide;
<11> N-(3-(4-(1H-indol-3-yl)piperidin-1-yl)propyl)-4-butoxybenzenesulfonamide;
<12> N-(3-(4-benzylpiperidin-1-yl)propyl)-1H-indol-2-sulfonamide;
<13> N-(3-(4-benzylpiperidin-1-yl)propyl)-4-phenoxybenzenesulfonamide;
<14> N-(3-(4-benzylpiperidin-1-yl)propyl)-4-methoxybenzenesulfonamide;
<15> 4-amino-N-(3-(4-benzylpiperidin-1-yl)propyl)benzenesulfonamide;
<16> N-(3-(4-benzylpiperidin-1-yl)propyl)-4-(butylamino)benzenesulfonamide;
<17> N-(4-(N-(3-(4-benzylpiperidin-1-yl)propyl)sulfamoyl)phenyl)-3-(piperidin-1-yl)propaneamide;
<18> N-(4-(N-(3-(4-benzylpiperidin-1-yl)propyl)sulfamoyl)phenyl)-3-(dimethylamino)propaneamide;
<19> N-(3-(4-benzylpiperidin-1-yl)propyl)-4-butoxybenzenesulfineamide;
<20> 4-butoxy-N-(3-(4-(3,4-dichlorobenzyl)piperidin-1-yl)propyl)benzenesulfonamide;
<21> 4-butoxy-N-(3-(4-(3,4-difluorobenzyl)piperidin-1-yl)propyl)benzenesulfonamide;
<22> 4-butoxy-N-(3-(4-(3,4-dichlorophenyl)piperidin-1-yl)propyl)benzenesulfonamide;
<23> 4-butoxy-N-(3-(4-(3,4-dichlorophenyl)piperazin-1-yl)propyl)benzenesulfonamide;
<24> 4-butoxy-N-(3-(4-(2,3-dichlorophenyl)piperazin-1-yl)propyl)benzenesulfonamide;
<25> 4-butoxy-N-(3-(4-((tetrahydro-2H-pyran-4-yl)methyl)piperazin-1-yl)propyl)benzenesulfonamide;
<26> 4-butoxy-N-(3-(4-(3-(trifluoromethyl)phenyl)piperazin-1-yl)propyl)benzenesulfonamide;
<27> N-(3-(4-benzylpiperazin-1-yl)propyl)-4-butoxybenzenesulfonamide;
<28> 4-butoxy-N-(3-(4-(4-chlorobenzyl)piperidin-1-yl)propyl)benzenesulfonamide;
<29> 4-butoxy-N-(3-(4-(4-methylbenzyl)piperidin-1-yl)propyl)benzenesulfonamide;
<30> 4-butoxy-N-(3-(4-(4-methoxybenzyl)piperidin-1-yl)propyl)benzenesulfonamide;
<31> 4-butoxy-N-(3-(4-(3-chlorobenzyl)piperidin-1-yl)propyl)benzenesulfonamide;
<32> 4-butoxy-N-(3-(4-(4-fluorobenzyl)piperidin-1-yl)propyl)benzenesulfonamide;
<33> 4-butoxy-N-(3-(4-(2,3-dichlorophenyl)piperidin-1-yl)propyl)benzenesulfonamide;
<34> N-(3-(4-benzylpiperidin-1-yl)propyl)-4-(trifluoromethoxy)benzenesulfonamide;
<35> N-(3-(4-benzylpiperidin-1-yl)propyl)-5-butoxypyridin-2-sulfonamide;
<36> N-(2-(4-benzylpiperidin-1-yl)ethyl)-1H-indol-2-sulfonamide;
<37> 4-butoxy-N-(3-(4-(3,4-dimethoxybenzyl)piperidin-1-yl)propyl)benzenesulfonamide;
<38> N-(2-(4-benzylpiperidin-1-yl)ethyl)naphthalen-2-sulfonamide;
<39> N-(2-(4-benzylpiperidin-1-yl)ethyl)naphthalen-1-sulfonamide;
<40> N-(2-(4-benzylpiperidin-1-yl)ethyl)-[1,1'-biphenyl]-4-sulfonamide;
<41> N-(2-(4-benzylpiperidin-1-yl)ethyl)quinolin-2-sulfonamide;
<42> N-(2-(4-phenylpiperidin-1-yl)ethyl)naphthalen-2-sulfonamide;
<43> N-(2-(4-phenylpiperidin-1-yl)ethyl)naphthalen-1-sulfonamide;
<44> N-(2-(4-phenylpiperidin-1-yl)ethyl)-[1,1'-biphenyl]-4-sulfonamide;
<45> N-(2-(4-phenylpiperidin-1-yl)ethyl)quinolin-2-sulfonamide;
<46> N-(2-(4-benzylpiperazin-1-yl)ethyl)naphthalen-2-sulfonamide;
<47> N-(2-(4-benzylpiperazin-1-yl)ethyl)naphthalen-1-sulfonamide;
<48> N-(2-(4-phenylpiperazin-1-yl)ethyl)-[1,1'-biphenyl]-4-sulfonamide;
<49> N-(2-(4-phenylpiperazin-1-yl)ethyl)quinolin-2-sulfonamide;
<50> N-(2-(4-(4-chlorophenyl)piperazin-1-yl)ethyl)naphthalen-2-sulfonamide;
<51> N-(2-(4-(4-chlorophenyl)piperazin-1-yl)ethyl)naphthalen-1-sulfonamide;
<52> N-(2-(4-(4-chlorophenyl)piperazin-1-yl)ethyl)-[1,1'-biphenyl]-4-sulfonamide;
<53> N-(2-(4-(4-chlorophenyl)piperazin-1-yl)ethyl)quinolin-2-sulfonamide;
<54> N-(3-(4-benzylpiperidin-1-yl)propyl)naphthalen-2-sulfonamide;
<55> N-(3-(4-benzylpiperidin-1-yl)propyl)naphthalen-1-sulfonamide;
<56> N-(3-(4-benzylpiperidin-1-yl)propyl)-[1,1'-biphenyl]-4-sulfonamide;
<57> N-(3-(4-benzylpiperidin-1-yl)propyl)quinolin-2-sulfonamide;
<58> N-(3-(4-benzylpiperidin-1-yl)propyl)-4'-fluoro-[1,1'-biphenyl]-4-sulfonamide;
<59> N-(3-(4-benzylpiperidin-1-yl)propyl)-4'-chloro-[1,1'-biphenyl]-4-sulfonamide;
<60> N-(3-(4-benzylpiperidin-1-yl)propyl)-4'-methyl-[1,1'-biphenyl]-4-sulfonamide;
<61> N-(3-(4-benzylpiperidin-1-yl)propyl)-4'-methoxy-[1,1'-biphenyl]-4-sulfonamide;
<62> N-(3-(4-benzylpiperidin-1-yl)propyl)naphthalen-2-sulfonamide;
<63> N-(3-(4-benzylpiperidin-1-yl)propyl)naphthalen-1-sulfonamide;
<64> N-(3-(4-benzylpiperidin-1-yl)propyl)-[1,1'-biphenyl]-4-sulfonamide;
<65> N-(3-(4-phenylpiperidin-1-yl)propyl)quinolin-2-sulfonamide;
<66> N-(2-(4-benzylpiperazin-1-yl)propyl)naphthalen-2-sulfonamide;
<67> N-(2-(4-benzylpiperazin-1-yl)propyl)naphthalen-1-sulfonamide;
<68> N-(3-(4-phenylpiperazin-1-yl)propyl)-[1,1'-biphenyl]-4-sulfonamide;
<69> N-(3-(4-phenylpiperazin-1-yl)propyl)quinolin-2-sulfonamide;
<70> N-(3-(4-(4-chlorophenyl)piperazin-1-yl)propyl)naphthalen-2-sulfonamide;
<71> N-(3-(4-(4-chlorophenyl)piperazin-1-yl)propyl)naphthalen-1-sulfonamide;
<72> N-(3-(4-(4-chlorophenyl)piperazin-1-yl)propyl)-[1,1'-biphenyl]-4-sulfonamide;
<73> N-(3-(4-(4-chlorophenyl)piperazin-1-yl)propyl)quinolin-2-sulfonamide;
<74> 4'-fluoro-N-(3-(4-(3-phenylpropyl)piperidin-1-yl)propyl)-[1,1'-biphenyl]-4-sulfonamide;
<75> N-(3-(4-(3-phenylpropyl)piperidin-1-yl)propyl)-[1,1'-biphenyl]-4-sulfonamide;
<76> N-(3-(4-(3-phenylpropyl)piperazin-1-yl)propyl)-[1,1'-biphenyl]-4-sulfonamide;
<77> N-(4-(4-benzylpiperidin-1-yl)butyl)-[1,1'-biphenyl]-4-sulfonamide;
<78> N-(4-(4-(3-phenylpropyl)piperazin-1-yl)butyl)-[1,1'-biphenyl]-4-sulfonamide; and
<79> N-(4-(4-(3-phenylpropyl)piperidin-1-yl)butyl)-[1,1'-biphenyl]-4-sulfonamide.

7. A pharmaceutical composition for use in preventing or treating mental illness, comprising the compound represented by Formula 1 of claim 1, an isomer thereof, a solvate thereof, a hydrate thereof, or a pharmaceutically acceptable salt thereof as an active ingredient.

8. The pharmaceutical composition according to claim 7, wherein the compound inhibits the reuptake of serotonin, norepinephrine, and dopamine.

9. The pharmaceutical composition according to claim 7, wherein the mental disorder is at least one selected from the group consisting of bulimia nervosa, mood disorder, depression, atypical depression, depression secondary to pain, major depressive disorder, dysthymic disorder, bipolar disorder, bipolar I disorder, bipolar II disorder, cyclothymic disorder, mood disorder due to a general medical condition, substance-induced mood disorder, pseudo dementia, Ganger syndrome, obsessive-compulsive disorder, panic disorder, panic disorder without agoraphobia, panic disorder with agoraphobia, agoraphobia without history of panic disorder, panic attacks, memory deficit, memory loss, attention deficit hyperactivity disorder, obesity, anxiety, generalized anxiety disorder, eating disorder, Parkinson's disease, Parkinson's symptoms, dementia, aging dementia, senile dementia, Alzheimer's disease, Down syndrome, acquired immunodeficiency syndrome dementia complex, memory dysfunction in aging, specific phobia, social phobia, social anxiety disorder, post-traumatic stress disorder, acute stress disorder, chronic stress disorder, drug addiction, drug abuse, drug abuse tendency, cocaine abuse, nicotine abuse, tobacco abuse, alcohol addiction, alcoholism, pathological kleptomaniac, withdrawal syndrome due to withdrawal of intoxicating substances, pain, chronic pain, inflammatory pain, neuropathic pain, diabetic neuropathic pain, migraine, tension-type headache, chronic tension-type headache, depression-related pain, back pain, cancer pain, irritable bowel pain, irritable bowel syndrome, postoperative pain, postmastectomy pain syndrome (PMPS), poststroke pain, drug-induced neuropathy, diabetic neuropathy, pain sustained by the sympathetic nervous system, trigeminal neuralgia, toothache, facial muscle pain, phantom limb pain, anorexia, premenstrual syndrome, premenstrual dysphoric disorder, late luteal phase syndrome, posttraumatic syndrome, chronic fatigue syndrome, persistent vegetative state, urinary incontinence, stress incontinence, urge incontinence, nocturnal incontinence, sexual dysfunction, premature ejaculation, erectile difficulty, erectile dysfunction, restless legs syndrome, periodic limb movement disorder, eating disorder, anorexia nervosa, sleep disorder, pervasive developmental disorder, autism, Asperger's disorder, Rett disorder, childhood disintegrative disorder, learning disorder, motor ability disorder, mutism, trichotillomania, narcolepsy, post-stroke depression, stroke-induced brain injury, stroke-induced nerve injury, Tourette syndrome, tinnitus, tic disorder, body dysmorphic disorder, oppositional defiant disorder, and post-stroke disorder.

10. A triple reuptake inhibitor of serotonin, norepinephrine, and dopamine for use in preventing or treating mental illness, comprising the compound represented by Formula 1 of claim 1, an isomer thereof, a solvate thereof, a hydrate thereof, or a pharmaceutically acceptable salt thereof as an active ingredient.

11. A health functional food composition for use in preventing or improving mental illness, comprising the compound represented by Formula 1 of claim 1, an isomer thereof, a solvate thereof, a hydrate thereof, or a pharmaceutically acceptable salt thereof as an active ingredient.
